(19) Europäisches Patentamt
European Patent Office
Office européen des brevets

(11) Publication number: **0 277 384**
**A2**

# (12) EUROPEAN PATENT APPLICATION

(21) Application number: **87202601.8**

(22) Date of filing: **22.12.87**

(51) Int. Cl.[4]: **C07D 233/90**, C07D 405/04, C07D 401/06, C07D 413/04, C07D 403/04, C07D 401/04, C07D 405/14, C07D 413/14, C07D 409/04, A01N 43/50, A01N 43/40

(30) Priority: **30.12.86 GB 8631019**
**24.12.86 CH 5256/86**

(43) Date of publication of application:
**10.08.88 Bulletin 88/32**

(84) Designated Contracting States:
**AT BE CH DE ES FR GB GR IT LI LU NL SE**

(71) Applicant: **JANSSEN PHARMACEUTICA N.V.**
**Turnhoutseweg 30**
**B-2340 Beerse(BE)**

(72) Inventor: **De Bruyn, Marcel F.L.**
**Pater Schrijversstraat 4**
**B-2323 Hoogstraten(BE)**
Inventor: **Van Lommen, Guy R.E.**
**Klets 34**
**B-2590 Berlaar(BE)**
Inventor: **Lutz, William R.**
**Auf der Bischoffhöhe 104**
**CH-4125 Riehen(CH)**

## (54) 1H-imidazole-5-carboxylic acid derivatives.

(57) A method of controlling weeds by applying thereto or to the locus thereof of an imidazole derivative of formula

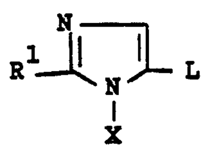

(I),

wherein
$R^1$ is hydrogen or mercapto;
L is cyano or a radical of formula

EP 0 277 384 A2

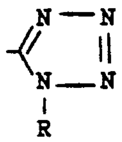 , 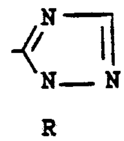 , 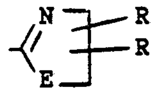 , 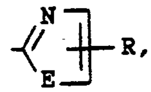 , 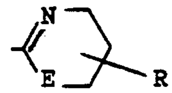 , 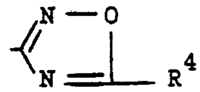

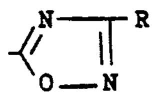 , 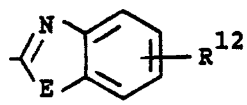 , 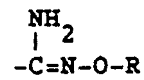 , 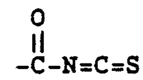 ,

$-C(=G)-D-R^2$ or $-C(=G^1)-O-R^5$;

X is 1-indanyl, 1-tetrahydronaphthalenyl, 5-benzocycloheptanyl, 4-tetrahydrobenzothienyl, 4-tetrahydrobenzofuryl, 5-tetrahydroquinolyl, 5-tetrahydroisoquinolyl, 8-tetrahydroquinolyl, 8-tetrahydroisoquinolyl, 9,10-dihydro-9-antracenyl, 9H-fluoren-9-yl, 5-dibenzo[a,d]-cycloheptenyl, 5-dibenzo[a,d]cycloheptanyl or 1-dihydronaphthalenyl each unsubstituted or substituted; or X is a group

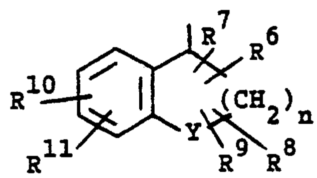 ,

or a group $-\underset{A}{\underset{|}{C}}H-Z$ ; wherein

n is 0, 1 or 2 or two; A is hydrogen, optionally substituted $C_3$-$C_7$cycloalkyl or pyridinyl, pyrimidinyl, naphthalenyl, furanyl and thienyl, each unsubstituted or substituted; Z is naphthalenyl, thienyl, furanyl, pyrimidinyl, phenyl or pyridinyl, each unsubstituted or substituted;
a salt or stereoisomeric form thereof, novel compounds for use in said method; herbicidal compositions containing such compounds as active ingredient and methods of preparing said compounds.

# 1$\underline{H}$-IMIDAZOLE-5-CARBOXYLIC ACID DERIVATIVES

## Background of the invention

A number of 1$\underline{H}$-imidazole-5-carboxylic acid derivatives are known from U.S. Patent No. 3,485,917 as antifungal agents. Further, some of these compounds are described as active agents in a method for inhibiting bud growth in U.S. Patent No. 3,873,297. U.S. Patent Nos. 3,354,173; 3,336,326; 4,038,286; 3,991,072 describe 1$\underline{H}$-imidazole-5-carboxylic esters and amides possessing hypnotic properties. A further series of 1$\underline{H}$-imidazole-carboxylic acids are known from U.S. Pat. No. 4,182,624 as plant-growth regulators, fungicides and herbicides. Additionally, a number of 1$\underline{H}$-imidazole-5-carboxylic acid derivatives are disclosed in the Publ. Eur. Pat. Appl. Nos. 0,185,961, published on July 2, 1986, and 0,219,798, published on April 29, 1987, as plant growth inhibitors.

## Description of the invention

The present invention is concerned with a method of controlling weeds by applying thereto or to the locus thereof of a herbicidally effective amount of a 1$\underline{H}$-imidazole derivative of formula

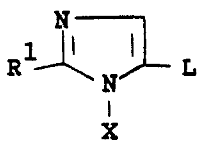

(I)

or a stereochemically isomeric form thereof, or a salt thereof, wherein
$R^1$ is hydrogen or mercapto;
L is cyano or a radical of formula

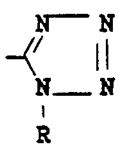 , 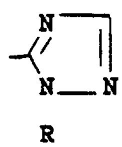 , 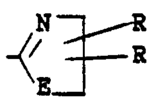 , 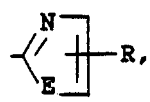 , 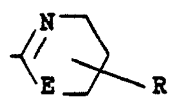 , 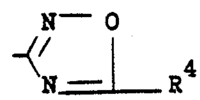 ,

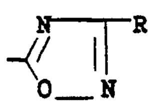 , 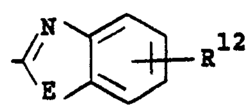 , 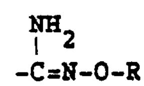 , 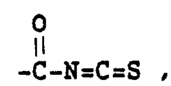 ,

$-C(=G)-D-R^2$ or $-C(=G^1)-O-R^5$;

each R independently is hydrogen or $C_1$-$C_5$alkyl;
E is oxygen, sulfur or -NR-;
$R^4$ is hydrogen, $C_1$-$C_5$alkyl or trifluoromethyl;
G is oxygen, sulfur or =NR;
$G^1$ is oxygen or sulfur;

D is sulfur, $-N(R^3)-$, $-N(R^3)-NH-$, $-N(R^3)-O-$, $-\overset{|}{N}-C(=G)-E-R^3$,

$-\overset{|}{N}-C(=G)-R^3$, $-\overset{|}{N}SO_2-R^3$, $-\overset{|}{N}-CN$, $-NH-CH_2-CH_2-O-$ or 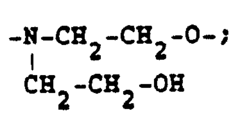

$R^2$ and each $R^3$ independently are hydrogen, $C_1$-$C_5$alkyl, $C_3$-$C_5$alkenyl, $C_3$-$C_5$alkynyl, $C_3$-$C_7$cycloalkyl or $C_1$-$C_5$alkyl substituted with aryl, $C_3$-$C_7$cycloalkyl, $C_3$-$C_7$cycloalkyloxy, $C_1$-$C_5$alkyloxy, hydroxy, carboxyl, $C_1$-$C_5$alkyloxycarbonyl or $C_1$-$C_5$alkylcarbonyl, or

$R^2$ and $R^3$ together with the nitrogen atom to which they are attached may form piperidinyl, pyrrolidinyl, 2-oxopiperidinyl, 2-oxypyrrolidinyl, morpholinyl, thiomorpholinyl, imidazolyl or piperazinyl being optionally substituted in the 4-position with $C_1$-$C_5$alkyl, $C_1$-$C_5$alkylcarbonyl, $C_1$-$C_5$alkoxycarbonyl or mono-and di$C_1$-$C_5$alkylaminocarbonyl, and said cyclic radicals derived from $R^2$ and $R^3$ each being optionally substituted with one to three $C_1$-$C_5$alkyl radicals;

$R^5$ is phenyl, naphthalenyl, or an aromatic five-or six-membered heterocycle connected on a carbon atom; said phenyl, naphthalenyl or heterocycle being optionally substituted where possible with 1 to 3 substituents independently selected from $C_1$-$C_5$alkyl, $C_2$-$C_5$alkenyl, $C_2$-$C_5$alkynyl, halo, $C_1$-$C_5$alkyloxy, $C_1$-$C_5$alkylthio, mono-, di-or trihalo$C_1$-$C_5$alkyl, mono-, di-or trihalo$C_1$-$C_5$alkyloxy and nitro;

or $R^5$ is a radical of formula $-N=CR^{14}R^{15}$; or $C_1$-$C_6$alkyl substituted with $-NR^{16}R^{17}$, $-NR^{16}-CO-R^{17}$, $-NR^{16}-CO-NR^{17}R^{18}$, hydroxy, mono-, di-or trihalo$C_2$-$C_8$alkyloxy, $-O-CO-R^{16}$, $-O-CO-O-R^{19}$, $-O-CO-NR^{16}R^{17}$, $-O-CO-CHCl-R^{16}$, $-O-CO-CCl_2-R^{16}$, $-O-SO_2-R^{19}$, $C_3$-$C_7$cycloalkyl, $-CO-R^{16}$, $-CO-NR^{16}R^{17}$, $-S(O)_mR^{19}$, $-SiR^{19}R^{20}R^{21}$ or $-PO(OR^{16})R^{22}$;

or $R^5$ is $C_1$-$C_6$alkyl substituted with one or two radicals independently selected from -CN and $-COOR^{16}$;

or $R^5$ is $C_1$-$C_6$alkyl substituted with one, two or three radicals independently selected from halo and mono-, di-or trihalo$C_1$-$C_6$alkyl;

$R^{14}$ is hydrogen, $C_1$-$C_6$alkyl, $C_3$-$C_7$cycloalkyl or aryl;

$R^{15}$ is $C_1$-$C_6$alkyl, $C_3$-$C_7$cycloalkyl or aryl;

or $R^{14}$ and $R^{15}$ taken together may form a $C_4$-$C_6$alkanediyl radical;

$R^{16}$, $R^{17}$ and $R^{18}$ independently are hydrogen, $C_1$-$C_6$alkyl, $C_3$-$C_5$alkenyl, $C_3$-$C_5$alkynyl, aryl, $C_1$-$C_5$alkyloxy$C_2$-$C_5$alkyl or $C_1$-$C_5$alkyloxycarbonyl$C_1$-$C_5$alkyl; or

$R^{16}$ and $R^{17}$ taken together may form a $C_3$-$C_6$alkanediyl radical;

$R^{19}$, $R^{20}$ and $R^{21}$ independently are $C_1$-$C_6$alkyl, $C_3$-$C_5$alkenyl, $C_3$-$C_5$alkynyl, or $C_1$-$C_5$alkyloxy$C_1$-$C_5$alkyl;

$R^{22}$ is hydrogen, $C_1$-$C_6$alkyl, hydroxy or $C_1$-$C_6$alkyloxy;

m is 0, 1 or 2;

X is 1-indanyl, 1-tetrahydronaphthalenyl, 5-benzocycloheptanyl, 4-tetrahydrobenzothienyl, 4-tetrahydrobenzofuryl, 5-tetrahydroquinolyl, 5-tetrahydroisoquinolyl, 8-tetrahydroquinolyl, 8-tetrahydroisoquinolyl, 9,10-dihydro-9-antracenyl, 9H-fluoren-9-yl, 5-dibenzo[a,d] cycloheptenyl, 5-dibenzo[a,d]cycloheptanyl or 1-dihydronaphthalenyl each unsubstituted or substituted with one to six substituents selected from the group consisting of $C_1$-$C_5$alkyl, mono-and di(aryl) $C_1$-$C_5$alkyl, $C_1$-$C_5$alkyloxy, halo, $C_3$-$C_7$alkenyl, amino, nitro, $C_1$-$C_5$alkylcarbonylamino, trifluoromethyl and difluoromethoxy, wherein two geminal substituents together with the carbon atom to which they are attached may form a spirocyclic $C_3$-$C_7$cycloalkyl group, or two of said substituents taken together may form a $C_1$-$C_5$alkanediyl or $C_5$-$C_7$cycloalkanediyl group, said $C_1$-$C_5$alkanediyl or $C_5$-$C_7$cycloalkanediyl group being optionally substituted with one or two radicals independently selected from $C_1$-$C_5$alkyl, mono-and di(aryl)$C_1$-$C_5$alkyl, $C_1$-$C_5$alkyloxy, halo, $C_3$-$C_7$alkenyl, trifluoromethyl, difluoromethoxy and aryl; or

X is a group

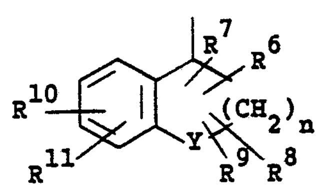 ,

or a group $-\underset{A}{\underset{|}{C}}H-Z$ ; wherein

n is zero, one or two;

Y is a group $-CH_2O-$, $-CH_2-S(O)_m-$, $-CH_2-N(R^{13})-$or $-CH=N-$, wherein the heteroatom is linked to the carbon atom of the benzene ring, and wherein m is zero, one or two;

$R^6$, $R^7$, $R^8$ and $R^9$ each independently are hydrogen, $C_1$-$C_5$alkyl, mono-and di(aryl)$C_1$-$C_5$alkyl, $C_1$-$C_5$alkyloxy, halo, $C_3$-$C_7$alkenyl, $C_1$-$C_5$alkyl substituted with one to three halo atoms, $C_1$-$C_5$alkyloxy, substituted with one to three halo atoms, or aryl; or

$R^6$ and $R^7$ taken together may form a fused benzene residue which may optionally be substituted with one

or two substituents each independently selected from hydrogen, $C_1$-$C_5$alkyl, $C_1$-$C_5$alkyloxy, halo, $C_1$-$C_5$alkyl substituted with one to three halo atoms, $C_1$-$C_5$alkyloxy substituted with one to three halo atoms, nitro, amino and -NH-CO-M; or where $R^6$ and $R^7$ are geminally substituted, they may form a spirocyclic carbon ring with 3 to 7 carbon atoms; or $R^6$ and $R^7$ taken together may form a $C_1$-$C_5$alkanediyl or a $C_5$-$C_7$cycloalkanediyl group being optionally substituted with one or two radicals independently selected from $C_1$-$C_5$alkyl, mono-and di(aryl)$C_1$-$C_5$alkyl, $C_1$-$C_5$alkyloxy, halo, $C_3$-$C_7$alkenyl, $C_1$-$C_5$alkyl substituted with one to three halo atoms, $C_1$-$C_5$alkyloxy substituted with one to three halo atoms and aryl; and

$R^{10}$, $R^{11}$ and $R^{12}$ are each independently hydrogen, $C_1$-$C_5$alkyl, $C_1$-$C_5$alkyloxy, halo, $C_1$-$C_5$alkyl substituted with one to three halo atoms, $C_1$-$C_5$alkyloxy substituted with one to three halo atoms, cyano, nitro, amino, mono-and di$C_1$-$C_5$alkylamino, or -NH-CO-M;

$R^{13}$ is hydrogen, $C_1$-$C_5$alkyl, $C_1$-$C_5$alkanoyl or 4-methylphenylsulfonyl;

A is hydrogen; $C_3$-$C_7$cycloalkyl optionally substituted with one or two $C_1$-$C_5$alkyl radicals; $C_1$-$C_7$alkyl optionally substituted with $C_1$-$C_7$alkyloxy or with an Ar radical; or $C_1$-$C_7$alkyl substituted with both a $C_1$-$C_7$alkyloxy and an Ar radical; or a radical selected from pyridinyl, pyrimidinyl, naphthalenyl, furanyl and thienyl, each unsubstituted or substituted with one or two radicals independently selected from $C_1$-$C_5$alkyl, $C_1$-$C_5$alkyloxy, halo, nitro, amino, mono-and di$C_1$-$C_5$alkylamino, -NH-CO-M, cyano, trifluoromethyl and difluoromethoxy;

said radical Ar being phenyl, pyridinyl, pyrimidinyl, naphthalenyl, furanyl or thienyl, each unsubstituted or substituted with one or two and in case Ar is phenyl also with three substituents selected from $C_1$-$C_5$alkyl, $C_1$-$C_5$alkyloxy, halo, nitro, amino, mono-and di$C_1$-$C_5$alkylamino, -NH-CO-M, cyano, trifluoromethyl and difluoromethoxy;

Z is naphthalenyl, thienyl, furanyl, pyrimidinyl, phenyl or pyridinyl, each unsubstituted or substituted with one or two substituents and in case Z is phenyl also with three substituents independently selected from $C_1$-$C_5$alkyl, $C_1$-$C_5$alkyloxy, halo, cyano, nitro, amino, mono-and di$C_1$-$C_5$alkylamino, -NH-CO-M, trifluoromethyl and difluoromethoxy; and

M is $C_1$-$C_5$alkyl; and

aryl is phenyl optionally substituted with one to three substituents each independently selected from $C_1$-$C_5$alkyl, $C_1$-$C_5$alkyloxy and halo.

Surprisingly, the compounds of formula (I) exhibit strong herbicidal properties, and are therefore useful to control weeds. This property gains importance by the fact, that some crops of useful plants are not damaged, or are only slightly harmed when treated with compounds of formula (I) at high dosages . Consequently, the compounds of formula (I) are valuable selective herbicides in crops of useful plants, such as sugar beet, rape, soybeans, cotton, sunflower, cereals, especially wheat, barley, rye and oats, rice, both upland rice and paddy rice, and maize. Especially in rice crops a broad range of application rates can be employed, preferably if the rice crops are transplanted rice crops, and if the compounds of formula (I) are applied after transplantation. In maize crops selective herbicidal action is observed both at preemergence and at postemergence treatment.

The active ingredients (a.i.) of formula (I) are usually applied at application rates of 0.01 to 5.0 kg of active ingredient per hectare in order to achieve satisfying results. Sometimes, depending on the environmental conditions, the application rates may exceed the above designated limitations. However, the preferred application rates are between 0.05 kg and 1.0 kg a.i. per hectare.

As used in the foregoing definitions $C_1$-$C_5$alkyl denotes straight or branch chained saturated hydrocarbon radicals having from 1 to 5 carbon atoms, e.g. methyl, ethyl, n-propyl, isopropyl, the four butyl isomers and the pentyl isomers; $C_1$-$C_6$alkyl includes $C_1$-$C_5$alkyl radicals and the higher homologs thereof having 6 carbon atoms; $C_2$-$C_8$alkyl denotes straight or branch chained saturated hydrocarbon radicals having from 2 to 8 carbon atoms; halo is fluoro, chloro, bromo or iodo, with fluoro and chloro being preferred; $C_3$-$C_5$alkenyl defines straight and branch chained hydrocarbon radicals containing one double bond and having from 3 to 5 carbon atoms such as, for example, 2-propenyl, 3-butenyl, 2-butenyl, 2-pentenyl, 3-pentenyl, 2-methyl-2-propenyl, or 3-methyl-2-butenyl, with 2-propenyl and 2-methyl-2-propenyl being preferred; $C_2$-$C_5$alkenyl includes $C_3$-$C_5$alkenyl and ethenyl; $C_3$-$C_7$alkenyl includes $C_3$-$C_5$alkenyl and the hexenyl and heptenyl isomers; $C_3$-$C_5$alkynyl defines straight and branch chained hydrocarbon radicals containing one triple bond and having from 3 to 5 carbon atoms such as, for example, propargyl, 2-butynyl, 3-butynyl, 2-pentynyl, 3-pentynyl or 4-pentynyl, with propargyl being preferred; $C_2$-$C_5$alkynyl includes $C_3$-$C_5$alkynyl and ethynyl; $C_3$-$C_7$alkynyl includes $C_3$-$C_5$alkynyl and the hexynyl and heptynyl isomers; and when said $C_3$-$C_7$alkynyl or said $C_3$-$C_7$alkenyl are substituted on a heteroatom, then the carbon atom of said $C_3$-$C_7$alkynyl or $C_3$-$C_7$alkenyl, connected to said heteroatom is saturated; $C_3$-$C_7$cycloalkyl defines cyclopropyl, cyclobutyl, cyclopentyl, cyclohexyl and cycloheptyl, with cyclopentyl and cyclopentyl being preferred; and $C_1$-$C_5$-alkanoyl denotes formyl, acetyl, propionyl, butyryl, isobutyryl, valeryl, isovaleryl or pivaloyl; $C_3$-$C_6$alkanediyl, $C_4$-$C_6$alkanediyl and $C_1$-$C_5$alkanediyl define bivalent straight or branch chained saturated hydrocarbon

radicals having from 3 to 6, 4 to 6 or from 1 to 5 carbon atoms such as methylene, 1,2-ethanediyl, 1,3-propanediyl, 1,4-butanediyl, 1,5-pentanediyl and 1,6-hexanediyl; $C_5$-$C_7$cycloalkanediyl denotes cyclopentanediyl, cyclohexanediyl and cycloheptanediyl.

The term mono-, di-or trihalo$C_1$-$C_5$alkyl as used hereinabove defines alkyl radicals wherein one, two or three hydrogens have been replaced by halo atoms. Examples of such radicals are fluoromethyl, difluoromethyl, trifluoromethyl, trichloromethyl, chloroethyl, trifluoroethyl and the like.

The radicals $R^6$, $R^7$, $R^8$ and $R^9$ as defined hereinabove may be substituted to any carbon atom making up the Y containing part of the bicyclic ring system, including the $CH_2$ or CH-groups of either the $-(CH_2)_n$ or $-CH_2S-$, $-CH_2O-$, $-CH_2NR^{13}$-or -CH = N-fragments.

In the above definitions $R^{16}$ and $R^{17}$ taken together may form a $C_3$-$C_6$alkanediyl radical. Where in the latter instance $R^{16}$ and $R^{17}$ are bound to the same nitrogen they may form together with said nitrogen e.g. a pyrrolidine or piperidine ring. Or said $R^{16}$ and $R^{17}$ being bound to a ureido rest [$-NR^{16}-CO-NR^{17}R^{18}$] can make up a ureido-containing ringsystem. The same applies for such $R^{16}$ and $R^{17}$ being bound to a carbamide rest [$-NR^{16}-CO-R^{17}$]. $R^5$ being an aromatic heterocycle comprises heterocycles containing in particular 5 or 6 ring atoms of which one can be oxygen or sulfur or of which 1 to 3 atoms can be nitrogen and optionally a further oxygen or sulfur. Examples of such heterocycles are thiophene, furan, pyrrol, imidazole, triazole, oxazole, oxadiazole, thiadiazole, pyridine, pyrimidine, pyridazine, pyrazine, triazine and the like.

The condensed cyclic ring systems being defined under the symbol X and being attached to the 1-position of the imidazole ring for example comprise the following basic structures which may be unsubstituted or substituted with the substituents as defined hereinabove:

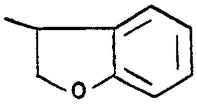 ,
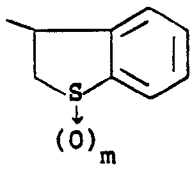 ,
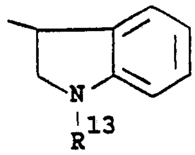 ,

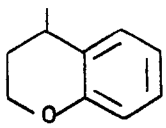 ,
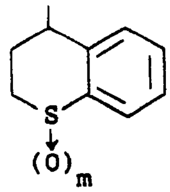 ,
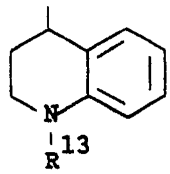 ,

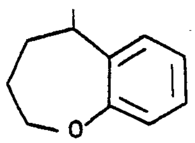 ,
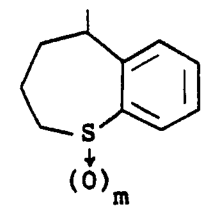 ,
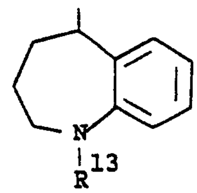 ,

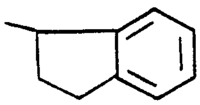 ,
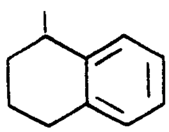 ,
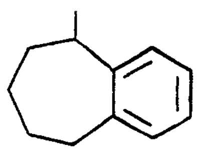 ,
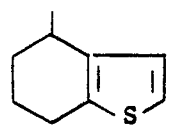 ,

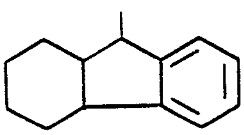 ,
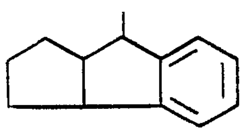 ,
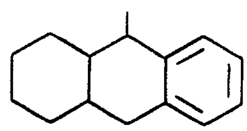 ,

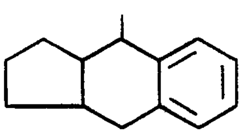 ,
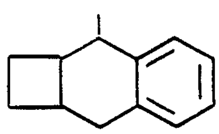 ,
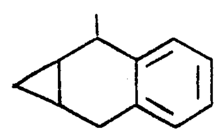 ,

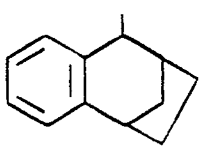 ,
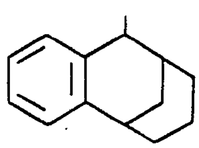 ,

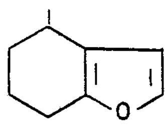 , 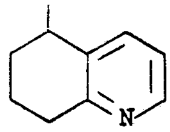 , 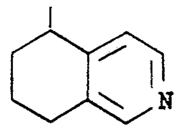 , 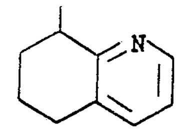 ,

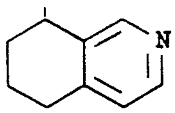 , 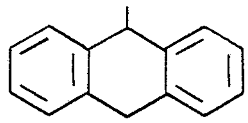 , 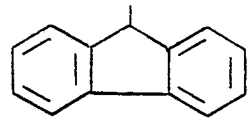 ,

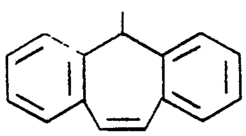 , 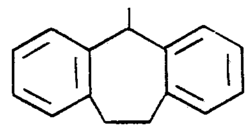 and 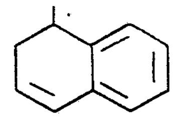

As defined hereinabove, $R^6$ and $R^7$ together with the two carbon atoms to which they are attached may form an optionally substituted benzene ring, in case these two carbon atoms are adjacent. As such, the Y-containing heterocycle carries two fused benzene rings. Typical examples thereof may be represented by the following formulae:

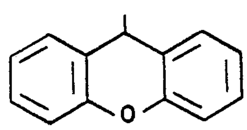 , 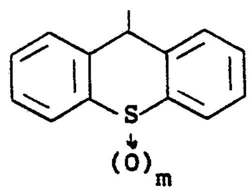 , 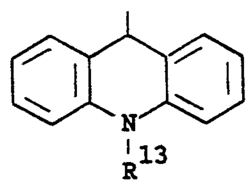 ,

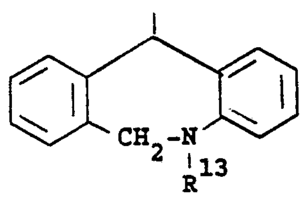 , 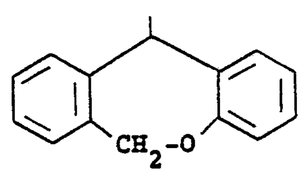 and 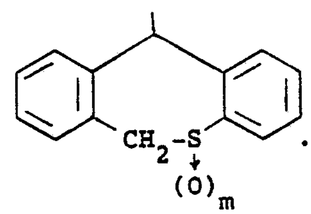 .

It is evident that these structures may be substituted as defined hereinabove.

As further defined hereinabove $R^6$ and $R^7$ being attached to the same carbon atom may form a spirocyclic ring together with said carbon atom. Typical embodiments of such spirocyclic rings are cyclopropane, cyclobutane, cyclopentane, cyclohexane and cycloheptane.

Depending on the nature of the radical X and/or the group L, the compounds of formula (I) may contain asymmetrical carbon atoms. Unless otherwise mentioned or indicated, the chemical designation of compounds, within the scope of the present invention, denotes the mixtures of all possible stereochemically isomeric forms, said mixtures containing all diastereomeres and enantiomeres of the basic molecular structure.

The absolute configuration of each chiral center may be indicated by the stereochemical descriptors R and S, this R and S notation corresponding to the rules described in Pure Appl. Chem. 1976, 45, 11-30. The relative configuration of the asymmetric centers in the compounds of formula (I) is denoted by cis and trans and where appropriate by the terms α and β, these stereochemical descriptors being used according to the rules described in Chemical Abstracts 1977 Index Guide, Appendix IV, § 203.

In some compounds the stereochemical configuration is not experimentally determined. In those cases it is conventionally agreed to designate the stereochemically isomeric form which is first isolated as "A" and the second as "B", without further reference to the actual stereochemical configuration.

Pure isomeric forms of the compounds of formula (I) can be separated from the mixtures by conventional separation methods. Preferably, if a specific stereochemical form is desired, said compound will be synthesized by stereoselective methods of preparation. These methods will advantageously employ pure forms of optically active starting materials.

The invention also comprises the salts which the compounds of formula (I) are able to form with organic or inorganic bases such as amines, alkali metal bases and earth alkali metal bases, or quaternary ammonium bases, or with organic or inorganic acids, such as mineral acids, sulfonic acids, carboxylic acids or phosphorus containing acids.

Preferred salt-forming alkali metal hydroxides and earth alkali metal hydroxides are the hydroxides of lithium, sodium, potassium, magnesium or calcium, more particularly sodium or potassium hydroxide. Examples of appropriate salt-forming amines are the primary, secondary and tertiary aliphatic and aromatic amines such as methylamine, ethylamine, propylamine, isopropylamine, the four butylamine isomers, dimethylamine, diethylamine, diethanolamine, dipropylamine, diisoproylamine, di-n-butylamine, pyrrolidine, piperidine, morpholine, trimethylamine, triethylamine, tripropylamine, pyridine, quinoline, isoquinoline and quinuclidine. Preferred amines are ethylamine, propylamine, diethylamine or triethylamine and more particularly isopropylamine, diethanolamine or 1,4-diazabicyclo[2.2.2]octane. Examples of quaternary ammonium salts generally contain cations arising from ammonium hydroxides or ammonium halogenide salts, for example, the ammonium, tetramethylammonium, tetraethylammonium, trimethylethylammonium or trimethylbenzylammonium cation.

Examples of salt-forming mineral acids are hydrofluoric acid, hydrochloric acid, hydrobromic acid, hydroiodic acid, sulfuric acid, nitric acid, chloric acid, perchloric acid or phosphoric acid. Preferred salt-forming sulfonic acids are toluenesulfonic acid, benzenesulfonic acid, methanesulfonic acid or trifluoromethanesulfonic acid. Preferred salt-forming carboxylic acids are acetic acid, trifluoroacetic acid, benzoic acid, chloroacetic acid, phthalic acid, maleic acid, malonic acid and citric acid. Phosphorus containing acids are the various phosphonous acids, phosphonic acids and phosphinic acids.

A particular subgroup amoung the compounds of formula (I) comprises those compounds of formula (I) wherein $R^1$ is a defined above, L is a radical of formula $-C(=G^1)-O-R^5$, wherein $G^1$ is as defined above and $R^5$ is $C_1$-$C_6$haloalkyl, $C_2$-$C_8$haloalkoxyalkyl, phenyl, naphtalenyl or an aromatic 5 or 6-membered heterocylcic being connected on a carbon atom; said phenyl naphthalenyl or heterocycle being optionally substituted with $C_1$-$C_4$alkyloxy, $C_1$-$C_4$alkylthio, $C_1$-$C_4$haloalkyl, $C_1$-$C_4$haloalkyloxy or nitro; or $R^5$ is $-N=CR^{14}R^{15}$ or $C_1$-$C_6$alkyl substituted with $-NR^{16}R^{17}$, $-NR^{16}COR^{17}$, $-COOR^{16}$, $-NR^{16}-CO-NR^{17}R^{18}$, $-SiR^{19}R^{20}R^{21}$, $-OCOR^{16}$, cyano, $C_3$-$C_7$cycloalkyl, $-OCOOR^{19}$, $-OCONR^{16}R^{17}$, $-PO(OR^{16})R^{22}$, $-S(O)_mR^{19}$, $-O-CO-CHCl-R^{16}$ or $-O-CO-CCl_2-R^{16}$; wherein

$R^{14}$ is hydrogen, $C_1$-$C_6$alkyl, $C_3$-$C_6$cycloalkyl or aryl;

$R^{15}$ is $C_1$-$C_6$alkyl, $C_3$-$C_6$cycloalkyl or aryl;

$R^{16}$, $R^{17}$ and $R^{18}$ independently are hydrogen, $C_1$-$C_6$alkyl, $C_3$-$C_5$alkenyl, $C_3$-$C_5$alkynyl, $C_2$-$C_5$alkoxyalkyl or $C_1$-$C_5$alkoxycarbonyl$C_1$-$C_5$alkyl; or

$R^{16}$ and $R^{17}$ taken together may form a $C_3$-$C_6$alkanediyl radical;

$R^{19}$, $R^{20}$ and $R^{21}$ independently are $C_1$-$C_6$alkyl, $C_3$-$C_5$alkenyl, $C_3$-$C_5$alkynyl or $C_2$-$C_5$alkyloxyalkyl;

$R^{22}$ is hydrogen, $C_{1-6}$alkyl or $C_1$-$C_6$alkyloxy;

m is 0, 1 or 2;

X is 1-indanyl, 1-tetrahydronaphthalenyl, 5-benzocycloheptanyl, 4-tetrahydrobenzothienyl, 4-tetrahydrobenzofuryl, 5-tetrahydro-quinolyl, 5-tetrahydroisoquinolyl, 8-tetrahydroquinolyl, 8-tetrahydroisoquinolyl, 9,10-dihydro-9-antracenyl, 9H-fluoren-9-yl, 5-dibenzo[a,d]cycloheptenyl, 5-dibenzo[a,d,]cycloheptanyl or 1-dihydronaphthalenyl each unsubstituted or substituted with one to six substituents selected from the group consisting of $C_1$-$C_5$alkyl, mono-and di(aryl) $C_1$-$C_5$alkyl, $C_1$-$C_5$alkyloxy, halo, $C_3$-$C_7$alkenyl, amino, nitro, $C_1$-$C_5$alkylcarbonylamino, trifluoromethyl and difluoromethoxy, wherein two geminal substituents together with the carbon atom to which they are attached may form a spirocyclic $C_3$-$C_7$cycloalkyl group, or two vicinal substituents taken together may form an annellated $C_4$-$C_7$-cycloalkyl ring; or

X is a group

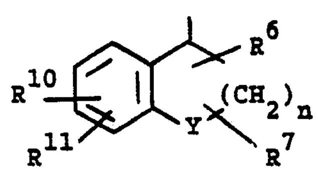

,

or a group, $-\underset{A}{C}H-Z$ ; wherein

n is zero, one or two;

Y is oxygen or sulfur;

$R^6$ and $R^7$ each independently are hydrogen, $C_1$-$C_5$alkyl, $C_1$-$C_5$haloalkyl, $C_1$-$C_5$alkyloxy or halo; or $R^6$ and $R^7$ taken together may form a fused benzene residue or where $R^6$ and $R^7$ are geminally substituted, they may form a spirocyclic carbon ring with 3 to 7 carbon atoms; or $R^6$ and $R^7$ taken together may form a $C_1$-$C_5$alkanediyl group ; and

$R^{10}$ and $R^{11}$ are each independently hydrogen, $C_1$-$C_5$alkyl, $C_1$-$C_5$alkyloxy, halo, $C_1$-$C_5$haloalkyl or $C_1$-$C_5$haloalkyloxy;

A is hydrogen; straight or branch chained $C_1$-$C_7$alkyl; $C_2$-$C_6$alkyloxyalkyl; benzyl or phenethyl each unsubstituted or substituted in the aliphatic part with $C_1$-$C_4$alkyl, $C_3$-$C_7$cycloalkyl, phenyl, pyridinyl or $C_1$-$C_4$alkyloxy or in the phenylring with halo, nitro or $C_1$-$C_4$alkyloxy; or a radical selected from pyridinyl, pyrimidinyl, naphthalenyl, furanyl and thienyl, each unsubstituted or substituted with one or two radicals independently selected from $C_1$-$C_5$alkyl, $C_1$-$C_5$alkyloxy, halo, nitro, trifluoromethyl and difluoromethoxy;

Z is phenyl, pyridinyl, $C_3$-$C_7$cycloalkyl each unsubstituted or substituted with one or two substituents independently selected from $C_1$-$C_5$alkyl, $C_1$-$C_5$alkyloxy, halo, nitro, trifluoromethyl and difluoromethoxy; and

aryl is phenyl optionally substituted with one to three substituents each independently selected from $C_1$-$C_5$alkyl, $C_1$-$C_5$alkyloxy and halo. As used in the previous definitions halo$C_1$-$C_5$alkyl for example is $FCH_2$-, $F_2CH$-, $F_3C$-, $Cl_3C$-, $CF_3CH_2$-, $CCl_3$-$CH_2$-, $CF_3CH_2CH_2$-and the like. Halo$C_1$-$C_5$alkyloxy defines the same radicals being connected to oxygen.

Another particular subgroup amoung the compounds of formula (I) are those compounds of formula (I) wherein L is other than a group -C(=$G^1$)-O-$R^5$. A further particular subgroup amoung the latter group comprises the compounds of formula (I) wherein L is -C(=G)-D-$R^2$.

Among the compounds of formula (I) those compounds are preferred wherein L is cyano, a radical of formula -C(=O)-D-$R^2$ or -COOR$^5$.

Among this group preference is given to compounds wherein D is -N($R^3$)-or -N($R^3$)-O-and $R^2$ is hydrogen, $C_1$-$C_5$alkyl, hydroxy$C_1$-$C_5$alkyl, $C_3$-$C_7$cycloalkyl, $C_3$-$C_5$alkenyl or $C_3$-$C_5$alkynyl and $R^3$ is hydrogen or $C_1$-$C_5$alkyl; and/or

$R^5$ is phenyl optionally substituted with 1 to 3 halo atoms, or a radical of formula -N=CR$^{14}$R$^{15}$ wherein $R^{14}$ and $R^{15}$ independently are hydrogen or $C_1$-$C_6$alkyl; or a $C_1$-$C_6$alkyl group substituted with di-$C_1$-$C_6$alkyloxyphosphonyl, tri-$C_1$-$C_6$alkylsilyl, amino, cyano, carboxyl or $C_1$-$C_5$alkyloxycarbonyl, or $C_1$-$C_6$alkyl substituted with one to three halo's; and/or wherein X is 1-indanyl, 1-tetrahydronaphtalenyl, 4-chromanyl, said three radicals being substituted with one or two $C_1$-$C_5$alkyl radicals or with spiro$C_5$-$C_6$alkanediyl; and/or X is $C_1$-$C_7$alkyl substituted with an Ar radical.

In particular L may be cyano, methylcarbamoyl, ethylcarbamoyl, carbamoyl, dimethylcarbamoyl, propylcarbamoyl, cyclopropylcarbamoyl, methoxycarbamoyl, allylcarbamoyl propargylcarbamoyl, hydroxyethylcarbamoyl, or L is a radical of formula -COOR$^5$ wherein $R^5$ is 3-chloropropyl, trimethylsilylmethyl, 2-trimethylsilylethyl, carboxylmethyl, methoxycarbonylmethyl, diethoxyphosphonylmethyl, cyanomethyl, 2-aminoethyl, 2,2,2-trifluoroethyl, 1-methoxycarbonylethyl or -N=C($CH_3$)$_2$.

Particularly preferred compounds of formula (I) are those preferred compounds of formula (I) wherein $R^1$ is hydrogen, X is 2,3-dihydro-2,2-dimethyl-1H-inden-1-yl, 2,2-dimethyltetrahydronaphthalenyl, 2,3-dimethylchroman-4-yl or 2-methyl-chroman-4-yl.

The most preferred compounds of the present invention are selected from the group comprising the following compounds, which compounds moreover are deemed novel:

N-methyl-1-(1,2,3,4-tetrahydro-1-naphthalenyl)-1H-imidazole-5-carboxamide, 1-(2,3-dihydro-2,2-dimethyl-1H-indenyl)-1H-imidazole-5-carboxamide and N-methyl-1-(2,3-dihydro-2,2-dimethyl-1H-indenyl)-1H-imidazole-5-carboxamide.

An additional feature of the present invention comprises the fact that many of the compounds of formula (I) are novel and have especially been developed to be used in the method according to the present invention. Novel compounds within the present invention are those compounds of formula (I) as defined hereinabove wherein

(i) when X is 1-aryl-1-ethyl then L is other than cyano; $CONH_2$; or COOR$^5$ wherein $R^5$ is monohalo$C_1$-$C_5$alkyl; or

(ii) when X is indanyl, or 1,2,3,4-tetrahydro-1-naphthalenyl, then L is other than COOR$^5$ wherein $R^5$ is di$C_1$-$C_5$alkylamino$C_1$-$C_5$alkyl.

A particular group of novel compounds are those novel compounds as defined hereinabove wherein when X is 1-aryl-1-$C_1$-$C_7$alkyl, then L is other than COOR$^5$ wherein $R^5$ is monohalo$C_1$-$C_5$alkyl.

Preferred novel compounds are those wherein L is cyano, a radical of formula -C(=O)-D-$R^2$ or -COOR$^5$.

Particularly preferred novel compounds are those preferred novel compounds wherein D is -N($R^3$)-or -N($R^3$)-O-and $R^2$ is hydrogen, $C_1$-$C_5$alkyl, hydroxy$C_1$-$C_5$alkyl, $C_3$-$C_7$cycloalkyl, $C_3$-$C_5$alkenyl or $C_3$-$C_5$alkynyl and $R^3$ is hydrogen or $C_1$-$C_5$alkyl; and/or $R^5$ is phenyl optionally substituted with 1 to 3 halo atoms, or a

radical of formula $-N=CR^{14}R^{15}$ wherein $R^{14}$ and $R^{15}$ independently are hydrogen or $C_1$-$C_6$alkyl; or a $C_1$-$C_6$alkyl group substituted with di-$C_1$-$C_6$alkyloxyphosphonyl, tri-$C_1$-$C_6$alkylsilyl, amino, cyano, carboxyl or $C_1$-$C_5$alkyloxycarbonyl, or $C_1$-$C_6$alkyl substituted with one to three halo's; and/or wherein X is 1-indanyl, 1-tetrahydronaphtalenyl, 4-chromanyl being substituted with one or two $C_1$-$C_5$alkyl radicals or with spiro$C_5$-$C_6$alkanediyl. In particular L may be cyano, methylcarbamoyl, ethylcarbamoyl, carbamoyl, dimethylcarbamoyl, propylcarbamoyl, cyclopropylcarbamoyl, methoxycarbamoyl, allylcarbamoyl, propargylcarbamoyl, hydroxyethylcarbamoyl, or L is a radical of formula $-COOR^5$ wherein $R^5$ is 3-chloropropyl, trimethylsilylmethyl, 2-trimethylsilylethyl, carboxylmethyl, methoxycarbonylmethyl, diethoxyphosphonylmethyl, cyanomethyl, 2-aminoethyl, 2,2,2-trifluoroethyl, 1-methoxycarbonylethyl or $-N=C(CH_3)_2$. Particularly preferred are those novel compounds wherein $R^1$ is hydrogen, X is 2,3-dihydro-2,2-dimethyl-1H-indenl-yl, 2,2-dimethyltetrahydronaphthalenyl, 2,3-dimethyl-chroman-4-yl or 2-methyl-chroman-4-yl.

The preparation of the compounds of formula (I) is generally carried out by the following methods.

The compounds of formula (I) can be obtained by condensing a compound of formula

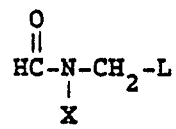

(II)

wherein X and L are as defined hereinabove, with a $C_1$-$C_4$alkyl ester of formic acid in the presence of suitable base such as, for example, an alkali metal alkoxide or hydride, e.g. sodium methoxide, potassium ethoxide, sodium hydride, lithium hydride, and the like, in a reaction-inert solvent; and treating the resultant intermediate of formula

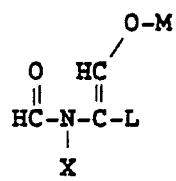

(III)

wherein X and L are as defined hereinabove and M is an alkali metal atom,

a) with an alkali metal isothiocyanate in the presence of an acid, thus obtaining a 2-mercaptoimidazole of formula

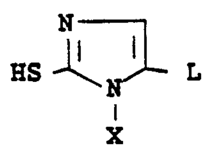

(Ia)

wherein X and L are as defined hereinabove, which optionally is converted into a compound of formula

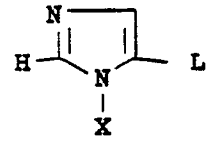

(Ib)

by reacting the starting compound with nitric acid optionally in the presence of an alkali metal nitrite, e.g. sodium nitrite; or with Raney-nickel in the presence of a lower aliphatic alcohol, preferably ethanol, at a temperature between 40°C and 80°C; or also by treating the starting compounds with an aqueous hydrogen peroxide solution preferably in the presence of a carbolic acid, e.g. acetic acid; or

b) with a carboxylic acid amide of 1 to 3 carbon atoms, preferably formamide, in the presence of an acid at a temperature between 50°C and 250°C, preferably between 120°C and 170°C; or

c) with an excess of ammonium carbonate or hydrogen carbonate in a suitable solvent, which may be a reaction-inert solvent or an acid, at a temperature between 20°C and 200°C, preferably between 25°C and the reflux temperature of the reaction mixture.

In the afore-mentioned processes reaction-inert solvents are, for example, aromatic hydrocarbons such as benzene, methylbenzene or dimethylbenzene; ethers such as, for example, 1,1'-oxybisethane, tetrahydrofuran or 1,4-dioxane; or other aprotic organic solvents. For the cyclization-reaction of the imidazole ring structure, strong mineral acids such as hydrolic acids, e.g. hydrochloride acid, are most conveniently employed. In the ring-forming variant c) other acids, e.g. acetic acid, can also be used. In this reaction an excess of acid of 5 to 50, preferably of 15 to 40 times the required molar amount is most preferably used. The excess of ammonium salt in this process is 2 to 50, preferably 10 to 30 times the required molar amount.

The compounds of formula (I-b) can also be prepared by the deamination reaction of a 4-amino-1H-imidazole derivative of formula (IV), wherein L and X are as defined under formula (I) and L in particular is cyano or an amide group. Said deamination reaction involves a diazotation and a reductive dediazotation step which may be conducted sequentially, i.e. with isolation of the intermediate diazonium salt (IV-a) or in a one-pot fashion wherein said diazonium salt is reduced in situ.

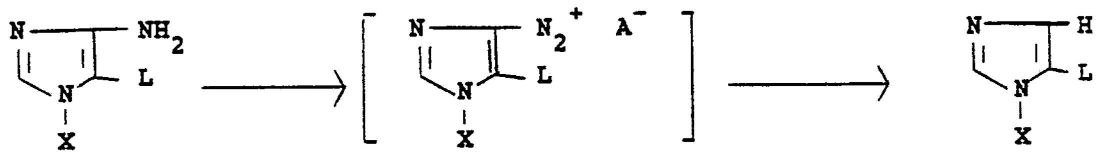

(IV) (IV-a) (I-b)

Treatment of the 4-amino-1H-imidazole derivative of formula (IV) in aqueous medium with an alkali metal nitrite, e.g. sodium or potassium nitrite, in the presence of an acid such as hydrochloric acid, sulfuric acid or nitric acid, or with nitronium tetrafluoroborate ($NO^+BF_4^-$) yields the diazonium salt (IV-a). In the latter, L and X are as defined under formula (I) and A represents an anion corresponding to the conjugated base of the acid employed in the diazotation reaction or the tetrafluoroborate anion. The intermediate diazonium salts (IV-a) are reduced to the compounds of formula (I-b) by treatment with an appropriate reductant such as hypophosphoric acid at an elevated temperature, preferably at the boiling temperature of the reaction mixture.

Alternatively, treatment of the 4-amino-1H-imidazole derivatives of formula (IV) with a $C_{1-5}$alkyl nitrite such as, 1,1-dimethylethyl nitrite or 3-methylbutyl nitrite in suitable aprotic solvent such as tetrahydrofuran, 1,4-dioxane, trichloromethane, or N,N-diethylformamide yields a compound of formula (I-b) directly. The latter deamination reaction may conveniently be conducted at an elevated temperature, generally at the boiling point of the reaction mixture.

The compounds of formula (I) can also be converted into each other following art-known functional group transformation reactions. The substituent L may be transformed into other substituents encompassed by the definition of L by convenient reactions known in the art for the modification of carboxylic acid derivatives, e.g. by hydrolysis and esterification and/or transesterification and/or amidation or transamidation or conventional ring formation reactions.

The compounds of formula (I), wherein L is a radical of formula $-C(=G)-D-R^2$ or $-C(=G^1)-OR^5$, can also be obtained from the structurally related carboxylic acids or thiocarboxylic acids or functional derivatives thereof by an amidation or esterification or from the related esters or amides by an appropriate transamidation or transesterification reaction. A preferred procedure is to convert the said acids into activated derivatives thereof following art-known procedures, for example, by treating the said acids with an appropriate halogenating agent, such as, for example, thionyl chloride, thionyl bromide, phosphoryl chloride, phosphoryl bromide, phosphorous trichloride, phosphorous tribromide, pentachlorophosphorane and the like. Or by dehydrating the carboxylic acid to the corresponding anhydride or by reacting the carboxylic acid with acyl halide, e.g. acetyl or 2,2-dimethylpropanoyl chloride, ethyl or 1,1-dimethylethyl carbonochloridate and the like.

The thus obtained activated derivatives of formula (V)

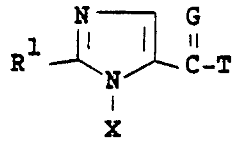

(V)

wherein R', G and X are as defined under formula (I), and T is a reactive leaving group, e.g. halo, in particular chloro or bromo, an -O-acyl group, e.g. -O-CO-$C_1$-$C_5$alkyl or -O-CO-O-$C_1$-$C_5$alkyl and the like, or a group

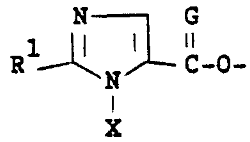

,

are reacted with the appropriate mercaptan or an amino compound of formula H-D-$R^2$, or with a hydroxy compound of formula H-O-$R^5$, wherein D, $R^2$ and $R^5$ are as defined under formula (I). It may be appropriate to add a suitable base such as, for example, a trialkylamine, e.g. triethylamine to the reaction mixture in order to remove the acid which is liberated during the course of the reaction by salt-formation. Alternatively the compounds of formula (I) wherein L is a radical of formula -C(=G)-D-$R^2$ or -C(=G)-O$R^5$ may also be prepared by treating the starting acids or thioacids and the amine, mercaptan or alcohol in the presence of a suitable reagent capable of forming amides, esters or thioesters, e.g. a carbodiimide such as dicyclohexylcarbodiimide (DCC), 2-halo-1-alkyl-pyridinium halides such as 2-chloro-1-methylpyridinium iodide, 1,1'-carbonylbis[1H-imidazole] and the like. The said amidation or esterification reactions are preferably conducted in a reaction-inert solvent such as, for example, a hydrocarbon, e.g. methylbenzene, dimethylbenzene; a halogenated hydrocarbon, e.g. dichloromethane, trichloromethane, an ether, e.g. tetrahydrofuran, dioxane; or a dipolar aprotic solvent, e.g. N,N-dimethylformamide, N,N-dimethylacetamide and the like solvents.

The cyano compounds (L is -CN) may be obtained by dehydration of the corresponding aminocarbonyl compounds. Suitable dehydrating agents for this procedure known in the art are, for example, pentachlorophosphorane, phosphoryl chloride, thionyl chloride, phosphorus pentoxide, anhydrides such as acetic acid anhydride, trifluoroacetic acid anhydride and the like agents. The reaction temperature depends mainly on the nature of the chosen dehydrating agent but in general it is contemplated that the process can conveniently be carried out at temperatures comprised between room temperature and the boiling point of the reaction mixture in particular between +20°C and +120°C. If desired, said dehydration reaction can be run in inert organic solvents such as, for example, a halogenated hydrocarbon, e.g. dichloromethane, trichloromethane, an ether, e.g. tetrahydrofuran, dioxane; or a polar aprotic solvent, e.g. N,N-dimethylformamide, N,N-dimethylacetamide and the like solvents.

The compounds of formula (I) wherein L is a radical

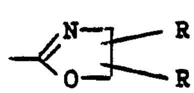

can be obtained by cyclizing the corresponding compounds of formula (I) wherein L is a radical

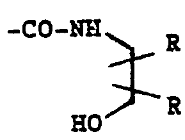

with an appropriate deshydrating agent such as phosphorous trichloride, phosphoryl chloride and in particular thionyl chloride. The said cyclization is preferably conducted at room temperature although in some instances increased temperatures may be recommendable. The starting hydroxylalkylamides can be

prepared by an appropriate amidation reaction as described hereinabove.

The compounds of formula (I) wherein L is an optionally substituted 1H-benzimidazolyl radical can be obtained by condensing the corresponding compounds wherein L is -CHO with a 1,2-diaminobenzene derivative in the presence of a suitable oxidant. The latter may in particular be nitrobenzene which concomitantly may act as a solvent. The said cyclization is conducted at a temperature in the range of +20°C to the boiling point of the reaction mixture. The starting aldehydes are conveniently prepared by reducing the corresponding alkyl esters with lithium aluminum hydride in tetrahydrofuran and subsequently oxidizing the thus obtained alcohol with manganese dioxide in acetone to the desired aldehyde.

The tetrazoles can be obtained by reacting the corresponding nitriles (L is -CN) with an azide in a reaction-inert organic solvent such as N,N-dimethylformamide or dimethyl sulfoxide, at temperatures between +20°C and +150°C.

The other heterocyclic compounds of formula (I), wherein L is

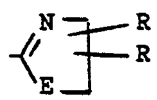 , 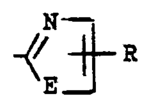 , 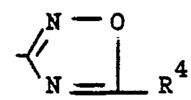 ,

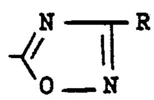 or 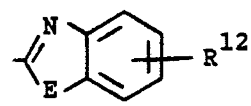 ,

are synthesized by treatment of the corresponding carboxylic acids (L is -COOH) or the derivatives thereof, for instance the acid chlorides, imino ethers, imino thioethers, amidines or amidoximes; with reagents such as diamines, aminoalcohols, aminomercaptans, amidoximes, α-haloketones, α-haloaldehydes, acid halogenides or carboxylic acid anhydrides. Reactions of this type are known in the art. For example general procedures are described in Chemistry of Carbon Compounds, Vol IV, Elsevier Publ. Co., 1957, in Heterocyclic Compounds, Wiley, N.Y., Vol. 5 (1957), Vol. 6 (1957), Vol. 7 (1961) and in the references cited therein.

The sulfur-containing compounds of formula (I) (L is $-CS-D-R^2$ and $-CS-O-R^5$) can be produced by treating the correspondig oxygen-containing compounds of formula (I) (L is $-CO-D-R^2$) with phosphorus pentasulfide or with 2,4-bis(4-methoxyphenyl)-2,4-disulfide-1,3,2,4-dithiadiphosphetane (Lawesson's reagent). Preferentially, this reaction is carried out in the presence of a base and in an organic solvent such as N,N-dimethylformamide, N,N-dimethylacetamide, hexamethylphosphoric triamide, acetonitrile, methylbenzene or dimethylbenzene.

The amidoximes of formula (I) (L is $-C(NH^2)=N-O-R$) can be obtained by reacting the nitriles (L is -CN) with hydroxylamine and optionally alkylating the resulting compound (L is $-C(NH_2)=N-OH$) by treatment with an alkylating agent.

The amidines of formula (I) [L is $-C(=NR)-NH-R^2$] can be obtained by first converting a nitrile or nitrilium salt, prepared by treatment of the nitrile with $R_3O^+BF_4^-$ , into an imino ether [L is $-C(=NR)-O-C_1-C_5$alkyl] by reaction with a $C_1$-$C_5$alkanol in the presence of an acidic or basic catalyst and subsequently reacting the imino ether with an amine $R^2-NH_2$.

Y being $-S-CH_2-$may be converted to the corresponding sulfoxide or sulfone by an appropriate oxidation procedure, e.g. with a peroxide or a periodate.

If the synthesis of stereochemically pure isomers is intended, stereoselective reaction steps and conditions are recommended. On the other hand conventional methods of separation can be used for obtaining pure isomers from a mixture of stereochemical isomers.

The starting materials for the preparation of the novel compounds of formula (I) are known, or they can be obtained by known methods of synthesis.

For example the compounds of formula (II) can be obtained by reacting an aminomethylene derivative of formula (VI).

$X-NH-CH_2-L$ (VI)

wherein L and X are as defined under formula (I), with formic acid in the presence of acetic anhydride. In turn, the compounds of formula (VI) can be prepared by reacting an amine of formula (VII)

$X-NH_2$ (VII)

wherein X is as defined under formula (I), with a bromomethylene derivative of formula

$Br-CH_2-L$ (VIII)

wherein L is as defined under formula (I), in the presence of an appropriate base, such as sodium carbonate.

The 4-amino-1H-imidazole derivatives of formula (IV) can be obtained by cyclizing an intermediate of formula

$NC\text{-}N=CH\text{-}N(X)\text{-}CH_2\text{-}L$ (IX)

wherein X and L are as defined hereinabove under catalysis of a base at elevated temperature in a suitable solvent, e.g. an alcohol. A preferred mode of carrying out said cyclization may comprise the reaction of the starting compound (IX) in an alcohol, in the presence of a catalytic amount of alkoxide obtained by dissolving an alkali metal in said alcohol, at the boiling point of the reaction mixture. Or, alternatively, by reacting (IX) with an alkali metal alkoxide in a polar solvent such as N,N-dimethylformamide or dimethyl sulfoxide. Generally, the reaction temperatures are in the range of +60°C to +140°C.

The intermediates of formula (IX) in turn can be prepared by alkylating an amidine of formula

NC-N=CH-NH-X (X)

wherein X is as defined under formula (I) with a bromomethylene derivative of formula (VIII), in the presence of an appropriate base, such as, for example an alkali metal hydroxide, an alkali or earth alkaline metal carbonate or hydrogen carbonate, an earth alkaline oxide, an alkali metal alkoxide or a trialkylamine, e.g. sodium hydroxide, potassium hydroxide, sodium carbonate, potassium hydrogen carbonate, magnesium oxide, calcium oxide, sodium methoxide, sodium ethoxide, potassium ethoxide, potassium isopropoxide, pyridine, N,N-diethylethanamine and the like. In some instances, the addition of a crown-ether may be recommendable. The reaction may conveniently be conducted at temperatures between +10°C and the boiling point of the reaction mixture, either without a solvent or in a solvent such as N,N-dimethylformamide, N,N-dimethylacetamide or dimethyl sulfoxide.

The compounds of formula (X) can be prepared by reacting an amine of formula (VII) with a $C_{1\text{-}5}$alkyl-N-cyanomethanimidate of formula

$C_{1\text{-}5}$alkyl-O-CH=N-CN (XI)

in an appropriate reaction-inert solvent such as trichloromethane, tetrahydrofuran, 1,4-dioxane, acetronitrile, N,N-dimethylformamide or N,N-dimethylacetamide. The said reaction can conveniently be carried out at temperatures between room temperature and the boiling point of the reaction mixture, in particular between +20°C and +80°C. Removal of the $C_{1\text{-}5}$alkanol which is liberated during the course of the reaction and of the solvent by destillation under reduced pressure yields the N-cyanoamidine of formula (X) which in general need not be purified before further convertion.

The 4-amino-1H-imidazole derivatives of formula (IV) can alternatively be obtained from the amines of formula (VII), by a combined N-alkylation and cyclization reaction in a one-pot procedure. The latter procedure is conducted in the same solvents and bases as mentioned hereinabove for the two step synthesis.

The amines of formula (VII) can be obtained by the reduction of an oxime of formula

$X^1$=N-OH (XII),

wherein $X^1$ is a geminal bivalent radical obtained by abstracting a further hydrogen atom from the carbon atom which links the X-radical with the 1H-imidazole-5-carboxylic acid group. Said reduction is conveniently conducted with hydrogen in the presence of a noble metal catalyst or with a metallic hydride reagent, e.g. lithium tetrahydroaluminate or diborane in a suitable reaction-inert solvent such as an ether, e.g. tetrahydrofuran, 1,4-dioxane and the like. The oxime of formula (XII) may also be reduced electrochemically.

Said oxime (XII) in turn, can be prepared from the corresponding ketone of formula

$X^1$=O (XIII),

wherein $X^1$ is as defined hereinabove, by reacting said ketone of formula (XIII) with hydroxylamine.

The amines of formula (VII) can also be prepared by the reductive amination of a ketone of formula (XIII) with formamide in the presence of formic acid and subsequent removal of the N-formyl group by treatment with a hydrohalic acid, e.g. hydrochloric acid.

The intermediates of formula (VI) can also be obtained by the reductive N-alkylation reaction of a ketone of formula (XIII) with an aminomethylene derivative (XIV), wherein $X^1$ and L are as defined hereinabove.

$$\underset{(XIII)}{X^1{=}O} + \underset{(XIV)}{H_2N\text{-}CH_2\text{-}L} \xrightarrow{\text{reductive } N\text{-alkylation}} \underset{(VI)}{X\text{-}NH\text{-}CH_2\text{-}L}$$

Said reductive N-alkylation reaction may conveniently be carried out by hydrogenating a stirred and, if desired, heated mixture of the reactants in a suitable reaction-inert organic solvent according to art-known catalytic hydrogenating procedures. Suitable solvents are, for example, alkanols, e.g. methanol, ethanol: ethers, such as tetrahydrofuran. The term "art-known catalytic hydrogenating procedures" means that the reaction is carried out under hydrogen atmosphere and in the presence of a catalyst such as, palladium-on-charcoal, platinum-on-charcoal and the like. In order to prevent the undesired further hydrogenation of certain functional groups in the reactants and the reaction products it may be advantageous to add an appropriate catalyst-poison to the reaction mixture, e.g. thiophene.

Alternatively, said reductive N-alkylation reactions may be conducted by treating a stirred and, if desired, heated mixture of the reactants with sodium cyanoborohydride, sodium borohydride, formic acid or a salt thereof, e.g. ammonium formiate.

The activated derivatives of formula (V), wherein X is other than unsubstituted 1-indanyl, 1,2,3,4-tetrahydro--1-naphthalenyl and other than a group -CH(A)Z, A being unsubstituted $C_1$-$C_5$alkyl and Z being phenyl, pyridinyl or thienyl, said phenyl being unsubstituted or substituted with up to three substituents selected from $C_1$-$C_5$alkyl, $C_1$-$C_5$alkyloxy or halo, when $R^1$ is hydrogen, G is oxygen and T is chloro or bromo, the salts and stereoisomeric forms thereof, are deemed novel and have especially been developed for the synthesis of the compounds of formula (I) and therefore constitute a further aspect of the present invention.

The carboxylic acids and corresponding $C_1$-$C_6$alkyl esters, which are structurally related to the activated derivatives of formula (V) are known from the Publ. Eur. Pat. Appln. Nos. 0,207,563, 0,234,656 and 0,240,050 and U.S. Patent Nos. 3,354,173; 3,336,326; 4,038,286; 3,991,072 .

The compounds of formula (I) are stable compounds and no precautionary measures are required for handling them. When used at the indicated rates of application, the compounds of formula (I) have good selective herbicidal properties which make them most suitable for use in crops of useful plants, in particular in sugar beet, soy beans, cereals and preferably in rice and maize. In some cases damage is also caused to weeds which up to now have only been controlled with total herbicides. At higher rates of application, all tested plants are so severely damaged in their development that they die.

The invention also relates to herbicidal compositions containing one or more inert carriers and, if desired, other adjuvants and as active ingredient a herbicidally effective amount of a compound of formula (I) as defined hereinabove and a process for preparing said compositions comprising the intimate admixture of the active ingredient with the carrier or carriers and, if desired, other adjuvants. Further the invention relates to methods of controlling weeds by the application of the novel compounds.

In the method for controlling weeds according to the invention the compounds of formula (I) are used in unmodified form or, preferably, together with the adjuvants conventionally employed in the art of formulation. They are therefore formulated following art-known procedures to emulsifiable concentrates, directly sprayable or dilutable solutions, dilute emulsions, wettable powders, soluble powders, dusts, granulates, and also encapsulations in e.g. polymer substances. As with the nature of the compositions, the methods of application, such as spraying, atomising, dusting, scattering or pouring, are chosen in accordance with the intended objectives and the prevailing circumstances.

The formulations, i.e. the compositions, preparations or mixtures containing the compound (active ingredient) of formula (I) and, where appropriate, a solid or liquid adjuvant, are prepared in known manner, e.g. by homogeneously mixing and/or grinding the active ingredients with extenders, e.g. solvents, solid carriers and, where appropriate, surface-active compounds (surfactants).

Suitable solvents are aromatic hydrocarbons, preferably the fractions containing 8 to 12 carbon atoms, e.g. dimethylbenzene mixtures or substituted naphthalenes, phthalates such as dibutyl phthalate or dioctyl phthalate, aliphatic or alicyclic hydrocarbons such as cyclohexane or paraffins, alcohols and glycols and their ethers and esters, such as ethanol, ethylene glycol, ethylene glycol monomethyl or monoethyl ether, ketones such as cyclohexanone, strongly polar solvents such as N-methyl-2-pyrrolidone, dimethylsulfoxide or dimethylformamide, as well as vegetable oils or epoxidised vegetable oils such as epoxidised coconut oil or soybean oil; or water.

The solid carriers used e.g. for dusts and dispersible powders are normally natural mineral fillers such as calcite, talcum, kaolin, montmorillonite or attapulgite. In order to improve the physical properties it is also possible to add highly dispersed silicic acid or highly dispersed absorbent polymers. Suitable granulated adsorptive carriers are of the porous type, for example pumice, broken brick, sepiolite or bentonite; and suitable nonsorbent carriers are materials such as calcite or sand. In addition, a great number of pregranulated materials of inorganic or organic nature can be used, e.g. especially dolomite or pulverised plant residues.

Depending on the nature of the compound of formula (I) to be formulated, suitable surface-active compounds are non-ionic, cationic and/or anionic surfactants having good emulsifying, dispersing and

wetting porperties. The term "surfactants" will also be understood as comprising mixtures of surfactants.

Suitable anionic surfactants can be both water-soluble soaps and water-soluble synthetic surface-active compounds.

Suitable soaps are the alkali metal salts, earth alkaline metal salts or unsubstituted or substituted ammonium salts of higher fatty acids ($C_{10}$-$C_{22}$), e.g. the sodium or potassium salts of oleic or stearic acid, or of natural fatty acid mixtures which can be obtained e.g. from coconut oil or tallow oil. In addition, there may also be mentioned fatty acid methyltaurin salts.

More frequently, however, so-called synthetic surfactants are used, especially fatty sulfonates, fatty sulfates, sulfonated benzimidazole derivatives or alkylarylsulfonates.

The fatty sulfonates or sulfates are usually in the form of alkali metal salts, earth alkaline metal salts or unsubstituted or substituted ammonium salts and contain an alkyl radical having from 8 to 22 carbon atoms which also includes the alkyl moiety derived from acyl radicals, e.g. the sodium or calcium salt of lignosulfonic acid, of dodecylsulfate or of a mixture of fatty alcohol sulfates obtained from natural fatty acids. These compounds also comprise the salts of sulfuric acid esters and sulfonic acids of fatty alcohol/ethylene oxide adducts. The sulfonated benzimidazole derivatives preferably contain 2 sulfonic acid groups and one fatty acid radical containing 8 to 22 carbon atoms. Examples of alkylarylsulfonates are the sodium, calcium or triethanolamine salts of dodecylbenzene sulfonic acid, dibutylnaphthalenesulfonic acid, or of a naphthalenesulfonic acid/formaldehyde condensation product. Also suitable are corresponding phosphates, e.g. salts of the phosphoric acid ester of an adduct of p-nonylphenol with 4 to 14 moles of ethylene oxide, or phospholipids.

Non-ionic surfactants are preferably polyglycol ether derivatives of aliphatic or cycloaliphatic alcohols, or saturated or unsaturated fatty acids and alkylphenols, said derivatives containing 3 to 10 glycol ether groups and 8 to 20 carbon atoms in the (alifatic) hydrocarbon moiety and 6 to 18 carbon atoms in the alkyl moiety of the alkylphenols.

Further suitable non-ionic surfactants are the water-soluble adducts of polyethylene oxide with polypropylene glycol, ethylenediaminopolypropylene glycol containing 1 to 10 carbon atoms in the alkyl chain, which adducts contain 20 to 250 ethylene glycol ether groups and 10 to 100 propylene glycol ether groups. These compounds usually contain 1 to 5 ethylene glycol units per propylene glycol unit.

Representative examples of non-ionic surfactants are nonylphenolpolyethoxyethanols, castor oil polyglycol ethers, polypropylene/polyethylene oxide adducts, tributylphenoxypolyethoxyethanol, polyethylene glycol and octylphenoxypolyethoxyethanol.

Fatty acid esters of polyethylene sorbitan, such as polyoxyethylene sorbitan trioleate, are also suitable non-ionic surfactants.

Cationic surfactants are preferably quaternary ammonium salts which contain, as N-substituent, at least one $C_8$-$C_{22}$alkyl radical and, as further substituents, unsubstituted or halogenated lower alkyl, benzyl or hydroxy-lower alkyl radicals. The salts are preferably in the form of halides, methylsulfates or ethylsulfates, e.g. stearyltrimethylammonium chloride or benzyldi(2-chloroethyl)ethylammonium bromide.

The surfactants customarily employed in the art of formulation are described e.g. in the following publications:
"McCutcheon's Detergents and Emulsifiers Annual", MC Publishing Corp., Ridgewood, New Jersey, 1981; H. Stache, "Tensid-Taschenbuch", 2nd Edition, C. Hanser Verlag, Munich & Vienna, 1981, M. and J. Ash, "Encyclopedia of Surfactants", Vol. I-III, Chemical Publishing Co., New York, 1980-81.

The herbicidal compositions which are preferably employed in the method of the invention usually contain 0.1 to 95%, preferably 0.1 to 80%, of a compound of formula (I), 1 to 99.9%, of a solid or liquid adjuvant, and 0 to 25%, preferably 0.1 to 25%, of a surfactant.

Preferred formulations are composed in particular of the following constituents (% = percentage by weight):

Emulsifiable concentratesactive ingredient: 1 to 20%, preferably 5 to 10%
surfactant: 5 to 30%, preferably 10 to 20%
liquid carrier: 50 to 94%, preferably 70 to 85%

Dustsactive ingredient: 0.1 to 10%, preferably 0.1 to 1%
solid carrier: 99.9 to 90%, preferably 99.9 to 99%

Suspension concentratesactive ingredient: 5 to 75%, preferably 10 to 50%
water: 94 to 25%, preferably 88 to 30%
surfactant: 1 to 40%, preferably 2 to 30%

Wettable powdersactive ingredient: 0.5 to 90%, preferably 1 to 80%
surfactant: 0.5 to 20%, preferably 1 to 15%
solid carrier: 5 to 95%, preferably 15 to 90%

Granulatesactive ingredient: 0.5 to 30%, preferably 3 to 15%
solid carrier: 99.5 to 70%, preferably 97 to 85%
The following examples are intended to illustrate and not to limit the scope of the present invention. Unless otherwise stated all parts therein are by weight.

EXPERIMENTAL PART

A. Preparation of Intermediates

Example 1

a) A mixture of 115 parts of 2,3-dihydro-2-propyl-4H-1-benzopyran-4-one, 80 parts of methyl glycine, 15 parts of potassium fluoride and 800 parts of methanol was hydrogenated at normal pressure and at room temperature with 5 parts of palladium-on-charcoal catalyst 10%. After the calculated amount of hydrogens was taken up, the catalyst was filtered off and the filtrate was evaporated. The residue was taken up in 1,1'-oxybisethane. The organic layer was washed with water and a sodium hydroxide solution 5%, dried, filtered and evaporated, yielding 97 parts (73.0%) of methyl (cis + trans)-N-(3,4-dihydro-2-propyl-2H-1-benzopyran-4-yl)glycine as an oily residue (interm. 1).

b) A mixture of 97 parts of methyl (cis + trans)-N-(3,4-dihyrdo-2-propyl-2H-1-benzopyran-4-yl)glycine, 360 parts of formic acid and 100 parts of acetic acid anhydride was stirred overnight at room temperature. The reaction mixture was evaporated and the residue was taken up in dichloromethane. The organic layer was washed with water, a sodium hydroxide solution and a hydrochloric acid solution 5%, dried, filtered and evaporated, yielding 78 parts (76.4%) of methyl (cis + trans)-N-(3,4-dihydro-2-propyl-2H-1-benzopyran-4-yl)-N-formylglycine as a residue (interm. 2).

c) To a stirred solution of 76 parts of methyl (cis + trans)-N-(3,4-dihydro-2-propyl-2H-1-benzopyran-4-yl)-N-formylglycine in 188 parts of tetrahydrofuran were added 13 parts of a sodium hydride dispersion 50%. After stirring for 1 hour at room temperature, 75 parts of methyl formate were added. After stirring overnight at room temperature, the precipitated product was filtered off and dissolved in 160 parts of methanol, 100 parts of water and 36 parts of concentrated hydrochloric acid. A solution of 60 parts of potassium thiocyanate in 30 parts of water was added and the whole was stirred overnight at room temperature. The separated oil was dried, yielding 56 parts (33.6%) of methyl (cis + trans)-1-(3,4-dihydro-2-propyl-2H-1-benzopyran-4-yl)-2-mercapto-1H-imidazole-5-carboxylate as an oily residue (interm. 3).

d) 56 parts of methyl (cis + trans)-1-(3,4-dihydro-2-propyl-2H-1-benzo pyran-4-yl)-2-mercapto-1H-imidazole-5-carboxylate were dissolved in 200 parts of a nitric acid solution 10%. After intense reaction, the mixture was cooled into crushed ice and treated with a sodium hydroxide solution. The product was extracted with dichloromethane. The extract was washed with water, dried, filtered and evaporated. The residue was purified by column chromatography (HPLC) over silica gel using a mixture of hexane and ethyl acetate (90:10 by volume) as eluent. The first fraction was collected and the eluent was evaporated. The residue was crystallized from 1,1'-oxybisethane. The product was filtered off and dried, yielding 4.6 parts (9.0%) of methyl cis-1-(3,4-dihydro-2-propyl-2H-1-benzopyran-4-yl)-1H-imidazole-5-carboxylate; mp. 106.5°C (interm. 4).
The second fraction was collected and the eluent was evaporated. The residue was converted into the nitrate salt in 2-propanone and 1,1'-oxybisethane. The salt was filtered off and dried, yielding 10.1 parts (16.3%) of methyl trans-1-(3,4-dihydro-2-propyl-2H-1-benzopyran-4-yl)-1H-imidazole-5-carboxylate mononitrate; mp 150.2°C (interm. 5).

Example 2

a) A mixture of 79.5 parts of 6-fluoro-2,3-dihydro-2-methyl-4H-1-benzopyran-4-one, 63 parts of hydroxylamine hydrochloride and 378 parts of pyridine was stirred overnight at reflux temperature. The reaction mixture was poured into water. The precipitated product was filtered off and dried in vacuo at 60°C, yielding 69.5 parts (80.9%) of 6-fluoro-2,3-dihydro-2-methyl-4H-1-benzopyran-4-one,oxime; mp. 142.5°C (interm. 6).

b) A mixture of 68.5 parts of 6-fluoro-2,3-dihydro-2-methyl-4H-1-benzopyran-4-one,oxime and 480 parts of methanol, saturated with ammonia was hydrogenated at normal pressure and at room temperature with 6 parts of Raney nickel catalyst. After the calculated amount of hydrogen was taken up, the catalyst was filtered off and the filtrate was evaporated, yielding 62 parts (97.8%) of 6-fluoro-3,4-dihydro-2-methyl-2H-1-benzopyran-4-amine as a residue (interm. 7).

c) A mixture of 62 parts of 6-fluoro-3,4-dihydro-2-methyl-2H-1-benzopyran-4-amine, 53 parts of methyl 2-bromoacetate, 38 parts of N,N-diethylethanamine and 52 parts of N,N-dimethylformamide was stirred overnight at room temperature. After the addition of dichloromethane, the precipitate was filtered off. The filtrate was washed four times with water, dried, filtered and evaporated. The residue was purified by column chromatography (HPLC) over silica gel using a mixture of hexane and ethyl acetate (80:20 by volume) as eluent. The desired fraction was collected and the eluent was evaporated, yielding 35.7 parts (43.7%) of methyl trans-N-(6-fluoro-3,4-dihydro-2-methyl-2H-1-benzopyran-4-yl)glycine as a residue (interm. 8).

d) A mixture of 35.7 parts of methyl trans-N-(6-fluoro-3,4-dihydro-2-methyl-2H-1-benzopyran-4-yl)-glycine, 128 parts of formic acid and 30 parts of acetic acid anhydride was combined while cooling in an ice bath and the whole was stirred overnight at room temperature. After the addition of water, the product was extracted with dichloromethane. The extract was washed with water and twice with a sodium carbonate solution, dried, filtered and evaporated, yielding 36.7 parts (93.1%) of methyl trans-N-(6-fluoro-3,4-dihydro-2-methyl-2H-1-benzopyran-4-yl)-N-formylglycine as a residue (interm. 9).

e) To a stirred solution of 36.7 parts of methyl trans-N-(6-fluoro-3,4-dihydro-2-methyl-2H-1-benzopyran-4-yl)-N-formylglycine in 225 parts of tetrahydrofuran were added portionwise 6.5 parts of a sodium hydride dispersion 50%. Upon completion, 22.1 parts of methyl formate were added and the whole was stirred overnight at 60°C. After cooling, the precipitated product was filtered off, washed with petroleum ether and combined with 104 parts of methanol, a solution of 36 parts of concentrated hydrochloric acid in 100 parts of water and a solution of 17.3 parts of potassium thiocyanate in 40 parts of water. The whole was stirred overnight at 60°C. After cooling, the product was filtered off and dried in vacuo at 80°C, yielding 28 parts (62.0%) of methyl trans-1-(6-fluoro-3,4-dihydro-2-methyl-2H-1-benzopyran-4-yl)-2-mercapto-1H-imidazole-5-carboxylate (interm. 10).

f) A mixture of 28 parts of methyl trans-1-(6-fluoro-3,4-dihydro-2-methyl-2H-1-benzopyran-4-yl)-2-mercapto-1H-imidazole-5-carboxylate, 0.1 parts of sodium nitrite, 52.5 parts of concentrated nitric acid and 90 parts of water was stirred for 2 hours at room temperature. After the addition of crushed ice, the whole was treated with a sodium hydroxide solution. The product was extracted with dichloromethane. The extract was dried, filtered and evaporated. The residue was crystallized from acetonitrile. The product was filtered off (the filtrate was set aside) and dried in vacuo at 60°C, yielding a first fraction of 15 parts (59.3%) of methyl trans-1-(6-fluoro-3,4-dihydro-2-methyl-2H-1-benzopyran-4-yl)-1H-imidazole-5-carboxylate mononitrate; mp. 124.6°C (interm. 11). The filtrate, which was set aside (see above) was evaporated, yielding a second fraction of interm. 11. Total yield: 23.6 parts (93.3%) of interm. 11.

Example 3

a) To a stirred mixture of 100 parts of 5-chloro-2,3-dihydro-1H-inden-1-one, 241 parts of iodomethane and 360 parts of 2-methyl-2-propanol are added portionwise 148 parts of 2-methyl-2-propanol, potassium salt at <50°C under nitrogen atmosphere. Upon complete addition, stirring is continued overnight at room temperature. The precipitate is filtered off, washed with dichloromethane and the filtrate is evaporated. The residue is distilled at 13.30 Pa, yielding 84 parts (71.9%) of 5-chloro-2,3-dihydro-2,2-dimethyl-1H-inden-1-one as a residue (interm. 12).

b) To a stirred and heated (130°C) mixture of 90 parts of formamide and 42 parts of formic acid are added dropwise 85 parts of 5-chloro-2,3-dihydro-2,2-dimethyl-1H-inden-1-one. Upon complete addition, stirring is continued for 12 hours at 150°C. After cooling, the reaction mixture is poured into water and the product is extracted with dichloromethane. The extract is dried, filtered and evaporated. The residue is

treated with 240 parts of concentrated hydrochloric acid and the whole is stirred for 1 hour at reflux temperature. After cooling, the whole is poured into crushed ice and treated with a sodium hydroxide solution. The product is extracted with dichloromethane. The extract is washed with water, dried, filtered and evaporated. Treatment of the residue with an acid base extraction, yields 33 parts (36.6%) of 5-chloro-2,3-dihydro-2,2-dimethyl-1H-inden-1-amine as an oily residue (interm. 13).

c) To a stirred solution of 32 parts of 5-chloro-2,3-dihydro-2,2-dimethyl-1H-inden-1-amine in 188 parts of N,N-dimethylformamide are added 17 parts of ethyl N-cyanomethanimidate. After stirring for 1 hour at 100°C, the mixture is cooled and the formed ethanol is distilled off in vacuo. A mixture of 25.3 parts of N-methyl α-bromoacetamide and 48 parts of potassium carbonate are added, together with a small amount of dodecahydro [1,4,7,10,13,16]hexaoxacyclooctadecin. The reaction mixture is stirred first for 1 hour at 80°C and then overnight at 130°C and then poured into 300 parts of water and the product extracted with ethyl acetate. The extract is washed twice with water, dried, filtered and evaporated. The residue is crystallized from 2,2'-oxybispropane. The product is filtered off and dried, yielding N-methyl 4-amino-1-(5-chloro-2,3-dihydro-2,2-dimethyl-1H-inden-1-yl)-1H-imidazole-5-carboxamide; (interm. 14).

## Example 4

a) A mixture of 80.3 parts of 2,3-dimethyl-4H-1-benzopyran-4-one and 480 parts of methanol was hydrogenated at normal pressure and at room temperature with 6 parts of Raney/nickel catalyst. After the calculated amount of hydrogen was taken up, the catalyst was filtered off and the filtrate was evaporated. The residue was crystallized from 2,2'-oxybispropane. The product was filtered off and dried in vacuo at 50°C, yielding 38 parts (46.3%) of 3,4-dihydro-2,3-dimethyl-2H-1-benzopyran-4-ol (interm. 15).

b) To stirred and cooled (ice bath) solution of 26 parts of chromium(VI) oxide in 90 parts of water were added 58.9 parts of concentrated sulfuric acid. The thus obtained solution was added dropwise to a solution of 38 parts of 3,4-dihydro-2,3-dimethyl-2H-1-benzopyran-4-ol in 248 parts of 2-propanone (exothermic reaction). Upon complete addition, stirring was continued for 4 hours at room temperature. The reaction mixture was poured into water and the product was extracted with trichloromethane. The extract was washed with water, dried, filtered and evaporated, yielding 36 parts (97.3%) of 2,3-dihydro-2,3-dimethyl-4H-1-benzopyran-4-one as a residue (interm. 16).
Following the procedures of example 2 the latter compound was converted to (±)-methyl (2α,3α,4β)-1-(3,4-dihydro-2,3-dimethyl-2H-1-benzopyran-4-yl)-1H-imidazole-5-carboxylate (interm. 17).

## Example 5

a) A solution of 20.4 parts of 2-[(1,2,3,4-tetrahydro-1-naphthalenyl)amino]acetonitrile and 11 parts of formic acid in 215 parts of dimethylbenzene was stirred and refluxed for 3 hours. The whole was washed successively with water, a sodium hydrogen carbonate solution, an ammonium hydroxide solution and water, dried, filtered and evaporated. The residue was crystallized from petroleumether. The product was filtered off and dried, yielding 13.4 parts (57%) of N-(cyanomethyl)-N-(1,2,3,4-tetrahydro-1-naphthalenyl)-formamide; mp. 100°C (interm. 18).

b) To a stirred mixture of 8.5 parts of sodium ethoxide in 450 parts of methylbenzene was added dropwise a solution of 13.4 parts of N-(cyanomethyl)-N-(1,2,3,4-tetrahydro-1-naphthalenyl)formamide in 19 parts of ethyl formate. Upon completion, stirring was continued overnight at room temperature. 200 Parts of water were added and the whole was stirred for 15 minutes. The layers were separated and the methylbenzene layer was extracted with water. The aqueous phase was acidified with a hydrochloric acid solution and the product was extracted three times with trichloromethane. The combined extracts were dried, filtered and evaporated, yielding 13 parts of N-(1-cyano-2-oxoethyl)-N-(1,2,3,4-tetrahydro-1-naphthalenyl)formamide as a residue (interm. 19).

## Example 6

a) To a stirred solution of 29.4 parts of methyl 1-(1,2,3,4-tetrahydro-2,2-dimethyl-1-naphthalenyl)-1H-imidazole-5-carboxylate in 180 parts of tetrahydrofuran was added dropwise 100 parts of a lithium tetrahydroaluminate solution 1 M in 1,1'-oxybisethane (exothermic reaction). Upon complete addition, stirring was continued for 2 hours at room temperature. After cooling, 30 parts of water were carefully added to the

mixture. Upon completion, the whole was stirred for 10 minutes at room temperature. The precipitate was filtered off and the filtrate was evaporated. The residue was dissolved in trichloromethane. The organic layer was washed with water, dried, filtered and evaporated. The residue was crystallized from acetonitrile. The product was filtered off and dried, yielding 22 parts (85.8%) of 1-(1,2,3,4-tetrahydro-2,2-dimethyl-1-naphthalenyl)-1H-imidazole-5-methanol; mp. 159.4°C (interm. 20).

b) To a stirred and heated solution of 19.4 parts of 1-(1,2,3,4-tetrahydro-2,2-dimethyl-1-naphthalenyl)-1H-imidazole-5-methanol in 560 parts of 2-propanone were added 100 parts of activated manganese(IV) oxide. After stirring for 1.5 hours at room temperature, the reaction mixture was filtered and the filtrate was evaporated. The residue was dissolved in dichloromethane. The organic layer was washed with a sodium hydroxide solution 5% and water, dried, filtered and evaporated. The residue was crystallized from acetonitrile. The product was filtered off and dried, yielding 16 parts (82.9%) of 1-(1,2,3,4-tetrahydro-2,2-dimethyl-1-naphthalenyl)-1H-imidazole-5-carboxaldehyde; mp. 138.6°C (interm. 21). All other intermediates are either known or can be obtained by analogous methods of preparation.

B. Preparation of final compounds

Example 7

20 Parts of 1-(2,3-dihydro-2,2-dimethyl-1H-inden-1-yl)-1H-imidazole-5-carboxylic acid was treated with 162 parts of thionyl chloride. After the evolution of gaseous hydrochloric acid and sulfur dioxide the excess thionyl chloride was destilled off. The residue was solved in 356 parts of tetrahydrofuran and 25 parts of methanamine was added to solution. The reaction mixture was stirred for 15 hours at a temperature between 20°C and 25°C. The precipitated product was collected and dried. Yield: 9.7 parts of 1-(2,3-dihydro-2,2-dimethyl-1H-inden-1-yl)-1H-imidazole-5-carboxylic acid methyl amide having a melting point of 100-100.5°C (compound 1.03).

Example 8

5.0 Parts of 1-(2,3-dihydro-2,2-dimethyl-1H-inden-1-yl)-1H-imidazole-5-carboxylic acid was refluxed during 1 hour in 32.4 parts of freshly distilled thionyl chloride. The excess thionyl chloride was destilled off and the residue was dissolved in 130 parts of dichloromethane. 5 Parts of 2-propanone, oxime and 5 parts of N,N-diethylethanamine were added. After stirring for 5 hours at room temperature the reaction mixture was washed with water and evaporated to dryness. The product was purified by column chromatography over silica gel using a mixture of hexane and ethyl acetate as eluent. The pure fractions were collected and the eluent was evaporated, yielding 5.1 parts of [(1-methylethylidene)amino] 1-(2,3-dihydro-2,2-dimethyl-1H-inden-1-yl)-1H-imidazole-5-carboxylate: mp. 81-83°C (compound 1.76).

Example 9

15 Parts of cyclopropanemethanol were cooled to about 10°C. While keeping this temperature, there were added portionwise 5 parts of dl-1-(1-phenylethyl)-1H-imidazole-5-carbonyl chloride hydrochloride. When all solid was dissolved, the whole was poured into 50 parts of water. The aqueous layer was alkalized with sodium hydroxide solution 5 N and extracted with ether. The extract was dried over magnesium sulfate, filtered and to the filtrate was added 2-propanol, previously saturated with gaseous hydrogen chloride. The precipitated salt was filtered off and dissolved in a small amount of 2-propanol. To the warm solution was added anhydrous ether until a turbid solution was obtained. After keeping for a few hours at room temperautre, the solid was filtered off and dried in vacuo, yielding 3.5 parts of (cyclopropylmethyl) dl-1-(1-phenylethyl)-1H-imidazole-5-carboxylate hydrochloride; mp. 148.5-151°C (compound 13.95).

Example 10

To a stirred and heated (±40°C) solution of 5 parts of 1-(2,3-dihydro-3,3-dimethyl-1H-inden-1-yl)-1H-imidazole-5-carboxylic acid in 45 parts of tetrahydrofuran were added 3.2 parts of 1,1′-carbonylbis-[1H-imidazole]. After stirring for 1 hour at 40°C, 1 part of gaseous methanamine was added to the thus obtained

mixture. The whole was stirred overnight at room temperature. The reaction mixture was evaporated and the residue was taken up in water. The product was extracted with trichloromethane. The extract was washed with water, dried, filtered and evaporated. The residue was purified by column chromatography over silica gel using a mixture of trichloromethane and methanol (95:5 by volume) as eluent. The pure fractions were collected and the eluent was evaporated. The residue was crystallized from acetonitrile. The product was filtered off and dried, yielding 3.54 parts (67.3%) of 1-(2,3-dihydro-3,3-dimethyl-1H-inden-1-yl)-N-methyl-1H-imidazole-5-carboxamide; mp. 167.4°C (compound 1.67).

## Example 11

To a stirred and heated solution of 4.0 parts of 1-(1,2,3,4-tetrahydro-2,2-dimethyl-1-naphthalenyl)-1H-imidazole-5-carboxylic acid in 67.5 parts of tetrahydrofuran were added 2.4 parts of 1,1'-carbonylbis-[1H-imidazole]. The whole was stirred for 2 hours at room temperature. The reaction mixture was evaporated and the residue was taken up in water. The product was extracted with trichloromethane. The extract was dried, filtered and evaporated. The residue was crystallized from 2,2'-oxybispropane. The product was filtered off and dried, yielding 3.27 parts (68.0%) of 1-[[1-(1,2,3,4-tetrahydro-2,2-dimethyl-1-naphthalenyl)-1H-imidazol-5-yl]carbonyl]-1H-imidazole; mp. 122.4°C (compound 2.96).

## Example 12

Through a stirred solution of 50 parts of 1-[[1-(1,2,3,4-tetrahydro-2,2-dimethyl-1-naphthalenyl)-1H-imidazol-5-yl]carbonyl]-1H-imidazole in 225 parts of tetrahydrofuran was bubbled gaseous ammonia during 2 hours. The reaction mixture was allowed to stand overnight at room temperature. The precipitated product was filtered off, washed with tetrahydrofuran and 2,2'-oxybispropane and suspended in acetonitrile. The precipitated product was filtered off and crystallized from ethanol. The product was filtered off and dried, yielding 26.6 parts (63.3%) of 1-(1,2,3,4-tetrahydro-2,2-dimethyl-1-naphthalenyl)-1H-imidazole-5-carboxamide; mp. 238.2°C (compound 2.25).

Following the same procedure as in example 12 there is also prepared: 1-(1,2,3,4-tetrahydro-1-naphthalenyl)-1H-imidazole-5-carboxamide; mp. 207-208°C (compound 2.08)

## Example 13

To a stirred mixture of 4 parts of 1-[[1-(1,2,3,4-tetrahydro-2,2-dimethyl-1-naphthalenyl)-1H-imidazol-5-yl]-carbonyl]-1H-imidazole and 48 parts of N,N-dimethylformamide were added 1.2 parts of methanethiol. After stirring for 12 hours at room temperature, the reaction mixture was evaporated. The residue was taken up in water and the product was extracted with trichloromethane. The extract was dried, filtered and evaporated. The residue was purified by column chromatography over silica gel using a mixture of trichloromethane and methanol (99:1 by volume) as eluent. The pure fractions were collected and the eluent was evaporated. The oily residue was solidified in 2,2'-oxybispropane. The product was filtered off and dried, yielding 2.28 parts (60.9%) of S-methyl 1-(1,2,3,4-tetrahydro-2,2-dimethyl-1-naphthalenyl)-1H-imidazole-5-carbothioate; mp. 80.7°C (compound 2.30).

## Example 14

To a stirred solution of 9.7 parts of 1-(1,2,3,4-tetrahydro-1-naphthalenyl)-1H-imidazole-5-carboxylic acid in 90 parts of N,N-dimethylformamide were added 1.9 parts of a sodium hydride dispersion 50%. After stirring for 15 minutes at room temperature, 3.3 parts of chloroacetonitrile were added dropwise to the thus obtained mixture. Upon complete addition, stirring was continued for 18 hours at room temperature. The reaction mixture was evaporated and the residue was taken up in water. The product was extracted with trichloromethane. The extract was dried, filtered and evaporated. The residue was purified by column chromatography over silica gel using a mixture of trichloromethane and methanol (95:5 by volume) as eluent. The pure fractions were collected and the eluent was evaporated. The residue was crystallized from 2,2'-oxybispropane. The product was filtered off and dried, yielding 7.4 parts (66.1%) of (cyanomethyl) 1-(1,2,3,4-tetrahydro-1-naphthalenyl)-1H-imidazole-5-carboxylate; mp. 109.6°C (compound 2.118).

Example 15

A mixture of 27.4 parts of 2-bromo-2-methylpropane and 100 parts of dimethyl sulfoxide was stirred for 1 hour at room temperature. After the addition of 4.8 parts of 1-(1,2,3,4-tetrahydro-1-naphthalenyl)-1H-imidazole-5-carboxylic acid and 16.8 parts of sodium hydrogen carbonate, stirring was continued overnight at room temperature. The reaction mixture was poured into 600 parts of water and the product was extracted with 1,1'-oxybisethane. The extract was dried, filtered and evaporated. The residue was purified by column chromatography over silica gel using trichloromethane as eluent. The pure fractions were collected and the eluent was evaporated. The residue was crystallized from 2,2'-oxybispropane. The product was filtered off and dried, yielding 3.3 parts (54.6%) of [(methylthio)methyl] 1-(1,2,3,4-tetrahydro-1-naphthalenyl)-1H-imidazole-5-carboxylate; mp. 99.4°C (compound 2.120).

Example 16

To a stirred mixture of 4.3 parts of (R)-(+)-1-(1-phenylethyl)-1H-imidazole-5-carboxylic acid and 80 parts of methanol were added 0.5 parts of sodium (exothermic reaction). After stirring for 30 minutes, the solvent was evaporated and the residue was suspended in 80 parts of acetonitrile. Then there were added 2.8 parts of 1-chloro-2-propanone and the whole was stirred and refluxed for 4 hours. The reaction mixture was cooled, filtered and the filtrate was evaporated. The residue was purified by column chromatography over silica gel using a mixture of trichloromethane and methanol (97.5:2.5 by volume) as eluent. The pure fractions were collected and the eluent was evaporated. The residue was converted into the hydrochloride salt in 2-propanone and 2-propanol. The salt was filtered off and dried, yielding 4 parts (64%) of (R)-(+)-2-oxopropyl 1-(1-phenylethyl)-1H-imidazole-5-carboxylate monohydrochloride; mp. 149.7°C $[\alpha]_D$ = +42.02° (c = 0.1% in ethanol) (compound 13.98).

Example 17

A mixture of 4 parts of methyl (2α,3α4β)-1-(3,4-dihydro-2,3-dimethyl-2H-1-benzopyran-4-yl)-1H-imidazole-5-carboxylate and 80 parts of an aqueous methanamine solution 40% was stirred for 4 hours at room temperature. The product was extracted with trichloromethane. The extract was dried, filtered and evaporated. The residue was converted into the nitrate salt in a mixture of 2-propanone and 1,1'-oxybisethane. The salt was filtered off and dried in vacuo at 60°C, yielding 3.6 parts (73.8%) of (2α,3α,4β)-1-(3,4-dihydro-2,3-dimethyl-2H-1-benzopyran-4-yl)-N-methyl-1H-imidazole-5-carboxamide mononitrate; mp. 189.5°C (compound 6.93).

Example 18

A solution of 12.5 parts of methyl 1-(1,2,3,4-tetrahydro-2,2-dimethyl-1-naphthalenyl)-1H-imidazole-5-carboxylate in 30 parts of a sodium hydroxide solution 50% and 30 parts of water was boiled for 1 hour. After cooling, the reaction mixture was acidified and the product was extracted with trichloromethane. The extract was washed with water, dried, filtered and evaporated. The residue was taken up in 90 parts of tetrahydrofuran and 6.4 parts of 1,1'-carbonylbis[1H-imidazole] were added. After stirring for 15 minutes at room temperature, 18 parts of methanamine were added at once and stirring was continued for 1 hour at this temperature. The tetrahydrofuran layer was evaporated and the residue was taken up in dichloromethane. The organic layer was washed with a sodium hydroxide solution 5% and water, dried, filtered and evaporated. The residue was purified by column chromatography over silica gel using a mixture of trichloromethane and methanol (90:10 by volume) as eluent. The pure fractions were collected and the eluent was evaporated. The residue was converted into the nitrate salt in 2-propanone and 2,2'-oxybispropane. The salt was filtered off and dried, yielding 1.4 parts (9.3%) of N-methyl-1-(1,2,3,4-tetrahydro-2,2-dimethyl-1-naphthalenyl)-1H-imidazole-5-carboxamide mononitrate; mp. 195.2°C (compound 2.04).

Example 19

A mixture of 3 parts of methyl 1-(1,2,3,4-tetrahydro-1-naphthalenyl)-1H-imidazole-5-carboxylate and 20 parts of hydrazine monohydrate was stirred and refluxed for 4 hours. The reaction mixture was poured into water and on scratching the product was crystallized. It was filtered off and recrystallized from ethanol 50%, yielding 1.8 parts of 1-(1,2,3,4-tetrahydro-1-naphthalenyl)-1H-imidazole-5-carboxylic acid hydrazide; mp. 177.5-180°C (compound 2.86).

Example 20

A mixture of 2.8 parts of (±)-methyl 1-(1-phenylethyl)-1H-imidazole-5-carboxylate, 32 parts of absolute ethanol and 5 parts of hydrazine monohydrate was stirred and refluxed for about 16 hours. The reaction mixture was evaporated under diminished pressure and the solid residue was triturated in hot benzene. The whole was filtered and the solid was recrystallized by dissolving it in a small volume of boiling 2-propanol and adding petroleumether to the obtained solution until it became turbid. On cooling to room temperature, a solid precipitated, which was filtered off and dried to yield 2 parts of (±)-1-(1-phenylethyl)-1H-imidazole-5-carboxylic acid hydrazide; mp. 134-135.5°C (compound 13.62).

Example 21

A mixture of 10 parts of methyl 1-[phenyl(2-pyridinyl)methyl]-1H-imidazole-5-carboxylate, 2.1 parts of 2-aminoethanol and 100 parts of water was stirred for 2 hours at reflux temperature. 80 Parts of methanol were added and stirring was continued for 1 week at reflux temperature. Water was added to the mixture and the product was extracted with dichloromethane. The extract was dried, filtered and evaporated. The residue was purified by column chromatography over silica gel using a mixture of trichloromethane and methanol (95:5 by volume) as eluent. The pure fractions were collected and the eluent was evaporated. The residue was crystallized from a mixture of 1,1'-oxybisethane and trichloromethane. The product was filtered off and dried in vacuo at 60°C, yielding 0.8 parts (7.2%) of N-(2-hydroxyethyl)-1-[phenyl(2-pyridinyl)methyl]-1H-imidazole-5-carboxamide; mp. 142.4°C (compound 13.77).

Example 22

To a stirred and warm solution of 10.8 parts of 1-(1,2,3,4-tetrahydro-2,2-dimethyl-1-naphthalenyl)-1H-imidazole-5-carboxylic acid in 94 parts of N,N-dimethylformamide were added 6.5 parts of 1,1'-carbonylbis-[1H-imidazole]. After stirring for 1 hour at room temperature, 3.9 parts of 2-aminoethanol were added. The reaction mixture was stirred overnight at room temperature. After evaporation, the residue was taken up in water and the product was extracted with trichloromethane. The extract was dried, filtered and evaporated. The residue was solidified in 2,2'-oxybispropane. The precipitated product was filtered off and crystallized from acetonitrile. The product was filtered off and dried, yielding 8.4 parts (67.0%) of N-(2-hydroxyethyl)-1-(1,2,3,4-tetrahydro-2,2-dimethyl-1-naphthalenyl)-1H-imidazole-5-carboxamide; mp. 150.3°C (compound 2.43).

Following the same procedure as in example 22 there were also prepared:

1-[[1-(3,4-dihyrdo-2,2-dimethyl-2H-1-benzopyran-4-yl)-1H-imidazol-5-yl]-carbonyl]piperazine; mp. 157.5°C (compound 6.24);

N-methyl-1-(1,2,3,4-tetrahydro-1-naphthalenyl)-1H-imidazole-5-carboxamide mononitrate; mp 155.5°C (compound 2.03); and

N-(2-hydroxy-1,1-dimethylethyl)-1-(1,2,3,4-tetrahydro-1-naphthalenyl)-1H-imidazole-5-carboxamide; mp. 159.4°C (compound 2.91).

Example 23

To a stirred solution of 4 parts of N-(2-hydroxyethyl)-1-(1,2,3,4-tetrahydro-2,2-dimethyl-1-naphthalenyl)-1H-imidazole-5-carboxamide in 30 parts of pyridine were added 1.4 parts of acetic acid anhydride. After stirring overnight at room temperature, the mixture was evaporated. The residue was taken up in water and

the product was extracted with trichloromethane. The extract was dried, filtered and evaporated. The residue was crystallized from acetonitrile. The product was filtered off and dried, yielding 3.0 parts (67.5%) of 2-[[1-(1-1,2,3,4-tetrahydro-2,2-dimethyl-1-naphthalenyl)-1H-imidazol-5-yl]carbonylamino]ethanol acetate-(ester); mp. 155.9°C (compound 2.99).

Example 24

To a stirred mixture of 4.3 parts of 1-[[1-(3,4-dihydro-2,2-dimethyl-2H-1-benzopyran-4-yl)-1H-imidazol-5-yl]carbonyl]piperazine and 225 parts of methylbenzene were added 0.85 parts of isocyanatomethane. After stirring for 2 hours at room temperature, the precipitated product was filtered off and purified by column chromatography over silica gel using a mixture of trichloromethane and methanol (90:10 by volume) as eluent. The pure fractions were collected and the eluent was evaporated. The residue was solidified in 2,2'-oxybispropane. The product was filtered off and dried, yielding 2.7 parts (54.3%) of 4-[[1-(3,4-dihydro-2,2-dimethyl-2H-1-benzopyran-4-yl)-1H-imidazol-5-yl]carbonyl]-N-methyl-1-piperazinecarboxamide; mp. 115.9°C (compound 6.81).

Example 25

A solution of 8.4 parts of N-methyl-1-(1,2,3,4-tetrahydro-1-naphthalenyl)-1H-imidazole-5-carboxamide and 13 parts of 2,4-bis(4-methoxyphenyl)-2,4-disulfide-1,3,2,4-dithiadiphosphetane in 40 parts of hexamethylphosphoric triamide was stirred for 24 hours at 100°C. The hexamethylphosphoric triamide layer was evaporated and the residue was stirred in a mixture of water and 1,1'-oxybisethane. The separated organic layer was washed with water, dried, filtered and evaporated. The residue was purified by column chromatography over silica gel using trichloromethane as eluent. The pure fractions were collected and the eluent was evaporated. The residue was crystallized from 2,2'-oxybispropane. The product was filtered off and dried, yielding 1.6 parts (14.5%) of N-methyl-1-(1,2,3,4-tetrahydro-1-naphthalenyl)-1H-imidazole-5-carbothioamide; mp. 174.1°C (compound 2.89).

Example 26

To a stirred and cooled (5°C, ice/water bath) suspension of 10.7 parts of 1-(1,2,3,4-tetrahydro-2,2-dimethyl-1-naphthalenyl)-1H-imidazole-5-carboxamide, 6.5 parts of pyridine and 120 parts of 1,4-dioxane were added dropwise 6.1 parts of trifluoroacetic acid anhydride during 30 minutes at this low temperature. The reaction mixture was allowed to reach room temperature and the whole was stirred overnight at room temperature. The reaction mixture was evaporated and the residue was taken up in ice water. The whole was treated with a sodium hydroxide solution 50% and the product was extracted with dichloromethane. The extract was washed with water, dried, filtered and evaporated. The residue was purified by column chromatography over silica gel using a mixture of trichloromethane and methanol (99:1 by volume) as eluent. The pure fractions were collected and the eluent was evaporated. The residue was crystallized from 2,2'-oxybispropane. The product was filtered off and dried, yielding 4.2 parts (41.7%) of 1-(1,2,3,4-tetrahydro-2,2-dimethyl-1-naphthalenyl)-1H-imidazole-5-carbonitrile; mp. 81.9°C (compound 2.26).

Example 27

9.6 Parts of thionyl chloride were added to 7.9 parts of N-(2-hydroxy-1,1-dimethylethyl)-1-(1,2,3,4-tetrahydro-1-naphthalenyl)-1H-imidazole-5-carboxamide (exothermic reaction). After stirring for 1 hour at room temperature, the reaction mixture was poured into 350 parts of 1,1'-oxybisethane. The whole was evaporated and the residue was taken up in water, sodium carbonate and trichloromethane. The separated organic layer was dried, filtered and evaporated. The residue was purified by column chromatography over silica gel, first using a mixture of trichloromethane and methanol (95:5 by volume) and then trichloromethane as eluents. The pure fractions were collected and the eluent was evaporated. The residue was further purified by column chromatography (HPLC) over silica gel using a mixture of methylbenzene,

trichloromethane and methanol (86:10:4 by volume) as eluent. The pure fractions were collected and the eluent was evaporated. The residue was crystallized from petroleum ether. The product was filtered off and dried, yielding 1.09 parts (14.7%) of 5-(4,5-dihydro-4,4-dimethyl-2-oxazolyl)-1-(1,2,3,4-tetrahydro-1-naphthalenyl)-1H-imidazole; mp. 107.4°C (compound 2.95).

Example 28

A mixture of 5.08 parts of 1-(1,2,3,4-tetrahydro-2,2-dimethyl-1-naphthalenyl)-1H-imidazole-5-carboxaldehyde, 2.1 parts of 1,2-benzenediamine and 20 parts of nitrobenzene was stirred for 30 minutes at reflux temperature. The remaining nitrobenzene was distilled off in vacuo and the residue was purified by column chromatography over silica gel using a mixture of trichloromethane and methanol (95:5 by volume) as eluent. The second fraction was collected and the eluent was evaporated. The residue was crystallized from acetonitrile. The product was filtered off and dried, yielding 2.46 parts (35.9%) of 2-[1-(1,2,3,4-tetrahydro-2,2-dimethyl-1-naphthalenyl)-1H-imidazol-5-yl]-1H-benzimidazole; mp. 228.5°C (compound 2.82).

Example 29

A mixture of 5.0 parts of 1-(1,2,3,4-tetrahydro-2,2-dimethyl-1-naphthalenyl)-1H-imidazole-5-carbonitrile, 1.5 parts of hydroxylamine hydrochloride, 1.16 parts of sodium carbonate, 16 parts of ethanol and 20 parts of water was stirred overnight at reflux temperature. The reaction mixture was evaporated and the residue was taken up in water. The product was extracted with trichloromethane. The extract was dried, filtered and evaporated. The residue was purified by column chromatography over silica gel using a mixture of trichloromethane and methanol (95:5 by volume) as eluent. The pure fractions were collected and the eluent was evaporated. The residue was crystallized from 2-propanol. The product was filtered off and dried, yielding 0.44 parts (7.7%) of N'-hydroxy-1-(1,2,3,4-tetrahydro-2,2-dimethyl-1-naphthalenyl)-1H-imidazole-5-carboximidamide; mp. 244.2°C (compound 2.76).

Example 30

A mixture of 7.5 parts of 1-(1,2,3,4-tetrahydro-2,2-dimethyl-1-naphthalenyl)-1H-imidazole-5-carbonitrile, 2.6 parts of sodium azide, 2.1 parts of ammonium chloride, 0.1 parts of lithium chloride and 47 parts of N,N-dimethylformamide was stirred for 16 hours at 125°C. After cooling to room temperature, the whole was filtered and the filtrate was evaporated. The residue was dissolved in alkaline water and dichloromethane was added. After shaking, the separated aqueous layer was acidified with a hydrochloric acid solution 10% to pH 4. The precipitated product was filtered off and dissolved in 750 parts of trichloromethane. The organic layer was dried, filtered and evaporated. The oil was dissolved in alkaline water. The whole was filtered and the filtrate was acidified with a hydrochloric acid solution 10% to pH 4. The product was filtered off, washed with water and dried at 110°C, yielding 4.01 parts (45.4%) of 5-[1-(1,2,3,4-tetrahydro-2,2-dimethyl-1-naphthalenyl)-1H-imidazol-2-yl]-1H-tetrazole; mp. 155.7°C (compound 2.129).

Example 31

To a stirred mixture of 12.3 parts of N-(1-cyano-2-oxoethyl)-N-(1,2,3,4-tetrahydro-1-naphthalenyl)-formamide, 6 parts of concentrated hydrochloric acid, 20 parts of water and 32 parts of ethanol was added a solution of 5.2 parts of potassium thiocyanate in 20 parts of water and the whole was stirred and refluxed for 3 hours. The reaction mixture was allowed to cool and the precipitated product was filtered off. It was purified a few times by column chromatography over silica gel using a mixture of trichloromethane and methanol (90:10 by volume) as eluent. The pure fractions were collected and the eluent was evaporated. The residue was crystallized from a mixture of methanol and water, yielding 1.3 parts (9%) of 1-(1,2,3,4-tetrahydro-1-naphthalenyl)-2-mercapto-1H-imidazole-5-carboxamide; mp. 227.7°C (compound 2.69).

Example 32

a) 12 Parts of 1-(2,3-dihydro-2,2-dimethyl-1H-inden-1-yl)-1H-imidazole-5-carboxylic acid was treated with 81 parts of thionyl chloride. After stirring for 1 hour at reflux temperature, the excess of thionyl chloride was distilled off. The residue was dissolved in 225 parts of tetrahydrofuran. Gaseous ammonia was bubbled through the mixture during 2 hours. After the addition of 250 parts of water, the precipitated product was filtered off and dried, yielding 10.7 parts (88.9%) of 1-(2,3-dihydro-2,2-dimethyl-1H-inden-1-yl)-1H-imidazole-5-carboxamide, mp. 218-219°C (compound 1.73).

b) A mixture of 29 parts of 1-(2,3-dihydro-2,2-dimethyl-1H-inden-1-yl)-1H-imidazole-5-carboxamide and 18.3 parts of pyridine was dispersed in 113 parts of 1,4-dioxane. 17.6 Parts of trifluoroacetic acid anhydride were added dropwise to the previously prepared mixture. After stirring for 24 hours, the reaction mixture was evaporated. The residue was dissolved in 1,1'-oxybisethane and extracted with water. The organic layer was dried, filtered and evaporated. The residue was crystallized from a mixture of 1,1'-oxybisethane and petroleum ether. The product was filtered off and dried, yielding 12.5 parts (43.2%) of 1-(2,3-dihydro-2,2-dimethyl-1H-inden-1-yl)-1H-imidazole-5-carbonitrile; mp. 73-73.5°C (compound 1.15).

c) A mixture of 2 parts of 1-(2,3-dihydro-2,2-dimethyl-1H-inden-1-yl)-1H-imidazole-5-carbonitrile, 18 parts of 1,2-ethanediamine and 1 part of silver nitrate was stirred and refluxed for 24 hours. The reaction mixture was poured into water and the product was extracted with dichloromethane. The extract was dried, filtered and evaporated. The residue was crystallized from 1,1'-oxybisethane. The product was filtered off and dried, yielding 1.8 parts (80.3%) of 2-(4,5-dihydro-1H-imidazol-2-yl)-1-(2,3-dihydro-2,2-dimethyl-1H-inden-1-yl)-1H-imidazole; mp. 155-156°C (compound 1.37).

Example 34

A mixture of 3 parts of 1-(2,3-dihydro-2,2-dimethyl-1H-inden-1-yl)-1H-imidazole-5-carbonitrile, 1.75 parts of hydroxylamine monohydrochloride, 1 part of sodium hydroxide and 16 parts of methanol was refluxed for 5 hours. The reaction mixture was poured into water. The precipitated product was filtered off and dispersed in 18 parts of tetrahydrofuran. 1.7 Parts of trifluoroacetic acid anhydride were added dropwise to the previous mixture. Upon complete addition, the mixture was stirred for 4 hours at room temperature. Th whole was poured into water. The precipitated product was filtered off and dried, yielding 0.5 parts (11.1%) of 3-[1-(2,3-dihydro-2,2-dimethyl-1H-inden-1-yl)-1H-imidazol-5-yl]-5-(trifluoromethyl)-1,2,4-oxadiazole; mp. 92-98°C (compound 1.35).

Example 35

To a stirred and heated (70°C) solution of 10 parts of N-methyl 4-amino-1-(5-chloro-2,3-dihydro-2,2-dimethyl-1H-inden-1-yl)-1H-imidazole-5-carboxamide in 47 parts of N,N-dimethylformamide are added 5.1 parts of 1,1-dimethylethyl nitrite. After stirring for 30 minutes, the reaction mixture is poured into water and the product extracted with 1,1'-oxybisethane. The extract is washed twice with water, dried, filtered and evaporated. The residue is converted into the nitrate salt in 2,2'-oxybispropane. The salt is filtered off and dried in vacuo, yielding N-methyl 1-(5-chloro-2,3-dihydro-2,2-dimethyl-1H-inden-1-yl)-1H-imidazole-5-carboxamide mononitrate (compound 1.75).

All other compounds listed in tables 1 to 15 can be obtained by analogous methods of preparation.

Table 1:

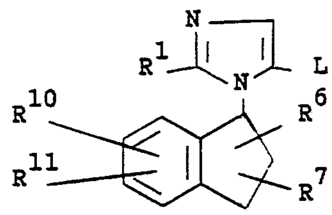

| Comp. No. | $R^1$ | $R^6$ | $R^7$ | $R^{10}$ | $R^{11}$ | L | physical data |
|---|---|---|---|---|---|---|---|
| 1.01 | H | 2-$CH_3$ | 2-$CH_3$ | H | H | -CO-N($CH_2$-$CH_2$OH)$_2$ | |
| 1.02 | H | 2-$CH_3$ | 2-$CH_3$ | H | H | -CO-NH-$CH_2$-$CH_2$OH | mp. 118-122°C |
| 1.03 | H | 2-$CH_3$ | 2-$CH_3$ | H | H | -CO-NH-$CH_3$ | mp. 122-127°C |
| 1.04 | H | 2-$CH_3$ | 2-$CH_3$ | H | H | -CO-NH-$OCH_3$ | mp. 66-68°C |
| 1.05 | H | 2-$CH_3$ | 2-$CH_3$ | H | H | -CO-$SCH_3$ | |
| 1.06 | H | 2-$CH_3$ | 2-$CH_3$ | H | H | -CO-$SC_2H_5$ | |
| 1.07 | H | 2-$CH_3$ | 2-$CH_3$ | H | H | -CO-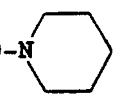 | |
| 1.08 | H | 2-$CH_3$ | 2-$CH_3$ | H | H | -CO-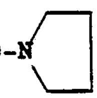 | |
| 1.09 | H | 2-$CH_3$ | 2-$CH_3$ | H | H | -CO-NH-$C_2H_5$ | |
| 1.10 | H | 2-$CH_3$ | 2-$CH_3$ | H | H | -CO-NH-$C_3H_7$-n | resin |
| 1.11 | H | 2-$CH_3$ | 2-$CH_3$ | H | H | -CO-N($CH_3$)$_2$ | mp. 96-96.5°C |
| 1.12 | H | 2-$CH_3$ | 2-$CH_3$ | H | H | -CO-NH-$C_3H_5$-c | |
| 1.13 | H | 2-$CH_3$ | 2-$CH_3$ | H | H | -CO-NH-$C_4H_9$-n | |
| 1.14 | H | 2-$CH_3$ | 2-$CH_3$ | H | H | -CO-$NH_2$ | mp. 218-219°C |
| 1.15 | H | 2-$CH_3$ | 2-$CH_3$ | H | H | -CN | mp. 73-73.5°C |
| 1.16 | H | 2-$CH_3$ | H | H | H | -CO-$NH_2$ | |
| 1.17 | H | 2-$CH_3$ | H | H | H | -CN | |
| 1.18 | H | 2-$CH_3$ | H | H | H | -CO-NH-$OCH_3$ | |
| 1.19 | H | 2-$CH_3$ | H | H | H | -CO-NH-$CH_3$ | |
| 1.20 | H | 2-$CH_3$ | H | H | H | -CO-NH-$C_2H_5$ | |
| 1.21 | H | 2-$CH_3$ | H | H | H | -CO-NH-$C_3H_5$-c | |
| 1.22 | H | 2-$CH_3$ | H | 5-$CH_3$ | 7-$CH_3$ | -CO-S-$CH_3$ | |
| 1.23 | H | 2-($CH_2$)$_4$-2 | | H | H | -CO-NH-$CH_3$ | |

| Comp. No. | $R^1$ | $R^6$ | $R^7$ | $R^{10}$ | $R^{11}$ | L | physical data |
|---|---|---|---|---|---|---|---|
| 1.24 | H | 2-$(CH_2)_4$-2 | | H | H | -CO-NH-$OCH_3$ | |
| 1.25 | H | 2-$C_2H_5$ | 2-$C_2H_5$ | H | H | -CO-NH-$CH_3$ | mp. 135-137°C |
| 1.26 | H | 2-$CH_3$ | 2-$C_2H_5$ | H | H | -CO-$NH_2$ | |
| 1.27 | H | 2-$CH_3$ | 2-$C_2H_5$ | H | H | -CN | |
| 1.28 | H | 2-$CH_3$ | 2-$C_2H_5$ | H | H | -CO-NH-$CH_3$ | |
| 1.29 | H | 2-$CH_3$ | 2-$C_2H_5$ | H | H | -CO-S-$CH_3$ | |
| 1.30 | H | 2-$CH_3$ | 2-$C_2H_5$ | H | H | -CO-NH-$OCH_3$ | |
| 1.31 | H | H | H | H | H | -CO-NH-$CH_3$ | $HNO_3$/mp.133.0°C |
| 1.32 | H | 2-$CH_3$ | 2-$CH_3$ | H | H | 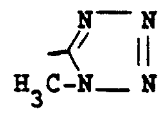 | |
| 1.33 | H | 2-$CH_3$ | 2-$CH_3$ | H | H | -CO-NH-NH-$CH_3$ | |
| 1.34 | H | 2-$CH_3$ | 2-$CH_3$ | H | H | 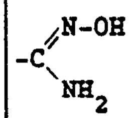 | mp. >100°C (dec.) |
| 1.35 | H | 2-$CH_3$ | 2-$CH_3$ | H | H | 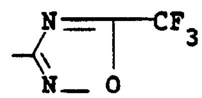 | mp. 92-98°C |
| 1.36 | H | 2-$CH_3$ | 2-$CH_3$ | H | H | -CO-N$(C_2H_5)_2$ | mp. 100-100.5°C |
| 1.37 | H | 2-$CH_3$ | 2-$CH_3$ | H | H | 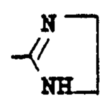 | mp. 155-156°C |
| 1.38 | SH | 2-$(CH_2)_3$-3 | | H | H | -CO-NH-$CH_3$ | |
| 1.39 | H | 2-$(CH_2)_3$-3 | | H | H | -CO-NH-$CH_3$ | A/mp. 190.1°C |
| 1.40 | SH | 2-$(CH_2)_4$-3 | | H | H | -CO-NH-$CH_3$ | |
| 1.41 | H | 2-$(CH_2)_4$-3 | | H | H | -CO-NH-$CH_3$ | |
| 1.42 | H | 2-$CH_3$ | 2-$CH_3$ | H | H | -CO-NH-$SO_2$-$CH_3$ | |
| 1.43 | H | 2-$CH_3$ | 2-$CH_3$ | H | H | -CO-N$(CH_3)$-CN | |
| 1.44 | H | 2-$CH_3$ | 2-$CH_3$ | H | H | -CO-NH-$COCH_3$ | |
| 1.45 | H | 2-$CH_3$ | 2-$CH_3$ | H | H | 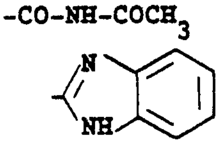 | |

| Comp. No. | $R^1$ | $R^6$ | $R^7$ | $R^{10}$ | $R^{11}$ | L | physical data |
|---|---|---|---|---|---|---|---|
| 1.46 | H | 2-$CH_3$ | 2-$CH_3$ | H | H | -CO-NH-$COOCH_3$ | |
| 1.47 | H | 2-$CH_3$ | 2-$CH_3$ | H | H | 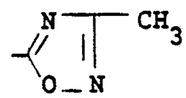 | |
| 1.48 | H | 2-$CH_3$ | 2-$CH_3$ | 6-$CH_3$ | H | -CO-NH-$CH_3$ | |
| 1.49 | H | 2-$CH_3$ | 2-$CH_3$ | 6-F | H | -CO-NH-$CH_3$ | |
| 1.50 | H | 2-$CH_3$ | 2-$CH_3$ | 6-$OCH_3$ | H | -CO-NH-$CH_3$ | |
| 1.51 | H | 2-$CH_3$ | 2-$CH_3$ | 6-Br | H | -CO-NH-$CH_3$ | |
| 1.52 | SH | 2-$CH_3$ | 2-$CH_3$ | 6-Br | H | -CO-NH-$CH_3$ | |
| 1.53 | H | 2-$CH_3$ | 2-$CH_3$ | 5-F | H | -CO-NH-$CH_3$ | |
| 1.54 | H | 2-$CH_3$ | 2-$CH_3$ | 5-$CH_3$ | H | -CO-NH-$CH_3$ | |
| 1.55 | H | 2-$(CH_2)_5$-2 | | H | H | -CO-NH-$CH_3$ | |
| 1.56 | SH | 2-$(CH_2)_5$-2 | | H | H | -CO-NH-$CH_3$ | |
| 1.57 | H | 2-$CH_3$ | 2-$CH_3$ | H | H | -CO- 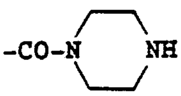 | |
| 1.58 | SH | 2-$CH_3$ | 2-$CH_3$ | H | H | -CO- 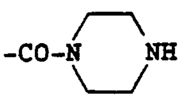 | |
| 1.59 | H | 2-$CH_3$ | 2-$CH_3$ | H | H | -CO- 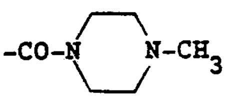 | |
| 1.60 | SH | 2-$CH_3$ | 2-$CH_3$ | H | H | -CO- 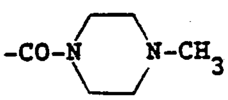 | |
| 1.61 | SH | 2-$CH_3$ | 2-$CH_3$ | H | H | -CO- 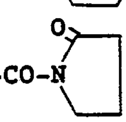 | |
| 1.62 | H | 2-$CH_3$ | 2-$CH_3$ | H | H | -CO- 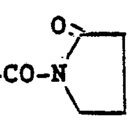 | |
| 1.63 | SH | 2-$CH_3$ | 2-$CH_3$ | H | H | 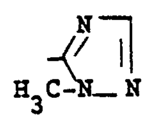 | |
| 1.64 | H | 2-$CH_3$ | 2-$CH_3$ | H | H | 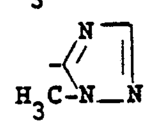 | |

| Comp. No. | $R^1$ | $R^6$ | $R^7$ | $R^{10}$ | $R^{11}$ | L | physical data |
|---|---|---|---|---|---|---|---|
| 1.65 | SH | 2-$CH_3$ | 2-$CH_3$ | H | H | -CO-NCS | |
| 1.66 | H | 2-$CH_3$ | 2-$CH_3$ | H | H | -CO-NCS | |
| 1.67 | H | 3-$CH_3$ | 3-$CH_3$ | H | H | -CO-NH-$CH_3$ | mp.167.4°C |
| 1.68 | H | 3-$CH_3$ | H | H | H | -CO-NH-$CH_3$ | $HNO_3$/mp.99.4°C |
| 1.69 | H | 2-$CH_3$ | 2-$CH_3$ | H | H | -CO-NH-$CH_3$ | $H_2O$ |
| 1.70 | H | 2-$CH_3$ | 2-$CH_3$ | H | H | -CO- 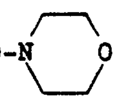 | mp. 148-149°C |
| 1.71 | H | 2-$CH_3$ | 2-$CH_3$ | H | H | -CO- 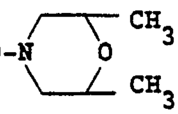 | mp. 155-157°C |
| 1.72 | H | 2-$CH_3$ | 2-$CH_3$ | H | H | -CO-N[CH($CH_3$)$_2$]$_2$ | mp. 228-229°C |
| 1.73 | H | 2-$CH_3$ | 2-$CH_3$ | H | H | -CO-$NH_2$ | mp. 218-219°C (±) |
| 1.74 | H | 2-$CH_3$ | 2-$CH_3$ | H | H | -CO-NH-$CH_3$ | mp.138-145°C $[\alpha]_D$=-54.7° 0.25 $H_2O$ |
| 1.75 | H | 2-$CH_3$ | 2-$CH_3$ | 5-Cl | H | -CO-NH-$CH_3$ | $HNO_3$/mp.199.1°C |
| 1.76 | H | 2-$CH_3$ | 2-$CH_3$ | H | H | -COO-N=C($CH_3$)$_2$ | mp.81-83°C |
| 1.77 | H | 2-$CH_3$ | 2-$CH_3$ | H | H | -COO-$C_6H_5$ | mp.99-101°C |
| 1.78 | H | 2-$CH_3$ | 2-$CH_3$ | H | H | -COO-($CH_2$)$_3$Cl | |
| 1.79 | H | 2-$CH_3$ | 2-$CH_3$ | H | H | -COO-$CH_2COOCH_3$ | |
| 1.80 | H | 2-$CH_3$ | 2-$CH_3$ | H | H | -COO-CH($CH_3$)$COOCH_3$ (R) | resin |
| 1.81 | H | 2-$CH_3$ | 2-$CH_3$ | H | H | -COO-CH($CH_3$)$COOCH_3$ (S) | resin |
| 1.82 | H | 2-$CH_3$ | 2-$CH_3$ | H | H | -COO-$CH_2$PO($OC_2H_5$)$_2$ | mp.88.5-90°C |
| 1.83 | H | 2-$CH_3$ | 2-$CH_3$ | H | H | -COO-$CH_2$COOH | |
| 1.84 | H | 2-$CH_3$ | 2-$CH_3$ | H | H | -COO-$CH_2$Si($CH_3$)$_3$ | |
| 1.85 | H | 2-$CH_3$ | 2-$CH_3$ | H | H | COO-($CH_2$)$_2$Si($CH_3$)$_3$ | |

| Comp. No. | $R^1$ | $R^6$ | $R^7$ | $R^{10}$ | $R^{11}$ | L | physical data |
|---|---|---|---|---|---|---|---|
| 1.86 | H | 2-$CH_3$ | 2-$CH_3$ | H | H | -$CONH_2$ | oil/mp.190-192°C $[\alpha]_D$=-91.1° |
| 1.87 | H | 2-$CH_3$ | 2-$CH_3$ | H | H | -$CONH_2$ | (+) |
| 1.88 | H | 2-$CH_3$ | 2-$CH_3$ | H | H | -CONH-$CH_3$ | (+) |
| 1.89 | H | 2-$CH_3$ | 3-$CH_3$ | H | H | -CONH-$CH_3$ | |
| 1.90 | H | 2-$CH_3$ | 3-$CH_3$ | H | H | -CONH-$CH_3$ | 1,2-cis; 2,3-cis |
| 1.91 | H | 2-$CH_3$ | 3-$CH_3$ | H | H | -CONH-$CH_3$ | 1,2-cis;2,3-trans |
| 1.92 | H | 2-$CH_3$ | 3-$CH_3$ | H | H | -CONH-$CH_3$ | 1,2-trans;2,3-cis |
| 1.93 | H | 2-$CH_3$ | 3-$CH_3$ | H | H | -CONH-$CH_3$ | 1,2-trans; 2,3-trans |
| 1.94 | H | 2-$CH_2$-$C_6H_5$ | H | H | H | -CONH-$CH_3$ | 1,2-trans;.$HNO_3$ mp.109.0°C |
| 1.95 | H | 2-$CH_2$-$C_6H_5$ | H | H | H | -CONH-$CH_3$ | 1/2$H_2O$, 1,2-cis mp.98.4°C |
| 1.96 | H | 2-$CH_3$ | 2-$CH_3$ | H | H | -CONH-$NH_2$ | mp. 164-169°C |
| 1.97 | H | 2-$CH_3$ | 2-$CH_3$ | H | H | -CONH$CH_2$-$C_6H_5$ | mp. 54-57°C |
| 1.98 | H | 2-$CH_3$ | 2-$CH_3$ | H | H | -CN | $[\alpha]_D$=-59.1°±1.2 resin |
| 1.99 | H | 2-$CH_3$ | 2-$CH_3$ | H | H | -$COOCH_2$-$CF_3$ | oil |

Table 2:

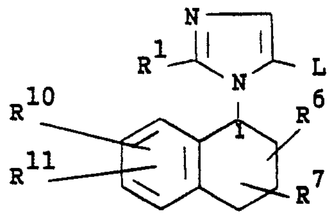

| Comp. No. | $R^1$ | $R^6$ | $R^7$ | $R^{10}$ | $R^{11}$ | L | physical data |
|---|---|---|---|---|---|---|---|
| 2.01 | H | H | H | 6-Cl | H | $-CO-NH-CH_3$ | $HNO_3$/mp. 177.7°C |
| 2.02 | H | H | H | 7-F | H | $-CO-NH-CH_3$ | $HNO_3$/mp. 164°C |
| 2.03 | H | H | H | H | H | $-CO-NH-CH_3$ | $HNO_3$/mp. 155.5°C |
| 2.04 | H | $2-CH_3$ | $2-CH_3$ | H | H | $-CO-NH-CH_3$ | $HNO_3$/mp. 195.2°C |
| 2.05 | H | H | H | H | H | -CO- 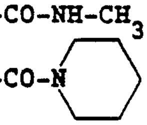 | $HNO_3$/mp. 148.3°C |
| 2.06 | H | H | H | H | H | -CO- 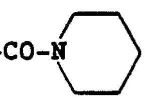 | mp. 118°C |
| 2.07 | H | H | H | H | H | $-CO-NH-C_2H_5$ | $HNO_3$/mp. 143.5°C |
| 2.08 | H | H | H | H | H | $-CO-NH_2$ | mp. 207-208°C |
| 2.09 | H | H | H | H | H | -CO- 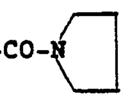 | mp. 200-201°C |
| 2.10 | H | H | H | H | H | -CO- 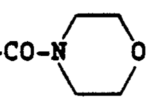 | solid |
| 2.11 | H | H | H | H | H | -CN | mp. 89-90°C |
| 2.12 | H | H | H | H | H | $-CO-NH-C_3H_5$-c | mp. 166-167°C |
| 2.13 | H | H | H | H | H | $-CO-NH-C_3H_7$-n | mp. 137-138°C |
| 2.14 | H | H | H | H | H | $-CO-NH-OCH_3$ | mp. 112-113°C |
| 2.15 | H | H | H | H | H | $-CO-NH-CH_3$ | mp. 105-106°C |
| 2.16 | H | H | H | H | H | $-CO-N(CH_3)_2$ | |
| 2.17 | H | H | H | H | H | $-CO-NH-C_4H_9$-n | |

| Comp. No. | $R^1$ | $R^6$ | $R^7$ | $R^{10}$ | $R^{11}$ | L | physical data |
|---|---|---|---|---|---|---|---|
| 2.18 | H | H | H | H | H | $-CO-NH-CH_2-C\equiv CH$ | $HNO_3$/ mp. 128°C |
| 2.19 | H | H | H | H | H | $-CO-NH-CH_3$ | |
| 2.20 | H | H | H | H | H | $-CO-SCH_3$ | $HNO_3$/ mp. 159°C |
| 2.21 | H | H | H | H | H | $-CO-NH-C_3H_7$-i | |
| 2.22 | H | H | H | H | H | $-CO-NH-C_5H_{11}$-n | |
| 2.23 | H | H | H | H | H | $-CO-S-C_2H_5$ | |
| 2.24 | H | H | H | H | H | $-CO-NH-C_6H_{11}$-c | |
| 2.25 | H | 2-$CH_3$ | 2-$CH_3$ | H | H | $-CO-NH_2$ | mp. 238.2°C |
| 2.26 | H | 2-$CH_3$ | 2-$CH_3$ | H | H | -CN | mp. 81.9°C |
| 2.27 | H | 2-$CH_3$ | 2-$CH_3$ | H | H | $-CO-NH-C_2H_5$ | |
| 2.28 | H | 2-$CH_3$ | 2-$CH_3$ | H | H | $-CO-N(CH_3)_2$ | mp. 116.9°C |
| 2.29 | H | 2-$CH_3$ | 2-$CH_3$ | H | H | $-CO-NH-C_3H_5$-c | |
| 2.30 | H | 2-$CH_3$ | 2-$CH_3$ | H | H | $-CO-S-CH_3$ | mp. 80.7°C |
| 2.31 | H | 2-$CH_3$ | 2-$CH_3$ | H | H | $-CO-S-C_2H_5$ | |
| 2.32 | H | 2-$CH_3$ | 2-$CH_3$ | H | H | -CO- 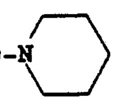 | |
| 2.33 | H | 2-$CH_3$ | 2-$CH_3$ | H | H | -CO- 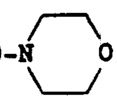 | |
| 2.34 | H | 2-$CH_3$ | 2-$CH_3$ | H | H | $-CO-NH-CH_2-C\equiv CH$ | HCl 1/2$H_2O$ mp. 141.2°C |
| 2.35 | H | 2-$CH_3$ | 2-$CH_3$ | H | H | $-CO-NH-CH_3$ | |
| 2.36 | H | 2-$CH_3$ | 2-$CH_3$ | H | H | $-CO-NH-OCH_3$ | $HNO_3$/mp.132.4°C |
| 2.37 | H | 2-$CH_3$ | 2-$CH_3$ | H | H | $-CO-NH-C_3H_7$-n | |
| 2.38 | H | 2-$CH_3$ | 2-$CH_3$ | H | H | -CO- 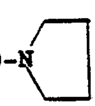 | |
| 2.39 | H | 2-$CH_3$ | H | H | H | $-CO-NH-CH_3$ | |

| Comp. No. | $R^1$ | $R^6$ | $R^7$ | $R^{10}$ | $R^{11}$ | L | physical data |
|---|---|---|---|---|---|---|---|
| 2.40 | H | 2-$CH_3$ | H | H | H | -CO-S-$CH_3$ | |
| 2.41 | H | 2-$CH_3$ | H | H | H | -CO-NH-$OCH_3$ | |
| 2.42 | H | H | H | H | H | -CO-NH-$CH_2$-$CH_2$OH | mp.124.1°C |
| 2.43 | H | 2-$CH_3$ | 2-$CH_3$ | H | H | -CO-NH-$CH_2$-$CH_2$OH | mp.150.3°C |
| 2.44 | H | 2-$CH_3$ | 2-$CH_3$ | H | H | -CO-N($CH_2$-$CH_2$OH)$_2$ | |
| 2.45 | H | H | H | H | H | -CO-N($CH_2$-$CH_2$OH)$_2$ | |
| 2.46 | H | H | H | 5-$CH_3$ | 7-$CH_3$ | -CO-NH-$CH_3$ | |
| 2.47 | H | H | H | 5-$CH_3$ | 7-$CH_3$ | -CO-S-$CH_3$ | |
| 2.48 | H | H | H | 5-$CH_3$ | 7-$CH_3$ | -CO-NH-$OCH_3$ | |
| 2.49 | H | 2-$(CH_2)_4$-2 | | H | H | -CO-$NH_2$ | |
| 2.50 | H | 2-$(CH_2)_4$-2 | | H | H | -CN | |
| 2.51 | H | 2-$(CH_2)_4$-2 | | H | H | -CO-NH-$CH_3$ | |
| 2.52 | H | 2-$(CH_2)_4$-2 | | H | H | -CO-S-$CH_3$ | |
| 2.53 | H | 2-$(CH_2)_4$-2 | | H | H | -CO-NH-$OCH_3$ | |
| 2.54 | H | 2-$(CH_2)_5$-2 | | H | H | -CO-$NH_2$ | |
| 2.55 | H | 2-$(CH_2)_5$-2 | | H | H | -CN | |
| 2.56 | H | 2-$(CH_2)_5$-2 | | H | H | -CO-NH-$CH_3$ | |
| 2.57 | H | 2-$(CH_2)_5$-2 | | H | H | -CO-S-$CH_3$ | |
| 2.58 | H | 2-$(CH_2)_5$-2 | | H | H | -CO-NH-$OCH_3$ | |
| 2.59 | SH | 2-$(CH_2)_5$-2 | | H | H | -CO-NH-$OCH_3$ | |
| 2.60 | H | 2-$C_2H_5$ | H | H | H | -CO-NH-$CH_3$ | |
| 2.61 | H | 2-$C_3H_7$-n | H | H | H | -CO-NH-$CH_3$ | |
| 2.62 | H | 2-$C_4H_9$-n | H | H | H | -CO-NH-$CH_3$ | |
| 2.63 | H | 2-$C_5H_{11}$-n | H | H | H | -CO-NH-$CH_3$ | |
| 2.64 | H | 2-$C_3H_7$-i | H | H | H | -CO-NH-$CH_3$ | |
| 2.65 | SH | 2-$CH_3$ | 2-$CH_3$ | H | H | -CO-$NH_2$ | |
| 2.66 | SH | 2-$CH_3$ | 2-$CH_3$ | H | H | -CN | |
| 2.67 | SH | 2-$CH_3$ | 2-$CH_3$ | H | H | -CO-NH-$CH_3$ | |
| 2.68 | SH | 2-$CH_3$ | 2-$CH_3$ | H | H | -CO-NH-$NH_2$ | |
| 2.69 | SH | H | H | H | H | -CO-$NH_2$ | mp. 220-227.7°C |

| Comp. No. | $R^1$ | $R^6$ | $R^7$ | $R^{10}$ | $R^{11}$ | L | physical data |
|---|---|---|---|---|---|---|---|
| 2.70 | H | H | H | H | H | 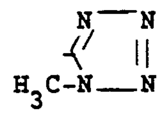 | |
| 2.71 | H | H | H | H | H | $-C(NH_2)=N-O-CH_3$ | |
| 2.72 | H | H | H | H | H | $-C(NH_2)=N-OH$ | |
| 2.73 | H | H | H | H | H | 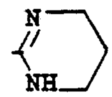 | |
| 2.74 | H | $2-(CH_2)_3-3$ | | H | H | $-CO-NH-CH_3$ | |
| 2.75 | H | $3-(CH_2)_3-4$ | | H | H | $-CO-NH-CH_3$ | |
| 2.76 | H | $2-CH_3$ | $2-CH_3$ | H | H | $-C(NH_2)=N-OH$ | mp. 244.2°C |
| 2.77 | H | $2-CH_3$ | $2-CH_3$ | H | H | 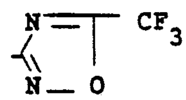 | |
| 2.78 | H | $2-CH_3$ | $2-CH_3$ | H | H | $-CO-N(C_2H_5)_2$ | |
| 2.79 | H | $2-CH_3$ | $2-CH_3$ | H | H | 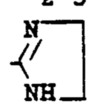 | |
| 2.80 | H | $2-CH_3$ | $2-CH_3$ | H | H | 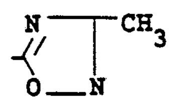 | |
| 2.81 | H | $2-CH_3$ | $2-CH_3$ | H | H | $-CO-NH-C_5H_{11}-n$ | |
| 2.82 | H | $2-CH_3$ | $2-CH_3$ | H | H | 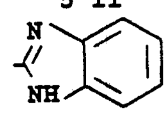 | mp. 228.5°C |
| 2.83 | H | $2-(CH_2)_4-3$ | | H | H | $-CO-NH-CH_3$ | |
| 2.84 | H | $2-(CH_2)_4-3$ | | H | H | $-CO-NH-CH_3$ | |
| 2.85 | H | H | H | H | H | $-CO-NH-CH_2-CH=CH_2$ | $HNO_3$/mp. 127.8°C |
| 2.86 | H | H | H | H | H | $-CO-NH-NH_2$ | mp. 177.5-180°C |
| 2.87 | H | H | H | H | H | $-CO-NH-NH-CO-CH_3$ | mp. 257.8°C |
| 2.88 | H | H | H | H | H | -CO- 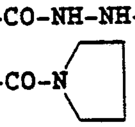 | $HNO_3$/mp. 158.8°C |

| Comp. No. | $R^1$ | $R^6$ | $R^7$ | $R^{10}$ | $R^{11}$ | L | physical data |
|---|---|---|---|---|---|---|---|
| 2.89 | H | H | H | H | H | $-CS-NH-CH_3$ | mp. 174.1°C |
| 2.90 | H | H | H | H | H | -CO-NH-OH | mp. 122.1°C |
| 2.91 | H | H | H | H | H | 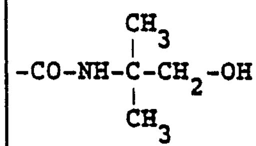 | mp. 159.4°C |
| 2.92 | H | $2-CH_3$ | $2-CH_3$ | H | H | $-CO-NH-CH_2-CH=CH_2$ | HCl $1/2H_2O$ mp. 128.2°C |
| 2.93 | H | $2-CH_3$ | $2-CH_3$ | H | H | $-CO-NH-(CH_2)_2-OCH_3$ | mp. 121.0°C |
| 2.94 | H | $2-CH_3$ | $2-CH_3$ | H | H | -CO-NH-OH | mp. 133.7°C |
| 2.95 | H | H | H | H | H | 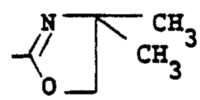 | mp. 107.4°C |
| 2.96 | H | $2-CH_3$ | $2-CH_3$ | H | H | -CO-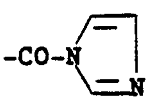 | mp. 122.4°C |
| 2.97 | H | $3-(CH_2)_4-4$ | | H | H | $-CO-NH-CH_3$ | $HNO_3$/mp. 177.8°C |
| 2.98 | SH | $2-CH_3$ | $2-CH_3$ | H | H | $-CO-NH-C_4H_9-n$ | |
| 2.99 | H | $2-CH_3$ | $2-CH_3$ | H | H | $CONH-(CH_2)_2OCOCH_3$ | mp. 155.9°C |
| 2.100 | H | $2-CH_3$ | $2-CH_3$ | H | H | $-COO-N=C(CH_3)_2$ | |
| 2.101 | H | $2-CH_3$ | $2-CH_3$ | H | H | $-COO-CH_2CN$ | |
| 2.102 | H | $2-CH_3$ | $2-CH_3$ | H | H | $-COO-CH_2CH_2OCOCH_3$ | mp. 78°C |
| 2.103 | H | $2-CH_3$ | $2-CH_3$ | H | H | $-COO-CH_2-CH_2-NH_2$ | |
| 2.104 | H | $2-CH_3$ | $2-CH_3$ | H | H | $-COO-CH_2-CH_2-NH-CO-NH-CH_3$ | |
| 2.105 | H | $2-CH_3$ | $2-CH_3$ | H | H | $-COO-CH_2-CH_2-NH-CO-C_6H_5$ | |
| 2.106 | H | $2-CH_3$ | $2-CH_3$ | H | H | $-COO-CH_2-CF_3$ | |
| 2.107 | H | $2-CH_3$ | $2-CH_3$ | H | H | $-COO-(3-NO_2-C_6H_4)$ | |
| 2.108 | H | $2-CH_3$ | $2-CH_3$ | H | H | $-COO-(4-Cl-C_6H_4)$ | |

| Comp. No. | $R^1$ | $R^6$ | $R^7$ | $R^{10}$ | $R^{11}$ | L | physical data |
|---|---|---|---|---|---|---|---|
| 2.109 | H | $2-CH_3$ | $2-CH_3$ | H | H | $-COO-CH(COOCH_3)-CH_2CN$ | |
| 2.110 | H | $2-CH_3$ | $2-CH_3$ | H | H | $COO(2,4,5-Cl_3-C_6H_2)$ | |
| 2.111 | H | $2-CH_3$ | $2-CH_3$ | H | H | $-COO-CH(COOCH_3)_2$ | |
| 2.112 | H | $2-CH_3$ | $2-CH_3$ | H | H | $-COO-N=C(CH_3)_2$ | HCl |
| 2.113 | H | $2-CH_3$ | $2-CH_3$ | H | H | $-COO-N=C(CH_3)_2$ | $HNO_3$ |
| 2.114 | SH | $2-CH_3$ | $2-CH_3$ | H | H | $-COO-C_6H_5$ | |
| 2.115 | H | H | H | H | H | $-COO-(CH_2)_2-N(CH_3)_2$ | $2HNO_3$/mp.133-136°C |
| 2.116 | H | H | H | H | H | $-COO-N=C(CH_3)_2$ | mp. 125.7°C |
| 2.117 | H | H | H | H | H | -COO-N= 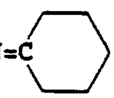 | $HNO_3$/mp. 159.2°C |
| 2.118 | H | H | H | H | H | $-COO-CH_2-CN$ | mp. 109.6°C |
| 2.119 | H | H | H | H | H | $-COO-C_6H_5$ | $HNO_3$/mp. 153.6°C |
| 2.120 | H | H | H | H | H | $-COO-CH_2-S-CH_3$ | mp. 99.4°C |
| 2.121 | H | H | H | H | H | $-COO-CH_2COOCH_3$ | HCl/mp. 189.5°C |
| 2.122 | H | H | H | H | H | $-COO-CH_2-CH_2-OCOCH_3$ | mp. 77.2°C |
| 2.123 | H | H | H | H | H | $-COO-CH_2-CH_2-NHCOCH_3$ | oil |
| 2.124 | H | H | H | H | H | $-COO-CH(CH_3)-$ $CO-OCH_3$ | HCl/mp. 151.5°C |
| 2.125 | H | H | H | H | H | $-COO-CH_2-CO-CH_3$ | HCl/mp. 130.2°C |
| 2.126 | H | H | H | H | H | $-COO-(CH_2)_2-OH$ | mp. 104.3°C |
| 2.127 | H | $2-CH_3$ | $2-CH_3$ | H | H | $-COO-CH_2-CH_2OH$ | mp. 108.1°C |
| 2.128 | H | $2-CH_3$ | $2-CH_3$ | H | H | $-CO-NH-C_3H_7-i$ | |
| 2.129 | H | $2-CH_3$ | $2-CH_3$ | H | H | 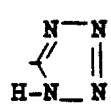 | mp. 155.7°C |
| 2.130 | H | $2-CH_3$ | $2-CH_3$ | H | H | $-CO-NH-CH_3$ | (+) |
| 2.131 | H | $2-CH_3$ | $2-CH_3$ | H | H | $-CO-NH-CH_3$ | (-) |

| Comp. No. | $R^1$ | $R^6$ | $R^7$ | $R^{10}$ | $R^{11}$ | L | physical data |
|---|---|---|---|---|---|---|---|
| 2.132 | H | 2-$CH_3$ | 2-$CH_3$ | H | H | -CO-$NH_2$ | (+) |
| 2.133 | H | 2-$CH_3$ | 2-$CH_3$ | H | H | -CO-$NH_2$ | (-) |
| 2.134 | H | 2-$CH_3$ | 3-$CH_3$ | H | H | -CO-NH-$CH_3$ | |
| 2.135 | H | 2-$CH_3$ | 3-$CH_3$ | H | H | -CO-NH-$CH_3$ | 1,2-cis;2,3-cis |
| 2.136 | H | 2-$CH_3$ | 3-$CH_3$ | H | H | -CO-NH-$CH_3$ | 1,2-cis;2,3-trans |
| 2.137 | H | 2-$CH_3$ | 3-$CH_3$ | H | H | -CO-NH-$CH_3$ | 1,2-trans;2,3-cis |
| 2.138 | H | 2-$CH_3$ | 3-$CH_3$ | H | H | -CO-NH-$CH_3$ | 1,2-trans; 2,3-trans |
| 2.139 | H | 2-$(CH_2)_3$-3 | | H | H | -CO-NH-$CH_3$ | |
| 2.140 | H | 2-$(CH_2)_4$-3 | | H | H | -CO-NH-$CH_3$ | |
| 2.141 | H | 2-$(CH_2)_2$-4 | | H | H | -CO-NH-$CH_3$ | |
| 2.142 | H | 2-$(CH_2)_3$-4 | | H | H | -CO-NH-$CH_3$ | |
| 2.143 | H | 2-$(CH_2)_2$-4 | | H | H | -CO-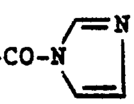 | mp. 219.6°C |

Table 3:

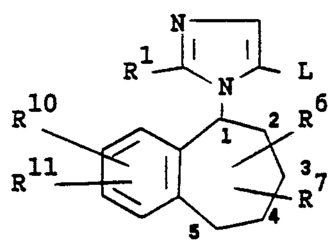

| Comp. No. | $R^1$ | $R^6$ | $R^7$ | $R^{10}$ | $R^{11}$ | L | physical data |
|---|---|---|---|---|---|---|---|
| 3.01 | H | H | H | H | H | $-CO-NH_2$ | |
| 3.02 | H | H | H | H | H | -CN | |
| 3.03 | SH | H | H | H | H | -CN | |
| 3.04 | H | H | H | H | H | $-CO-NH-CH_3$ | |
| 3.05 | H | H | H | H | H | $-CO-NH-OCH_3$ | |
| 3.06 | H | H | H | H | H | $-CO-S-CH_3$ | |
| 3.07 | H | $2-CH_3$ | $2-CH_3$ | H | H | $-CO-NH-CH_3$ | |
| 3.08 | SH | $2-CH_3$ | $2-CH_3$ | H | H | $-CO-NH-CH_3$ | |
| 3.09 | H | $2-CH_3$ | H | H | H | $-CO-NH-CH_3$ | |
| 3.10 | SH | $2-CH_3$ | H | H | H | $-CO-NH-CH_3$ | |

Table 4:

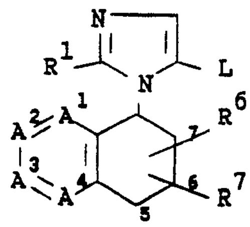

| Comp. No. | $R^1$ | $R^6$ | $R^7$ | L | $A^1=A^2-A^3=A^4$ | physical data |
|---|---|---|---|---|---|---|
| 4.01 | H | H | H | $-CO-NH-CH_3$ | CH=CH-CH=N | |
| 4.02 | SH | H | H | $-CO-NH-CH_3$ | CH=CH-CH=N | |
| 4.03 | H | $7-CH_3$ | $7-CH_3$ | $-CO-NH-CH_3$ | CH=CH-CH=N | |
| 4.04 | SH | $7-CH_3$ | $7-CH_3$ | $-CO-NH-CH_3$ | CH=CH-CH=N | |
| 4.05 | H | $7-CH_3$ | $7-CH_3$ | $-CO-NH_2$ | CH=CH-CH=N | |
| 4.06 | SH | $7-CH_3$ | $7-CH_3$ | $-CO-NH_2$ | CH=CH-CH=N | |
| 4.07 | H | H | H | -CN | CH=CH-CH=N | |
| 4.08 | SH | H | H | -CN | CH=CH-CH=N | |
| 4.09 | H | $7-CH_3$ | $7-CH_3$ | -CN | CH=CH-CH=N | |
| 4.10 | SH | $7-CH_3$ | $7-CH_3$ | -CN | CH=CH-CH=N | |
| 4.11 | H | H | H | $-CO-NH-CH_3$ | CH=CH-N=CH | |
| 4.12 | SH | H | H | $-CO-NH-CH_3$ | CH=CH-N=CH | |
| 4.13 | H | $7-CH_3$ | $7-CH_3$ | $-CO-NH-CH_3$ | CH=CH-N=CH | |
| 4.14 | SH | $7-CH_3$ | $7-CH_3$ | $-CO-NH-CH_3$ | CH=CH-N=CH | |
| 4.15 | H | $7-CH_3$ | $7-CH_3$ | $-CO-NH_2$ | CH=CH-N=CH | |
| 4.16 | SH | $7-CH_3$ | $7-CH_3$ | $-CO-NH_2$ | CH=CH-N=CH | |
| 4.17 | H | H | H | -CN | CH=CH-N=CH | |
| 4.18 | SH | H | H | -CN | CH=CH-N=CH | |
| 4.19 | H | $7-CH_3$ | $7-CH_3$ | -CN | CH=CH-N=CH | |
| 4.20 | SH | $7-CH_3$ | $7-CH_3$ | -CN | CH=CH-N=CH | |
| 4.21 | H | H | H | $-CO-NH-CH_3$ | CH=N-CH=CH | |
| 4.22 | SH | H | H | $-CO-NH-CH_3$ | CH=N-CH=CH | |
| 4.23 | H | $7-CH_3$ | $7-CH_3$ | $-CO-NH-CH_3$ | CH=N-CH=CH | |
| 4.24 | SH | $7-CH_3$ | $7-CH_3$ | $-CO-NH-CH_3$ | CH=N-CH=CH | |

| Comp. No. | $R^1$ | $R^6$ | $R^7$ | L | $A^1=A^2-A^3=A^4$ | physical data |
|---|---|---|---|---|---|---|
| 4.25 | H | 7-$CH_3$ | 7-$CH_3$ | -CO-$NH_2$ | CH=N-CH=CH | |
| 4.26 | SH | 7-$CH_3$ | 7-$CH_3$ | -CO-$NH_2$ | CH=N-CH=CH | |
| 4.27 | H | H | H | -CN | CH=N-CH=CH | |
| 4.28 | SH | H | H | -CN | CH=N-CH=CH | |
| 4.29 | H | 7-$CH_3$ | 7-$CH_3$ | -CN | CH=N-CH=CH | |
| 4.30 | SH | 7-$CH_3$ | 7-$CH_3$ | -CN | CH=N-CH=CH | |
| 4.31 | H | H | H | -CO-NH-$CH_3$ | N=CH-CH=CH | |
| 4.32 | SH | H | H | -CO-NH-$CH_3$ | N=CH-CH=CH | |
| 4.33 | H | 7-$CH_3$ | 7-$CH_3$ | -CO-NH-$CH_3$ | N=CH-CH=CH | |
| 4.34 | SH | 7-$CH_3$ | 7-$CH_3$ | -CO-NH-$CH_3$ | N=CH-CH=CH | |
| 4.35 | H | 7-$CH_3$ | 7-$CH_3$ | -CO-$NH_2$ | N=CH-CH=CH | |
| 4.36 | SH | 7-$CH_3$ | 7-$CH_3$ | -CO-$NH_2$ | N=CH-CH=CH | |
| 4.37 | H | H | H | -CN | N=CH-CH=CH | |
| 4.38 | SH | H | H | -CN | N=CH-CH=CH | |
| 4.39 | H | 7-$CH_3$ | 7-$CH_3$ | -CN | N=CH-CH=CH | |
| 4.40 | SH | 7-$CH_3$ | 7-$CH_3$ | -CN | N=CH-CH=CH | |

Table 5:

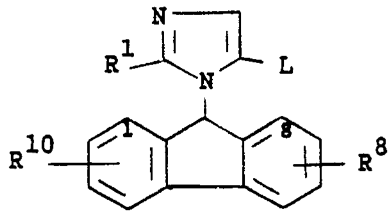

| Comp.No. | $R^1$ | $R^{10}$ | $R^8$ | L | physical data |
|---|---|---|---|---|---|
| 5.01 | H | H | H | $-CO-NH-CH_3$ | mp. 198°C |
| 5.02 | SH | H | H | $-CO-NH-CH_3$ | |
| 5.03 | H | H | H | $-CO-NH_2$ | mp. 201.1°C |
| 5.04 | SH | H | H | $-CO-NH_2$ | |
| 5.05 | H | H | H | -CN | |
| 5.06 | SH | H | H | -CN | |
| 5.07 | H | H | H | $-CO-SCH_3$ | |
| 5.08 | H | H | H | $-CO-NH-OCH_3$ | |
| 5.09 | H | $1-CH_3$ | H | $-CO-NH-CH_3$ | |
| 5.10 | H | $1-CH_3$ | $8-CH_3$ | $-CO-NH-CH_3$ | |
| 5.11 | H | H | H | -CO- 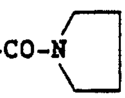 | mp. 163.4°C |
| 5.12 | H | H | H | -CO- 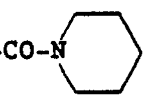 | mp. 159.0°C |
| 5.13 | H | H | H | $-CO-NH-CH_2C \equiv CH$ | mp. 174.6°C |
| 5.14 | H | H | H | $-CO-NH-CH_2-CH_3$ | mp. 148.3°C |
| 5.15 | H | H | H | $-CO-N(CH_3)_2$ | mp. 140.4°C |
| 5.16 | H | H | H | -CO- 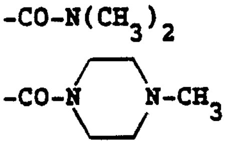 | mp. 200.6°C |
| 5.17 | H | H | H | $-CO-NH-CH(CH_3)_2$ | mp. 176.3°C |
| 5.18 | H | H | H | $-CO-NH-CH_2-CH=CH_2$ | mp. 72.6°C |
| 5.19 | H | H | H | $-CO-NH-C_6H_{11}-c$ | mp. 174.1°C |
| 5.20 | H | H | H | -CO- 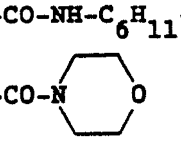 | mp. 177.4°C |

| Comp.No. | $R^1$ | $R^6$ | $R^8$ | L | physical data |
|---|---|---|---|---|---|
| 5.21 | H | H | H | -CO-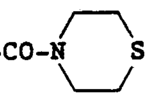 | mp. 201.0°C |
| 5.22 | H | H | H | $-CO-NH-C_2H_5$ | |
| 5.23 | H | H | H | -CO-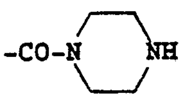 | mp. 158.3°C |
| 5.24 | H | H | H | $-CO-NH-C_3H_5$-c | HCl/mp.237.6°C |
| 5.25 | H | H | H | -CO-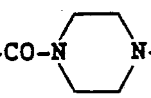$-CO-CH_3$ | $HNO_3$/mp.187.8°C |
| 5.26 | H | H | H | $-COO-N=C(CH_3)_2$ | mp. 150.1°C |
| 5.27 | H | H | H | -COO-N=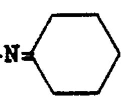 | mp. 146.8°C |

Table 6:

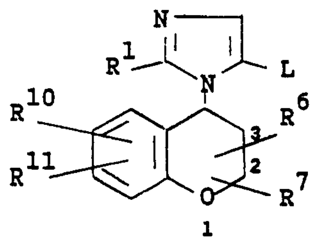

| Comp. No. | $R^1$ | $R^6$ | $R^7$ | $R^{10}$ | $R^{11}$ | L | physical data |
|---|---|---|---|---|---|---|---|
| 6.01 | H | 2-$CH_3$ | 2-$CH_3$ | H | H | -CO-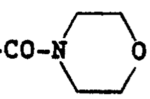 | mp. 132°C |
| 6.02 | H | 2-$CH_3$ | 2-$CH_3$ | H | H | -CO-NH-$C_2H_5$ | mp. 188°C |
| 6.03 | H | 2-$CH_3$ | 2-$CH_3$ | H | H | -CO-NH-$C_3H_7$-i | mp. 153°C |
| 6.04 | H | 2-$CH_3$ | 2-$CH_3$ | H | H | -CO-NH-$C_3H_7$-n | mp. 191°C |
| 6.05 | H | 2-$CH_3$ | 2-$CH_3$ | H | H | -CO-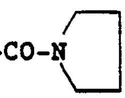 | mp. 115°C |
| 6.06 | H | 2-$CH_3$ | 2-$CH_3$ | H | H | -CO-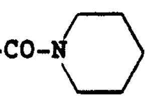 | mp. 128°C |
| 6.07 | H | 2-$CH_3$ | 2-$CH_3$ | H | H | -CO-NH-$CH_3$ | mp. 188°C |
| 6.08 | H | 2-$CH_3$ | 2-$CH_3$ | H | H | -CO-S-$CH_3$ | mp. 120°C |
| 6.09 | H | 2-$CH_3$ | 2-$CH_3$ | H | H | -CO-N($CH_3$)$_2$ | mp. 178°C |
| 6.10 | H | 2-$CH_3$ | 2-$CH_3$ | H | H | -CO-$NH_2$ | mp. 156°C |
| 6.11 | H | 2-$CH_3$ | 2-$CH_3$ | H | H | -CO-NH-$CH_2$-$CH_2$OH | mp. 178°C |
| 6.12 | H | 2-$CH_3$ | 2-$CH_3$ | H | H | -CO-NH-$NH_2$ | mp. 168.2°C |
| 6.13 | H | 2-$CH_3$ | 2-$CH_3$ | H | H | -CN | |
| 6.14 | H | 2-$CH_3$ | 2-$CH_3$ | H | H | -CO-NH-$C_4H_9$-n | |
| 6.15 | H | 2-$CH_3$ | H | H | H | -CO-$NH_2$ | |
| 6.16 | H | 2-$CH_3$ | H | H | H | -CO-NH-NH-$CH_3$ | |
| 6.17 | H | 2-$CH_3$ | H | H | H | -CO-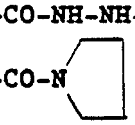 | |
| 6.18 | H | 2-$CH_3$ | H | H | H | -CO-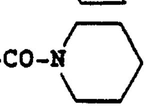 | |

| Comp. No. | $R^1$ | $R^6$ | $R^7$ | $R^{10}$ | $R^{11}$ | L | physical data |
|---|---|---|---|---|---|---|---|
| 6.19 | H | 2-$CH_3$ | H | H | H | -CO-NH-$CH_2$-$CH_2$OH | |
| 6.20 | H | 2-$CH_3$ | H | H | H | -CO-N$(CH_3)_2$ | |
| 6.21 | H | 2-$CH_3$ | H | H | H | -CO-NH-$C_3H_7$-n | |
| 6.22 | H | 3-$CH_3$ | H | H | H | -CO-NH-$CH_3$ | |
| 6.23 | H | 3-$CH_3$ | H | H | H | -CO-NH-$C_3H_7$-n | |
| 6.24 | H | 2-$CH_3$ | 2-$CH_3$ | H | H | -CO-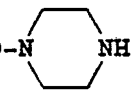 | mp. 157.5°C |
| 6.25 | H | 2-$CH_3$ | 2-$CH_3$ | H | H | -CO-NH-OH | mp. 149°C |
| 6.26 | SH | 2-$CH_3$ | 2-$CH_3$ | H | H | -CO-NH-$CH_3$ | |
| 6.27 | H | 2-$CH_3$ | 2-$CH_3$ | H | H | -CO-$SCH_3$ | |
| 6.28 | H | 2-$CH_3$ | 2-$CH_3$ | H | H | -CO-NH-$CH_2$-CH=$CH_2$ | mp. 168°C |
| 6.29 | H | 2-$CH_3$ | 2-$CH_3$ | H | H | -CO-NH-$CH_2$-C≡CH | mp. 140.1°C |
| 6.30 | H | 2-$CH_3$ | 2-$CH_3$ | H | H | -CO-NH-NH-$CH_3$ | mp. 188°C |
| 6.31 | H | H | H | H | H | -CO-NH-$CH_3$ | mp. 192°C |
| 6.32 | H | 2-$(CH_2)_4$-3 | | H | H | -CO-NH-$CH_3$ | $HNO_3$/mp. 162°C |
| 6.33 | H | 2-$(CH_2)_4$-3 | | H | 6-Br | -CO-NH-$CH_3$ | $HNO_3$/mp. 160°C |
| 6.34 | H | 2-$(CH_2)_3$-3 | | H | H | -CO-NH-$CH_3$ | |
| 6.35 | H | 2-$CH_3$ | H | H | H | -CO-NH-$CH_3$ | cis/mp.247.1°C |
| 6.36 | H | 2-$CH_3$ | H | H | H | -CO-NH-$CH_3$ | trans/$HNO_3$ mp. 179.2°C |
| 6.37 | H | 2-$CH_3$ | 2-$CH_3$ | H | H | 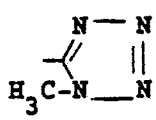 | |
| 6.38 | H | 2-$CH_3$ | 2-$CH_3$ | H | H | -C$(NH_2)$=N-OH | |
| 6.39 | H | 2-$CH_3$ | 2-$CH_3$ | H | H | 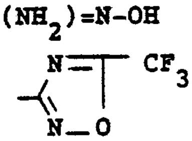 | |
| 6.40 | H | 2-$CH_3$ | 2-$CH_3$ | H | H | 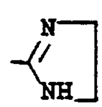 | |

| Comp. No. | $R^1$ | $R^6$ | $R^7$ | $R^{10}$ | $R^{11}$ | L | physical data |
|---|---|---|---|---|---|---|---|
| 6.41 | H | $2-CH_3$ | $2-CH_3$ | H | H | 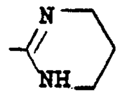 | |
| 6.42 | H | $2-CH_3$ | H | H | H | $-CO-NH_2$ | trans |
| 6.43 | H | $2-CH_3$ | H | H | H | $-CO-NH_2$ | cis |
| 6.44 | H | $2-CH_3$ | H | H | H | $-CO-NH-CH_3$ | trans/$HNO_3$ mp.176.2°C |
| 6.45 | H | $2-CH_3$ | H | H | H | $-CO-NH-CH_3$ | cis |
| 6.46 | H | $2-CH_3$ | H | H | H | $-CO-NH-C_2H_5$ | trans |
| 6.47 | H | $2-CH_3$ | H | H | H | $-CO-NH-C_2H_5$ | cis |
| 6.48 | H | $2-CH_3$ | H | H | H | $-CO-NH-C_3H_7-n$ | trans |
| 6.49 | H | $2-CH_3$ | H | H | H | $-CO-NH-C_3H_7-n$ | cis |
| 6.50 | H | $2-CH_3$ | H | H | H | $-CO-N(CH_3)_2$ | trans |
| 6.51 | H | $2-CH_3$ | H | H | H | $-CO-N(CH_3)_2$ | cis |
| 6.52 | H | $2-CH_3$ | H | H | H | -CO- 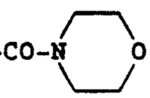 | trans |
| 6.53 | H | $2-CH_3$ | H | H | H | -CO- 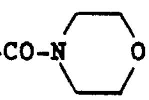 | cis |
| 6.54 | H | $2-CH_3$ | H | H | H | -CO- 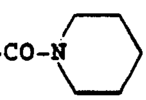 | trans |
| 6.55 | H | $2-CH_3$ | H | H | H | -CO- 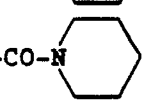 | cis |
| 6.56 | H | $2-CH_3$ | H | H | H | -CO- 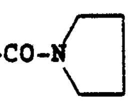 | trans |
| 6.57 | H | $2-CH_3$ | H | H | H | -CO- 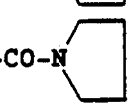 | cis |
| 6.58 | H | $2-CH_3$ | H | H | H | $-CO-NH-NH_2$ | trans |
| 6.59 | H | $2-CH_3$ | H | H | H | $-CO-NH-NH_2$ | cis |
| 6.60 | H | $2-CH_3$ | H | H | H | -CN | trans |
| 6.61 | H | $2-CH_3$ | H | H | H | -CN | cis |
| 6.62 | H | $2-CH_3$ | H | H | H | $-CO-NH-NH-CH_3$ | trans |

| Comp. No. | $R^1$ | $R^6$ | $R^7$ | $R^{10}$ | $R^{11}$ | L | physical data |
|---|---|---|---|---|---|---|---|
| 6.63 | H | $2-CH_3$ | H | H | H | $-CO-NH-NH-CH_3$ | cis |
| 6.64 | H | $2-CH_3$ | H | H | H | -CO-NH-OH | trans |
| 6.65 | H | $2-CH_3$ | H | H | H | -CO-NH-OH | cis |
| 6.66 | H | H | H | 8-Cl | H | $-CO-NH-CH_3$ | $HNO_3$/mp. 183.1°C |
| 6.67 | H | $2-(CH_2)_4-2$ | | 6-Br | H | $-CO-NH-CH_3$ | $HNO_3$/mp. 176.2°C |
| 6.68 | H | $2-(CH_2)_4-2$ | | H | H | $-CO-NH-CH_3$ | $HNO_3$/mp. 157.2°C |
| 6.69 | H | H | H | 6-Cl | H | $-CO-NH-CH_3$ | $HNO_3$/mp. 181.6°C |
| 6.70 | H | $2-CH_3$ | $2-CH_3$ | H | H | $-CO-N(CH_3)-OCH_3$ | mp. 104.7°C |
| 6.71 | H | $2-CH_3$ | $2-CH_3$ | H | H | $-CO-NHOCH_3$ | mp. 214.1°C |
| 6.72 | H | $2-CH_3$ | $2-CH_3$ | H | H | $-CO-NH-CH(CH_2-OH)-CH_2-CH_3$ | mp. 163.3°C |
| 6.73 | H | $2-CH_3$ | $2-CH_3$ | H | H | $-CO-NH-O-CH_2-C_6H_5$ | mp. 86.3°C |
| 6.74 | H | $2-C_2H_5$ | H | H | H | $-CO-NH-CH_3$ | cis/mp. 205.6°C |
| 6.75 | H | $2-CH_3$ | $2-CH_3$ | H | H | $-CO-NH-(CH_2)_2OCH_3$ | mp. 141.2°C |
| 6.76 | H | $2-CH_3$ | $3-CH_3$ | H | H | $-CO-NH-CH_3$ | (A+B)/$HNO_3$ mp. 157.3°C |
| 6.77 | H | $2-CH_3$ | $2-CH_3$ | H | H | 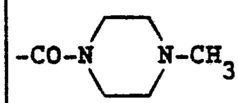 | mp. 119.7°C |
| 6.78 | H | $2-CH_3$ | $2-CH_3$ | H | H | $-CO-NH-CH_2-COOCH_3$ | |
| 6.79 | H | $2-CH_3$ | $2-CH_3$ | H | H | $-CO-NH-CH_2-CH_2-$ $O-CO-CH_3$ | mp. 106.4°C |
| 6.80 | H | $2-CH_3$ | $2-CH_3$ | H | H | 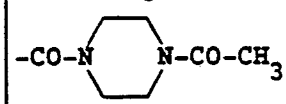 | mp. 176.2°C |
| 6.81 | H | $2-CH_3$ | $2-CH_3$ | H | H | 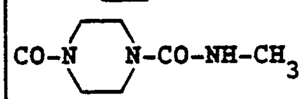 | mp. 115.9°C |
| 6.82 | H | $2-CH_3$ | H | 6-F | H | $-CO-NH-CH_3$ | trans/$HNO_3$ mp. 185.2°C |
| 6.83 | H | $2-CH_3$ | H | 6-F | H | $-CO-NH-CH_3$ | cis/mp. 208.2°C |
| 6.84 | H | $2-CH_3$ | H | H | H | $-COO-N=C(CH_3)$ | |
| 6.85 | H | $2-CH_3$ | H | H | H | $COO-CH_2-CH_2-Si(CH_3)_3$ | |

| Comp. No. | $R^1$ | $R^6$ | $R^7$ | $R^{10}$ | $R^{11}$ | L | physical data |
|---|---|---|---|---|---|---|---|
| 6.86 | H | $2-CH_3$ | H | H | H | $COO-CH_2-COOCH_3$ | |
| 6.87 | H | $2-CH_3$ | H | H | H | $COO-(CH_2)_3-Cl$ | |
| 6.88 | H | $2-CH_3$ | H | H | H | $COO-CH_2COOH$ | |
| 6.89 | H | $2-CH_3$ | $2-CH_3$ | H | H | $-COO-N=C(CH_3)_2$ | $HNO_3$/mp. 146.9°C |
| 6.90 | H | $2-CH_3$ | $2-CH_3$ | H | H | -COO-N= 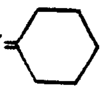 | $HNO_3$/mp. 144.5°C |
| 6.91 | SH | $2-CH_3$ | $3-CH_3$ | H | H | $CONH-CH_3$ | |
| 6.92 | H | $2-CH_3$ | $3-CH_3$ | H | H | $CONH-CH_3$ | |
| 6.93 | H | $2-CH_3$ | $3-CH_3$ | H | H | $CONH-CH_3$ | 2,3-cis; 3,4-trans .$HNO_3$/mp. 189.5°C |
| 6.94 | H | $2-CH_3$ | $3-CH_3$ | H | H | $CONH-CH_3$ | 2,3-cis; 3,4-cis |
| 6.95 | H | $2-CH_3$ | $3-CH_3$ | H | H | $CONH-CH_3$ | 2,3-trans; 3,4-cis |
| 6.96 | H | $2-CH_3$ | $3-CH_3$ | H | H | $CONH-CH_3$ | 2,3-trans 3,4-trans |
| 6.97 | H | $2-CH_3$ | $3-CH_3$ | H | H | $CONH-CH_3$ | 2,3-cis 3,4-trans(+) |
| 6.98 | H | $2-CH_3$ | $3-CH_3$ | H | H | $CONH-CH_3$ | 2,3-cis 3,4trans(-) |
| 6.99 | H | $2-CH_3$ | $3-CH_3$ | H | H | $CONH-CH_3$ | 2,3-cis;3,4-cis(+) |
| 6.100 | H | $2-CH_3$ | $3-CH_3$ | H | H | $CONH-CH_3$ | 2,3-cis;3,4-cis(-) |
| 6.101 | H | $2-CH_3$ | $3-CH_3$ | H | H | $CONH-CH_3$ | 2,3-trans 3,4-cis (+) |
| 6.102 | H | $2-CH_3$ | $3-CH_3$ | H | H | $CONH-CH_3$ | 2,3-trans 3,4-cis (-) |
| 6.103 | H | $2-CH_3$ | $3-CH_3$ | H | H | $CONH-CH_3$ | 2,3-trans 3,4-trans (+) |
| 6.104 | H | $2-CH_3$ | $3-CH_3$ | H | H | $CONH-CH_3$ | 2,3-trans 3,4-trans (-) |
| 6.105 | H | $2-C_3H_7-i$ | H | H | H | $CONH-CH_3$ | trans/$HNO_3$/1/2$H_2O$ mp. 156.3°C |
| 6.106 | H | $2-C_4H_9-t$ | H | H | H | $CONH-CH_3$ | trans/$HNO_3$/$H_2O$ mp. 156.3°C |

Table 7:

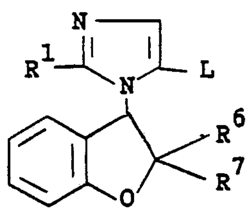

| Comp. No. | $R^1$ | $R^6$ | $R^7$ | L | physical data |
|---|---|---|---|---|---|
| 7.1 | SH | H | H | $CO-NH-CH_3$ | |
| 7.2 | H | H | H | $CO-NH-CH_3$ | |
| 7.3 | SH | $CH_3$ | $CH_3$ | $CO-NH-CH_3$ | |
| 7.4 | H | $CH_3$ | $CH_3$ | $CO-NH-CH_3$ | $.HNO_3$/mp. 190.8°C |
| 7.5 | H | $CH_3$ | $CH_3$ | $CO-NH-CH_3$ | (+) |
| 7.6 | H | $CH_3$ | $CH_3$ | $CO-NH-CH_3$ | (-) |
| 7.7 | H | $CH_3$ | H | $CO-NH-CH_3$ | (-) |
| 7.8 | H | $CH_3$ | H | $CO-NH-CH_3$ | trans |
| 7.9 | H | $CH_3$ | H | $CO-NH-CH_3$ | cis |
| 7.10 | H | $CH_3$ | $CH_3$ | CN | |
| 7.11 | H | $CH_3$ | $CH_3$ | $CO-NH_2$ | |
| 7.12 | H | H | H | $CO-NH-OCH_3$ | |
| 7.13 | H | H | H | $CO-S-CH_3$ | |
| 7.14 | H | H | H | $CO-NH-(CH_2)_2-CH_3$ | |
| 7.15 | H | H | H | $CO-N(CH_3)_2$ | |

Table 8:

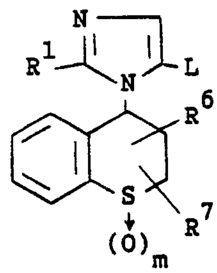

| comp. No. | $R^1$ | m | $R^6$ | $R^7$ | L | physical data |
|---|---|---|---|---|---|---|
| 8.01 | H | 0 | H | H | $CONHCH_3$ | HCl 1/2 $H_2O$ mp. 176.3°C |
| 8.02 | SH | 0 | H | H | $CONHCH_3$ | mp. 163.3°C |
| 8.03 | H | 0 | $3-CH_3$ | $3-CH_3$ | $CONHCH_3$ | |
| 8.04 | SH | 0 | $3-CH_3$ | $3-CH_3$ | $CONHCH_3$ | |
| 8.05 | H | 0 | H | H | CN | $HNO_3$/mp. 136.5°C |
| 8.06 | H | 0 | H | H | $CONH_2$ | |
| 8.07 | H | 1 | H | H | $CONHCH_3$ | |
| 8.08 | SH | 1 | H | H | $CONHCH_3$ | |
| 8.09 | H | 1 | $3-CH_3$ | $3-CH_3$ | $CONHCH_3$ | |
| 8.10 | SH | 1 | $3-CH_3$ | $3-CH_3$ | $CONHCH_3$ | |
| 8.11 | H | 2 | H | H | $CONHCH_3$ | |
| 8.12 | SH | 2 | H | H | $CONHCH_3$ | |
| 8.13 | H | 2 | $3-CH_3$ | $3-CH_3$ | $CONHCH_3$ | |
| 8.14 | SH | 2 | $3-CH_3$ | $3-CH_3$ | $CONHCH_3$ | |
| 8.15 | H | 0 | $2-CH_3$ | H | $CONHCH_3$ | |
| 8.16 | H | 0 | $2-CH_3$ | H | $CONHCH_3$ | cis |
| 8.17 | H | 0 | $2-CH_3$ | H | $CONHCH_3$ | trans |
| 8.18 | H | 0 | $2-CH_3$ | $3-CH_3$ | $CONHCH_3$ | |
| 8.19 | H | 0 | $2-CH_3$ | $3-CH_3$ | $CONHCH_3$ | 2,3-cis/3,4-trans |
| 8.20 | H | 0 | $2-CH_3$ | $3-CH_3$ | $CONHCH_3$ | 2,3-cis/3,4-cis |
| 8.21 | H | 0 | $2-CH_3$ | $3-CH_3$ | $CONHCH_3$ | 2,3-trans/3,4-cis |
| 8.22 | H | 0 | $2-CH_3$ | $3-CH_3$ | $CONHCH_3$ | 2,3-trans/3,4-trans |

| comp. No. | $R^1$ | m | $R^6$ | $R^7$ | L | physical data |
|---|---|---|---|---|---|---|
| 8.23 | H | 1 | $2-CH_3$ | H | $CONHCH_3$ | |
| 8.24 | H | 2 | $2-CH_3$ | H | $CONHCH_3$ | |
| 8.25 | H | 0 | $2-C_2H_5$ | H | $CONHCH_3$ | |
| 8.26 | H | 0 | $2-C_2H_5$ | H | $CONHCH_3$ | cis |
| 8.27 | H | 0 | $2-C_2H_5$ | H | $CONHCH_3$ | trans |

Table 9:

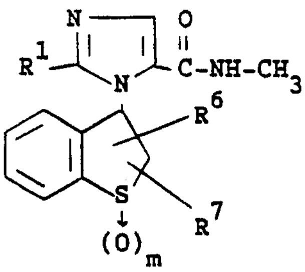

| comp. No. | $R^1$ | m | $R^6$ | $R^7$ | physical data |
|---|---|---|---|---|---|
| 9.01 | H | 0 | H | H | |
| 9.02 | SH | 0 | H | H | |
| 9.03 | H | 0 | $2-CH_3$ | $2-CH_3$ | |
| 9.04 | SH | 0 | $2-CH_3$ | $2-CH_3$ | |
| 9.05 | H | 1 | H | H | |
| 9.06 | SH | 1 | H | H | |
| 9.07 | H | 1 | $2-CH_3$ | $2-CH_3$ | |
| 9.08 | SH | 1 | $2-CH_3$ | $2-CH_3$ | |
| 9.09 | H | 2 | H | H | |
| 9.10 | SH | 2 | H | H | |
| 9.11 | H | 2 | $2-CH_3$ | $2-CH_3$ | |
| 9.12 | SH | 2 | $2-CH_3$ | $2-CH_3$ | |
| 9.13 | H | 0 | $2-CH_3$ | $2-CH_3$ | (+) |
| 9.14 | H | 0 | $2-CH_3$ | $2-CH_3$ | (-) |
| 9.15 | H | 0 | $2-C_2H_5$ | $2-C_2H_5$ | |
| 9.16 | H | 0 | $2-C_2H_5$ | $2-C_2H_5$ | (+) |
| 9.17 | H | 0 | $2-C_2H_5$ | $2-C_2H_5$ | (-) |
| 9.18 | H | 0 | $2-CH_3$ | H | |
| 9.20 | H | 0 | $2-CH_3$ | H | cis |
| 9.21 | H | 0 | $2-CH_3$ | H | trans |
| 9.22 | H | 0 | $2-C_2H_5$ | H | |

| comp. No. | $R^1$ | m | $R^6$ | $R^7$ | physical data |
|---|---|---|---|---|---|
| 9.23 | H | 0 | 2-$C_2H_5$ | H | cis |
| 9.24 | H | 0 | 2-$C_2H_5$ | H | trans |
| 9.25 | H | 1 | 2-$CH_3$ | H | |
| 9.26 | H | 1 | 2-$C_2H_5$ | H | |
| 9.27 | H | 1 | 2-$C_2H_5$ | 2-$C_2H_5$ | |
| 9.28 | H | 2 | 2-$CH_3$ | H | |
| 9.29 | H | 2 | 2-$C_2H_5$ | H | |
| 9.30 | H | 2 | 2-$C_2H_5$ | 2-$C_2H_5$ | |
| 9.31 | H | 2 | 2-$CH_3$ | H | cis |
| 9.32 | H | 2 | 2-$CH_3$ | H | trans |
| 9.33 | H | 2 | 2-$C_2H_5$ | H | cis |
| 9.35 | H | 2 | 2-$C_2H_5$ | H | trans |

Table 10:

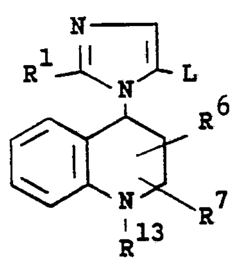

| Comp.No. | $R^1$ | $R^6$ | $R^7$ | $R^{13}$ | L |
|---|---|---|---|---|---|
| 10.01 | H | H | H | $CH_3$ | CO-NH-$CH_3$ |
| 10.02 | SH | H | H | $CH_3$ | CO-NH-$CH_3$ |
| 10.03 | H | H | H | $C_2H_5$ | CO-NH-$CH_3$ |
| 10.04 | SH | H | H | $C_2H_5$ | CO-NH-$CH_3$ |
| 10.05 | H | H | H | CO-$CH_3$ | CO-NH-$CH_3$ |
| 10.06 | H | 3-$CH_3$ | 3-$CH_3$ | $CH_3$ | CO-NH-$CH_3$ |
| 10.07 | H | 3-$CH_3$ | 3-$CH_3$ | $CH_3$ | CN |
| 10.08 | SH | 3-$CH_3$ | 3-$CH_3$ | $CH_3$ | CO-$NH_2$ |

Table 11:

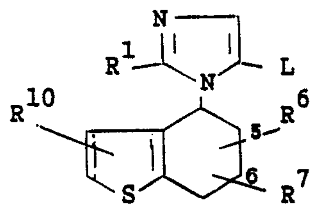

| Comp. No. | $R^1$ | $R^6$ | $R^7$ | $R^{10}$ | L | physical data |
|---|---|---|---|---|---|---|
| 11.01 | H | H | H | H | $-CO-NH_2$ | |
| 11.02 | H | H | H | H | -CN | |
| 11.03 | H | H | H | H | $-CO-NH-CH_3$ | mp. 125.2°C |
| 11.04 | H | H | H | H | $-CO-NH-OCH_3$ | |
| 11.05 | H | H | H | H | $-CO-SCH_3$ | |
| 11.06 | H | $5-CH_3$ | $5-CH_3$ | H | $-CO-NH-CH_3$ | |
| 11.07 | H | $5-CH_3$ | $5-CH_3$ | H | $-CO-SCH_3$ | |
| 11.08 | H | $5-CH_3$ | $5-CH_3$ | H | $-CO-NH-OCH_3$ | |
| 11.09 | H | $6-CH_3$ | $6-CH_3$ | H | $-CO-NH-CH_3$ | |
| 11.10 | SH | $6-CH_3$ | $6-CH_3$ | H | $-CO-NH-CH_3$ | |
| 11.11 | SH | H | H | H | $-CO-NH_2$ | |

Table 12:

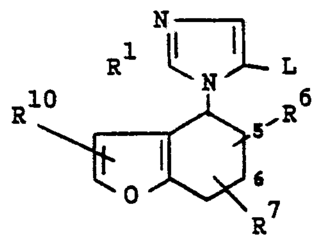

| Comp. No. | $R^1$ | $R^6$ | $R^7$ | $R^{10}$ | L | physical data |
|---|---|---|---|---|---|---|
| 12.01 | H | H | H | H | $-CO-NH_2$ | |
| 12.02 | H | H | H | H | -CN | |
| 12.03 | H | H | H | H | $-CO-NH-CH_3$ | |
| 12.04 | H | H | H | H | $-CO-NH-OCH_3$ | |
| 12.05 | H | H | H | H | $-CO-SCH_3$ | |
| 12.06 | H | $5-CH_3$ | $5-CH_3$ | H | $-CO-NH-CH_3$ | |
| 12.07 | H | $5-CH_3$ | $5-CH_3$ | H | $-CO-SCH_3$ | |
| 12.08 | H | $5-CH_3$ | $5-CH_3$ | H | $-CO-NH-OCH_3$ | |
| 12.09 | H | $6-CH_3$ | $6-CH_3$ | H | $-CO-NH-CH_3$ | |
| 12.10 | SH | $6-CH_3$ | $6-CH_3$ | H | $-CO-NH-CH_3$ | |
| 12.11 | SH | H | H | H | $-CO-NH_2$ | |

Table 13:

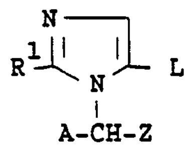

| Comp. No. | $R^1$ | A | Z | L | physical data |
|---|---|---|---|---|---|
| 13.01 | H | $C_3H_7$-n | 3-Cl-$C_6H_4$ | -CO-NH-$CH_3$ | |
| 13.02 | H | $C_3H_7$-n | 3-Cl-$C_6H_4$ | -CO-$NH_2$ | |
| 13.03 | H | $C_3H_7$-n | 3-Cl-$C_6H_4$ | -CO-$SCH_3$ | |
| 13.04 | H | $C_3H_7$-n | 3-Cl-$C_6H_4$ | -CO-NH-$OCH_3$ | |
| 13.05 | H | $C_3H_7$-n | 3-Cl-$C_6H_4$ | -CO-NH-$C_3H_7$-n | |
| 13.06 | H | $C_3H_7$-n | 2-Cl-$C_6H_4$ | -CO-NH-$CH_3$ | mp. 138°C |
| 13.07 | H | $C_3H_7$-n | 2-Cl-$C_6H_4$ | -CO-$NH_2$ | |
| 13.08 | H | $C_3H_7$-n | 2-Cl-$C_6H_4$ | -CO-NH-$C_2H_5$ | |
| 13.09 | H | $C_3H_7$-n | 2-Cl-$C_6H_4$ | -CO-S-$CH_3$ | |
| 13.10 | H | $C_3H_7$-n | 2-Cl-$C_6H_4$ | -CN | |
| 13.11 | H | $C_3H_7$-n | $C_6H_5$ | -CO-NH-$CH_3$ | $HNO_3$/mp. 181.3°C |
| 13.12 | H | $C_3H_7$-n | $C_6H_5$ | -CO-$NH_2$ | |
| 13.13 | H | $C_3H_7$-n | $C_6H_5$ | -CO-S-$CH_3$ | |
| 13.14 | H | $C_3H_7$-n | $C_6H_5$ | -CO-NH-$OCH_3$ | |
| 13.15 | H | $C_3H_7$-n | $C_6H_5$ | -CO-NH-$C_3H_7$-n | |
| 13.16 | H | benzyl | $C_6H_5$ | -CO-NH-$CH_3$ | $HNO_3$/mp. 202.2°C |
| 13.17 | H | benzyl | $C_6H_5$ | -CO-$NH_2$ | |
| 13.18 | H | benzyl | $C_6H_5$ | -CO-NH-$C_2H_5$ | |
| 13.19 | H | benzyl | $C_6H_5$ | -CO-$SCH_3$ | |
| 13.20 | H | benzyl | $C_6H_5$ | -CN | |
| 13.21 | H | $C_3H_7$-n | 2-$CH_3$-$C_6H_4$ | -CO-NH-$CH_3$ | |
| 13.22 | H | $C_3H_7$-n | 2-$CH_3$-$C_6H_4$ | -CO-$NH_2$ | |
| 13.23 | H | $C_3H_7$-n | 2-$CH_3$-$C_6H_4$ | -CO-$SCH_3$ | |
| 13.24 | H | $C_3H_7$-n | 2-$CH_3$-$C_6H_4$ | -CO-NH-$OCH_3$ | |
| 13.25 | H | $C_3H_7$-n | 2-$CH_3$-$C_6H_4$ | -CO-NH-$C_3H_7$-n | |
| 13.26 | H | 2-pyridinyl | $C_6H_5$ | -CO-NH-$CH_3$ | HCl/mp. 234.5°C |

| Comp. No. | $R^1$ | A | Z | L | physical data |
|---|---|---|---|---|---|
| 13.27 | H | 2-pyridinyl | $C_6H_5$ | $-CO-NH_2$ | |
| 13.28 | H | 2-pyridinyl | $C_6H_5$ | $-CO-N(CH_3)_2$ | mp. 154°C |
| 13.29 | H | 2-pyridinyl | $C_6H_5$ | $-CO-SCH_3$ | |
| 13.30 | H | 2-pyridinyl | $C_6H_5$ | -CN | |
| 13.31 | H | $C_3H_7$-i | 3-Cl-$C_6H_4$ | $-CO-NH-CH_3$ | |
| 13.32 | H | $C_3H_7$-i | 3-Cl-$C_6H_4$ | $-CO-NH_2$ | |
| 13.33 | H | $C_3H_7$-i | 3-Cl-$C_6H_4$ | $-CO-SCH_3$ | |
| 13.34 | H | $C_3H_7$-i | 3-Cl-$C_6H_4$ | $-CO-NH-OCH_3$ | |
| 13.35 | H | $C_3H_7$-i | 3-Cl-$C_6H_4$ | $-CO-NH-C_3H_7$-n | |
| 13.36 | H | $C_3H_7$-i | $C_6H_5$ | $-CO-NH-CH_3$ | |
| 13.37 | H | $C_3H_7$-i | $C_6H_5$ | $-CO-NH_2$ | |
| 13.38 | H | $C_3H_7$-i | $C_6H_5$ | $-CO-NH-C_2H_5$ | |
| 13.39 | H | $C_3H_7$-i | $C_6H_5$ | $-CO-S-CH_3$ | |
| 13.40 | H | $C_3H_7$-i | $C_6H_5$ | -CN | |
| 13.41 | H | cyclohexyl | $C_6H_5$ | $-CO-NH-CH_3$ | $HNO_3$/mp. 138°C |
| 13.42 | H | cyclohexyl | $C_6H_5$ | $-CO-NH_2$ | |
| 13.43 | H | cyclohexyl | $C_6H_5$ | $-CO-S-CH_3$ | |
| 13.44 | H | cyclohexyl | $C_6H_5$ | $-CO-NH-OCH_3$ | |
| 13.45 | H | cyclohexyl | $C_6H_5$ | $-CO-NH-C_3H_7$-n | |
| 13.46 | H | $C_3H_7$-n | 2-$OCH_3$-$C_6H_4$ | $-CO-NH-CH_3$ | |
| 13.47 | H | $C_3H_7$-n | 2-$OCH_3$-$C_6H_4$ | $-CO-NH_2$ | |
| 13.48 | H | $C_3H_7$-n | 2-$OCH_3$-$C_6H_4$ | $-CO-NH-C_2H_5$ | |
| 13.49 | H | $C_3H_7$-n | 2-$OCH_3$-$C_6H_4$ | $-CO-S-CH_3$ | |
| 13.50 | H | $C_3H_7$-n | 2-$OCH_3$-$C_6H_4$ | -CN | |
| 13.51 | H | $-CH_2-CH_2-C_6H_5$ | $C_6H_5$ | $-CO-NH-CH_3$ | $HNO_3$/mp. 135°C |
| 13.52 | H | $-CH_2-CH_2-C_6H_5$ | $C_6H_5$ | $-CO-NH_2$ | |
| 13.53 | H | $-CH_2-CH_2-C_6H_5$ | $C_6H_5$ | $-CO-SCH_3$ | |
| 13.54 | H | $-CH_2-CH_2-C_6H_5$ | $C_6H_5$ | $-CO-NH-OCH_3$ | |
| 13.55 | H | $-CH_2-CH_2-C_6H_5$ | $C_6H_5$ | $-CO-NH-C_3H_7$-n | |
| 13.56 | H | $CH(OC_2H_5)-C_6H_5$ | $C_6H_5$ | $-CO-NH-CH_3$ | $HNO_3$/mp. 185°C |

| Comp. No. | $R^1$ | A | Z | L | physical data | |
|---|---|---|---|---|---|---|
| 13.57 | H | $-CH(OC_2H_5)-C_6H_5$ | $C_6H_5$ | $-CO-NH_2$ | | |
| 13.58 | H | $-CH(OC_2H_5)-C_6H_5$ | $C_6H_5$ | $-CO-NH-C_2H_5$ | | |
| 13.59 | H | $-CH(OC_2H_5)-C_6H_5$ | $C_6H_5$ | $-CO-SCH_3$ | | |
| 13.60 | H | $-CH(OC_2H_5)-C_6H_5$ | $C_6H_5$ | -CN | | |
| 13.61 | H | $CH_3$ | $C_6H_5$ | $-CO-NH-CH_3$ | 1/2 $H_2O$ mp. 85-88°C | |
| 13.62 | H | $CH_3$ | $C_6H_5$ | $-CO-NH-NH_2$ | mp. 134-135.5°C | |
| 13.63 | H | $CH_3$ | $C_6H_5$ | $-CO-N(CH_3)_2$ | mp. 122-123°C | |
| 13.64 | H | $CH_3$ | $C_6H_5$ | $-CO-NH_2$ | mp. 136.5-137°C | |
| 13.65 | H | $CH_3$ | $C_6H_5$ | -CN | mp. 97-99°C | |
| 13.66 | H | $CH_3$ | $C_6H_5$ | -CO- 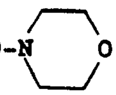 | HCl/mp.240-242°C | |
| 13.67 | H | $CH_3$ | $C_6H_5$ | $-CO-NH_2$ | (+)/mp. 115.9°C $[\alpha]_D$=+84.6° | * |
| 13.68 | H | $CH_3$ | $C_6H_5$ | $-CO-NH_2$ | (-)/mp. 123.8°C $[\alpha]_D$=-80.4° | * |
| 13.69 | H | $CH_3$ | $C_6H_5$ | -CN | (+)/mp. 129.3°C $[\alpha]_D$=+40.6° | * |
| 13.70 | SH | $CH_3$ | $C_6H_5$ | $-CO-NH_2$ | +/$H_2O$/mp.245.3°C $[\alpha]_D$=+84.7° | * |
| 13.71 | SH | $CH_3$ | $C_6H_5$ | $-CO-NH_2$ | -/$H_2O$/mp.244.8°C $[\alpha]_D$=-85.8° | * |
| 13.72 | SH | H | $C_6H_5$ | $-CO-NH_2$ | mp.255.3-262.2°C | |
| 13.73 | H | $CH_3$ | $C_6H_5$ | -CO- 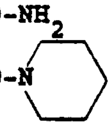 | mp. 160.5°C | |
| 13.74 | H | $CH_3$ | $C_6H_5$ | -CO- 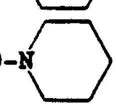 | (+)-R mp.184.0°C $[\alpha]_D$=+67.3° | * |
| 13.75 | H | $CH_3$ | $C_6H_5$ | -CO- 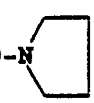 | (+)-R mp.126.4°C | |
| 13.76 | H | $CH_3$ | $C_6H_5$ | -CO- 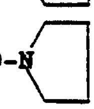 | mp.109.8°C | |

| Comp. No. | $R^1$ | A | Z | L | physical data |
|---|---|---|---|---|---|
| 13.77 | H | 2-pyridinyl | $C_6H_5$ | $-CO-NH-(CH_2)_2-OH$ | mp. 142.4°C |
| 13.78 | H | 1-naphthalenyl | $C_6H_5$ | $-CO-NH-CH_3$ | mp. 221.7°C |
| 13.79 | H | $C_3H_7$-n | 4-F-$C_6H_4$ | $-CO-NH-CH_3$ | $HNO_3$/mp. 178.5°C |
| 13.80 | H | 2-pyridinyl | $C_6H_5$ | $-CO-NH-CH_3$ | 2 HCl/mp.216.6°C |
| 13.81 | H | $C_3H_7$-n | 3-F-$C_6H_4$ | $-CO-NH-CH_3$ | oil |
| 13.82 | H | $C_3H_7$-n | 2-Br-$C_6H_4$ | $-CO-NH-CH_3$ | mp. 151.5°C |
| 13.83 | H | $C_3H_7$-n | 4-Cl-$C_6H_4$ | $-CO-NH-CH_3$ | HCl/mp. 111.9°C |
| 13.84 | H | $C_3H_7$-n | 3-Br-$C_6H_4$ | $-CO-NH-CH_3$ | oil |
| 13.85 | H | $C_3H_7$-n | 4-Br-$C_6H_4$ | $-CO-NH-CH_3$ | mp. 130.1°C |
| 13.86 | H | $C_3H_7$-i | 2-Cl-$C_6H_4$ | $-CO-NH-CH_3$ | mp. 181.5°C |
| 13.87 | H | $C_3H_7$-n | 2,4-$(Cl)_2$-$C_6H_3$ | -CN | |
| 13.88 | H | 2-pyridinyl | 2-$CH_3$-$C_6H_4$ | $-CO-NH-CH_3$ | mp. 206.5°C |
| 13.89 | H | $C_3H_7$-n | 2-Cl-$C_6H_4$ | $-COO-N=C(CH_3)_2$ | |
| 13.90 | H | $C_3H_7$-n | 2-Cl-$C_6H_4$ | $COO-CH_2-Si(CH_3)_3$ | |
| 13.91 | H | $C_3H_7$-n | 2-Cl-$C_6H_4$ | $-COO-CH_2-PO(OC_2H_5)_2$ | |
| 13.92 | H | $C_3H_7$-n | 2-Cl-$C_6H_4$ | $-COO-CH_2-PO_3H_2$ | |
| 13.93 | H | $C_3H_7$-n | 2-Cl-$C_6H_4$ | $-COO-(CH_2)_3-Cl$ | |
| 13.94 | H | $CH_3$ | $C_6H_5$ | $-COO-(CH_2)_2-Cl$ | HCl/ 1/2$H_2O$ mp. 83.5-85°C |
| 13.95 | H | $CH_3$ | $C_6H_5$ | $-COO-CH_2-C_3H_5$-c | HCl/ mp. 148.5-151°C |
| 13.96 | H | $CH_3$ | $C_6H_5$ | $COO-(CH_2)_2-C_6H_{11}$-c | HCl/mp.121-125°C |
| 13.97 | H | $CH_3$ | $C_6H_5$ | $COO-(CH_2)_2$- 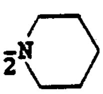 | bp.195-205°C (0.4 mmHg) |
| 13.98 | H | $CH_3$ | $C_6H_5$ | $-COO-CH_2-COCH_3$ | (R)-(+)/HCl mp.149.7°C [α] =+42.0° ** |
| 13.99 | H | 2-pyridinyl | 2-pyridinyl | $-CO-NH-CH_3$ | |
| 13.100 | H | 2-pyridinyl | 2,6-dimethylphenyl | $CO-NH-CH_3$ | |
| 13.101 | H | $C_2H_5$ | 2-Cl-$C_6H_4$ | -CO-NH-CH | mp. 214.4°C |

* = c= 1% in methanol
** = c = 0.1% in ethanol

Table 14:

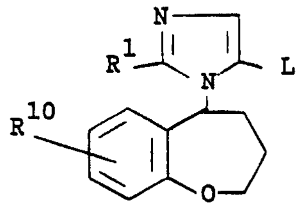

| Comp. No. | $R^1$ | $R^{10}$ | L | physical data |
|---|---|---|---|---|
| 14.1 | H | H | $-CO-NH-CH_3$ | |
| 14.2 | H | H | $-CO-NH-OCH_3$ | |
| 14.3 | H | H | $-CO-SCH_3$ | |
| 14.4 | H | H | $-CO-NH-C_3H_7-n$ | |
| 14.5 | H | H | $-CO-N(CH_3)_2$ | |
| 14.6 | H | $6-CH_3$ | $-CO-NHCH_3$ | |
| 14.7 | SH | $6-CH_3$ | $-CO-NHCH_3$ | |

Table 15:

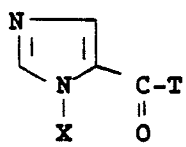

| Comp. No. | X | T | physical data |
|---|---|---|---|
| 15.1 | 2,3-dihydro-2,2-dimethyl-1H-indenyl | Cl | |
| 15.2 | 2,3-dihydro-2,2-dimethyl-1H-indenyl | Br | |
| 15.3 | 2,2-dimethyl-1,2,3,4-tetrahydronaphthalen-1-yl | Cl | |
| 15.4 | 2,3-dihydro-2,2-dimethyl-4H-1-benzopyran-4-yl | Cl | |
| 15.5 | 2,3-dihydro-2,3-dimethyl-4H-1-benzopyran-4-yl | Cl | |
| 15.6 | 2,3-dihydro-2,2-dimethylbenzofuran-3-yl | Cl | |
| 15.7 | 1-(2-pyridinyl)butyl | Cl | |
| 15.8 | 1-(2-methoxyphenyl)butyl | Cl | |
| 15.9 | 1-(2-chlorophenyl)butyl | Cl | |

C. Composition examples

Example 36: Composition examples for solid compounds of formula (I) (percentages are by weight)

| a) Wettable powders | a) | b) | c) |
|---|---|---|---|
| compound of formula (I) | 20% | 50% | 0.5% |
| sodium lignosulfonate | 5% | 5% | 5% |
| sodium laurylsulfate | 3% | - | - |
| sodium diisobutylnaphthalenesulfonate | - | 6% | 6% |
| octylphenol polyethylene glycol ether (7-8 moles of ethylene oxide) | - | 2% | 2% |
| highly dispersed silicic acid | 5% | 27% | 27% |
| kaolin | 67% | 10% | - |
| sodium chloride | - | - | 59.5% |

The active ingredient was thoroughly mixed with the adjuvants and the mixture was thoroughly ground in a suitable mill, affording wettable powders which could be diluted with water to give suspensions of the desired concentration.

| b) Emulsifiable concentrate | a) | b) |
|---|---|---|
| compound of formula (I) | 10% | 1% |
| octylphenol polyethylene glycol ether (4-5 moles of ethylene oxide) | 3% | 3% |
| calcium dodecylbenzenesulfonate | 3% | 3% |
| castor oil polyglycol ether (36 moles of ethylene oxide) | 4% | 4% |
| cyclohexanone | 30% | 10% |
| dimethylbenzene mixture | 50% | 79% |

Emulsions of any required concentration could be obtained from this concentrate by dilution with water.

| c) Dusts | a) | b) |
|---|---|---|
| compound of formula (I) | 0.1% | 1% |
| talcum | 99.9% | - |
| kaolin | - | 99% |

Usable dusts were obtained by mixing the active ingredient with the carriers, and grinding the mixture in a suitable mill.

d) Extruder granulate

| | a) | b) |
|---|---|---|
| compound of formula (I) | 10% | 1% |
| sodium lignosulfate | 2% | 2% |
| carboxymethylcellulose | 1% | 1% |
| kaolin | 87% | 96% |

The active ingredient was mixed and ground with the adjuvants, and the mixture was subsequently moistened with water. The mixture was extruded and dried in a stream of air.

e) Coated granulate

| | |
|---|---|
| compound of formula (I) | 3% |
| polyethylene glycol (mol. wt. 200) | 2% |
| kaolin | 95% |

The finely ground active ingredient was uniformly applied, in a mixer, to the kaolin moistened with polyethylene glycol. Non-dusty coated granulates were obtained in this manner.

f) Suspension concentrate

| | a) | b) |
|---|---|---|
| compound of formula (I) | 40% | 5% |
| ethylene glycol | 10% | 10% |
| nonylphenol polyethylene glycol ether (15 moles of ethylene oxide) | 6% | 1% |
| sodium lignosulfate | 10% | 5% |
| carboxymethylcellulose | 1% | 1% |
| 37% aqueous formaldehyde solution | 0.2% | 0.2% |
| silicone oil in the form of a 75% aqueous emulsion | 0.8% | 0.8% |
| water | 32% | 77% |

The finely ground active ingredient was intimately mixed with the adjuvants, giving a suspension concentrate from which suspension of any desired concentration could be obtained by dilution with water.

g) Salt solution

| | |
|---|---|
| compound of formula (I) | 5% |
| isopropylamine | 1% |
| octylphenol polyethylene glycol ether (78 moles of ethylene oxide) | 3% |
| water | 91% |

Example 37: Composition examples for liquid active ingredients of formula (I) (throughout, percentages are by weight)

a) Emulsifiable concentrates

| | a) | b) | c) |
|---|---|---|---|
| compound of formula (I) | 20% | 40% | 50% |
| calcium dodecylbenzenesulfonate | 5% | 8% | 5.8% |
| castor oil polyethylene glycol ether (36 moles of ethylene oxide) | 5% | - | - |
| tributylphenol polyethylene glycol ether (30 moles of ethylene oxide) | - | 12% | 4.2% |
| cyclohexanone | - | 15% | 20% |
| dimethylbenzene mixture | 70% | 25% | 20% |

Emulsions of any required concentration could be produced from such concentrate by dilution with water.

b) Solutions

| | a) | b) | c) | d) |
|---|---|---|---|---|
| compound of formula (I) | 80% | 10% | 5% | 95% |
| ethylene glycol monoethyl ether | 20% | - | - | - |
| polyethylene glycol (MG 400) | - | 70% | - | - |
| N-methyl-2-pyrrolidone | - | 20% | - | - |
| epoxidised coconut oil | - | - | 1% | 5% |
| petroleum distillate (boiling range 160-190°C) | - | - | 94% | - |

These solutions were suitable for application in the form of microdrops.

c) Granulates

| | a) | b) |
|---|---|---|
| compound of formula (I) | 5% | 10% |
| kaolin | 94% | - |
| highly dispersed silicic acid | 1% | - |
| attapulgite | - | 90% |

The active ingredient was dissolved in methylene chloride, the solution was sprayed onto the carrier, and the solvent was subsequently evaporated off in vacuo.

d) Dusts

| | a) | b) |
|---|---|---|
| compound of formula (I) | 2% | 5% |
| highly dispersed silicic acid | 1% | 5% |
| talcum | 97% | - |
| kaolin | - | 90% |

D. Biological examples

Example 38: Preemergence herbidicidal action

In a greenhouse, immediately after sowing the test plants in seed dishes, the surface of the soil was treated with an aqueous dispersion of the test compounds, obtained from a 25% emulsifiable concentrate or from a 25% wettable powder with test compounds, which, on account of their insufficient solubility, could not be formulated to emulsifiable concentrates. Two different concentration series were used, corresponding to 1 and 0.5 kg of test compound per hectare respectively. The seed dishes were kept in the greenhouse at 22˜25°C and 50˜70% relative humidity. The test was evaluated 3 weeks later in accordance with the following rating:

1 = plants had not germinated or were totally withered
2-3 = very strong action
4-6 = average action
7-8 = slight action
9 = no action

**Results: Preemergence test**

**dosage: 4 kg active ingredient per hectare**

| plant tested / compound tested | digitaria | poa | bromus | setaria | echinochloa | avena fatua | cynodon |
|---|---|---|---|---|---|---|---|
| 2.01 | 1 | 1 | 1 | 3 | 1 | 2 | 2 |
| 2.02 | 1 | 1 | 1 | 1 | 1 | 2 | 1 |
| 2.03 | 1 | 1 | 1 | 1 | 1 | 1 | 1 |
| 2.04 | 1 | 1 | 1 | 1 | 1 | 1 | 1 |
| 2.07 | 1 | 1 | 1 | 1 | 1 | 4 | 1 |
| 6.02 | 1 | 3 | 1 | 1 | 1 | 9 | 2 |
| 6.07 | 1 | 1 | 1 | 1 | 1 | 1 | 1 |
| 6.08 | 1 | 2 | 6 | 3 | 1 | 5 | 2 |
| 6.11 | 1 | 2 | 1 | 1 | 1 | 9 | 1 |
| 6.28 | 2 | 2 | 2 | 2 | 2 | 9 | 1 |
| 6.29 | 1 | 1 | 2 | 5 | 2 | 5 | 1 |

Example 39: Postemergence herbicidal action:

A large number of weeds and cultivated plants were sprayed postemergence in the 4-to 6-leaf stage with an aqueous active ingredient dispersion in rates of 4 kg of test compound per hectare and kept at 24°-26°C and 45-60% relative humidity. The test was evaluated at least 15 days after treatment in accordance with the same rating as employed in the preemergence test.

Results: dosage 4 kg active ingredient per hectare

| plant tested / compound tested | solanum | sinapis | phaseolus |
|---|---|---|---|
| 1.02 | 3 | 3 | 2 |
| 1.03 | 2 | 2 | 2 |
| 1.04 | 2 | 2 | 2 |
| 1.15 | 2 | 2 | 2 |
| 1.31 | 2 | 2 | 2 |
| 1.68 | 2 | 3 | 2 |
| 1.73 | 1 | 3 | 3 |
| 1.77 | 2 | 3 | 2 |
| 1.80 | 3 | 2 | 2 |
| 1.81 | 3 | 2 | 2 |
| 1.85 | 2 | 3 | 2 |
| 1.96 | 3 | 2 | 2 |
| 1.97 | 3 | 2 | 2 |
| 2.07 | 2 | 2 | 2 |
| 2.20 | 2 | 2 | 3 |
| 2.34 | 3 | 2 | 2 |
| 2.85 | 2 | 3 | 3 |
| 2.92 | 3 | 2 | 2 |
| 2.125 | 2 | 2 | 3 |
| 5.18 | 3 | 2 | 2 |
| 6.36 | 2 | 3 | 2 |
| 6.69 | 2 | 4 | 2 |
| 6.76 | 2 | 3 | 2 |
| 11.03 | 3 | 1 | 3 |
| 13.26 | 2 | 3 | 2 |
| 13.11 | 3 | 3 | 2 |

Example 40: Herbicidal action against paddy rice associated weeds

The seeds of the waterweeds Echinochloa crus galli and Monochoria vaginalis were sown in plastic containers (60cm² surface, 500 ml by volume) together. The containers were watered up to the soil surface and after three days the water level was raised slightly above the soil surface (3-5 mm). Three days after sowing an aqueous emulsion of the active compound was applied by spraying the containers at a rate of application of 4 kg of a.i. per hectare (dilution 550 l/ha). The containers were kept in a greenhouse for three weeks under conditions optimal for the waterweeds, i.e. at a temperature between 20 and 25°C and under high humidity.

The evaluation of the tests was made in accordance with the rating given in example 39.

<u>Results</u>: dosage 4 kg active ingredient per hectare

| plant tested<br>compound tested | Echinochloa | Monochoria |
|---|---|---|
| 1.02 | 1 | 1 |
| 1.03 | 1 | 1 |
| 1.04 | 1 | 1 |
| 1.10 | 1 | 1 |
| 1.11 | 1 | 1 |
| 1.14 | 1 | 1 |
| 1.15 | 1 | 1 |
| 1.31 | 1 | 1 |
| 1.36 | 1 | 1 |
| 1.67 | 1 | 1 |
| 1.68 | 1 | 1 |
| 1.73 | 1 | 1 |
| 1.74 | 1 | 1 |
| 1.76 | 1 | 1 |
| 1.77 | 1 | 1 |
| 1.80 | 1 | 1 |
| 1.81 | 1 | 1 |
| 1.82 | 1 | 1 |
| 1.85 | 1 | 1 |
| 1.95 | 1 | 1 |
| 1.96 | 1 | 1 |
| 1.97 | 1 | 1 |
| 2.02 | 1 | 2 |
| 2.03 | 1 | 1 |
| 2.07 | 1 | 1 |
| 2.08 | 1 | 1 |

| plant tested compound tested | Echinochloa | Monochoria |
|---|---|---|
| 2.11 | 1 | 1 |
| 2.12 | 1 | 1 |
| 2.13 | 1 | 1 |
| 2.14 | 1 | 1 |
| 2.15 | 1 | 1 |
| 2.20 | 1 | 1 |
| 2.34 | 1 | 1 |
| 2.42 | 1 | 1 |
| 2.43 | 1 | 1 |
| 2.85 | 1 | 1 |
| 2.88 | 2 | 1 |
| 2.90 | 1 | 1 |
| 2.92 | 1 | 1 |
| 2.99 | 1 | 1 |
| 2.118 | 2 | 1 |
| 2.122 | 2 | 1 |
| 2.125 | 2 | 1 |
| 2.126 | 1 | 1 |
| 5.01 | 1 | 1 |
| 5.14 | 1 | 1 |
| 5.15 | 1 | 1 |
| 5.17 | 2 | 1 |
| 5.18 | 1 | 1 |
| 5.24 | 1 | 1 |
| 6.07 | 1 | 2 |
| 6.08 | 1 | 1 |
| 6.09 | 1 | 1 |
| 6.02 | 1 | 1 |
| 6.04 | 1 | 1 |
| 6.11 | 1 | 1 |
| 6.28 | 1 | 1 |
| 6.29 | 1 | 1 |
| 6.35 | 1 | 1 |
| 6.36 | 1 | 1 |
| 6.66 | 1 | 1 |
| 6.67 | 1 | 1 |
| 6.68 | 1 | 1 |
| 6.69 | 1 | 1 |
| 6.70 | 1 | 1 |
| 6.71 | 1 | 1 |
| 6.74 | 1 | 1 |
| 6.76 | 1 | 1 |
| 6.79 | 1 | 1 |
| 11.03 | 1 | 1 |
| 13.11 | 1 | 1 |
| 13.26 | 1 | 1 |
| 13.41 | 1 | 1 |
| 13.51 | 1 | 1 |

| plant tested compound tested | Echinochloa | Monochoria |
|---|---|---|
| 13.56 | 1 | 1 |
| 13.77 | 1 | 1 |
| 13.79 | 1 | 1 |
| 13.82 | 1 | 1 |
| 13.83 | 1 | 1 |

## Claims

1. A method for controlling weeds, said method comprising applying to said weeds or to the locus thereof of a herbicidally effective amount of a chemical compound having the formula

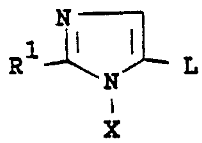 (I)

or a stereochemically isomeric form thereof, or a salt thereof, wherein
$R^1$ is hydrogen or mercapto;
L is cyano or a radical of formula

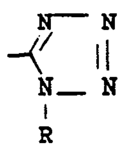 , 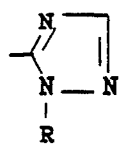 , 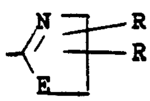 , 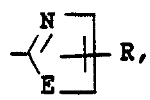 , 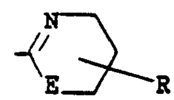 , 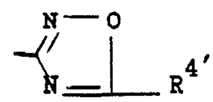

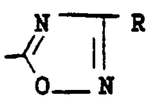 , 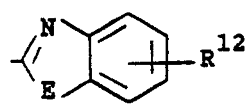 , 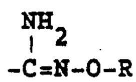 , 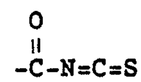 ,

$-C(=G)-D-R^2$ or $-C(=G^1)-O-R^5$;

each R independently is hydrogen or $C_1$-$C_5$alkyl;
E is oxygen, sulfur or -NR-;
$R^4$ is hydrogen, $C_1$-$C_5$alkyl or trifluoromethyl;
G is oxygen, sulfur or =NR;
$G^1$ is oxygen or sulfur;

D is sulfur, $-N(R^3)-$, $-N(R^3)-NH-$, $-N(R^3)-O-$, $-\overset{|}{N}-C(=G)-E-R^3$,

$-\overset{|}{N}-C(=G)-R^3$, $-\overset{|}{N}SO_2-R^3$, $-\overset{|}{N}-CN$, $-NH-CH_2-CH_2-O-$ or $-N-CH_2-CH_2-O-$;
$\quad | $
$CH_2-CH_2-OH$

$R^2$ and each $R^3$ independently are hydrogen, $C_1$-$C_5$alkyl, $C_3$-$C_5$alkenyl, $C_3$-$C_5$alkynyl, $C_3$-$C_7$cycloalkyl or $C_1$-$C_5$alkyl substituted with aryl, $C_3$-$C_7$cycloalkyl, $C_3$-$C_7$cycloalkyloxy, $C_1$-$C_5$alkyloxy, hydroxy, carboxyl, $C_1$-$C_5$alkyloxycarbonyl or $C_1$-$C_5$alkylcarbonyl, or
$R^2$ and $R^3$ together with the nitrogen atom to which they are attached may form piperidinyl, pyrrolidinyl, 2-

oxopiperidinyl, 2-oxopyrrolidinyl, morpholinyl, thiomorpholinyl, imidazolyl or piperazinyl being optionally substituted in the 4-position with $C_1$-$C_5$alkyl, $C_1$-$C_5$alkylcarbonyl, $C_1$-$C_5$alkyloxycarbonyl or mono-and di$C_1$-$C_5$alkylaminocarbonyl, and said cyclic radicals derived from $R^2$ and $R^3$ each being optionally substituted with one to three $C_1$-$C_5$alkyl radicals;

$R^5$ is phenyl, naphthalenyl, or an aromatic five-or six-membered heterocycle connected on a carbon atom; said phenyl, naphthalenyl or heterocycle being optionally substituted where possible with 1 to 3 substituents independently selected from $C_1$-$C_5$alkyl, $C_2$-$C_5$alkenyl, $C_2$-$C_5$alkynyl, halo, $C_1$-$C_5$alkyloxy, $C_1$-$C_5$alkylthio, mono-, di-or trihalo$C_1$-$C_5$alkyl, mono-, di-or trihalo$C_1$-$C_5$alkyloxy and nitro;

or $R^5$ is a radical of formula $-N=CR^{14}R^{15}$; or $C_1$-$C_6$alkyl substituted with $-NR^{16}R^{17}$, $-NR^{16}$-CO-$R^{17}$, $-NR^{16}$-CO-$NR^{17}R^{18}$, hydroxy, mono-, di-or trihalo$C_2$-$C_8$alkyloxy, -O-CO-$R^{16}$, -O-CO-O-$R^{19}$, -O-CO-$NR^{16}R^{17}$, -O-CO-CHCl-$R^{16}$, -O-CO-$CCl_2$-$R^{16}$, -O-$SO_2$-$R^{19}$, $C_3$-$C_7$cycloalkyl, -CO-$R^{16}$, -CO-$NR^{16}R^{17}$, $-S(O)_mR^{19}$, $-SiR^{19}R^{20}R^{21}$ or -PO-$(OR^{16})R^{22}$;

or $R^5$ is $C_1$-$C_6$alkyl substituted with one or two radicals independently selected from -CN and -COOR$^{16}$;

or $R^5$ is $C_1$-$C_6$alkyl substituted with one, two or three radicals independently selected from halo and mono-, di-or trihalo$C_1$-$C_6$alkyl;

$R^{14}$ is hydrogen, $C_1$-$C_6$alkyl, $C_3$-$C_7$cycloalkyl or aryl;

$R^{15}$ is $C_1$-$C_6$alkyl, $C_3$-$C_7$cycloalkyl or aryl;

or $R^{14}$ and $R^{15}$ taken together may form a $C_4$-$C_6$alkanediyl radical;

$R^{16}$, $R^{17}$ and $R^{18}$ independently are hydrogen, $C_1$-$C_6$alkyl, $C_3$-$C_5$alkenyl, $C_3$-$C_5$alkynyl, aryl, $C_1$-$C_5$alkyloxy$C_2$-$C_5$alkyl or $C_1$-$C_5$alkyloxycarbonyl$C_1$-$C_5$alkyl; or

$R^{16}$ and $R^{17}$ taken together may form a $C_3$-$C_6$alkanediyl radical;

$R^{19}$, $R^{20}$ and $R^{21}$ independently are $C_1$-$C_6$alkyl, $C_3$-$C_5$alkenyl, $C_3$-$C_5$alkynyl, or $C_1$-$C_5$alkyloxy$C_1$-$C_5$alkyl;

$R^{22}$ is hydrogen, $C_1$-$C_6$alkyl, hydroxy or $C_1$-$C_6$alkyloxy;

m is 0, 1 or 2;

X is 1-indanyl, 1-tetrahydronaphthalenyl, 5-benzocycloheptanyl, 4-tetrahydrobenzothienyl, 4-tetrahydrobenzofuryl, 5-tetrahydroquinolyl, 5-tetrahydroisoquinolyl, 8-tetrahydroquinolyl, 8-tetrahydro isoquinolyl, 9,10-dihydro-9-antracenyl, 9H-fluoren-9-yl, 5-dibenzo[a,d]cycloheptenyl, 5-dibenzo[a,d]cycloheptanyl or 1-dihydronaphthalenyl each unsubstituted or substituted with one to six substituents selected from the group consisting of $C_1$-$C_5$alkyl, mono-and di(aryl) $C_1$-$C_5$alkyl, $C_1$-$C_5$alkyloxy, halo, $C_3$-$C_7$alkenyl, amino, nitro, $C_1$-$C_5$alkylcarbonylamino, trifluoromethyl and difluoromethoxy, wherein two geminal substituents together with the carbon atom to which they are attached may form a spirocyclic $C_3$-$C_7$cycloalkyl group, or two of said substituents taken together may form a $C_1$-$C_5$alkanediyl or $C_5$-$C_7$cycloalkanediyl group, said $C_1$-$C_5$alkanediyl or $C_5$-$C_7$cycloalkanediyl group being optionally substituted with one or two radicals independently selected from $C_1$-$C_5$alkyl, mono-and di(aryl)$C_1$-$C_5$alkyl, $C_1$-$C_5$alkyloxy, halo, $C_3$-$C_7$alkenyl, trifluoromethyl, difluoromethoxy and aryl; or

X is a group

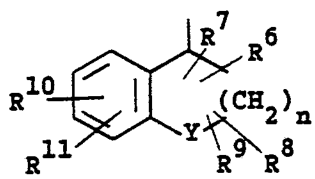 ,

or a group - 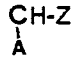 ; wherein

n is zero, one or two;

Y is a group $-CH_2O-$, $CH_2-S(O)_m$, $-CH_2-N(R^{13})$-or $-CH=N-$, wherein the heteroatom is linked to the carbon atom of the benzene ring, and wherein m is zero, one or two;

$R^6$, $R^7$, $R^8$ and $R^9$ each independently are hydrogen, $C_1$-$C_5$alkyl, mono-and di(aryl)$C_1$-$C_5$alkyl, $C_1$-$C_5$alkyloxy, halo, $C_3$-$C_7$alkenyl, $C_1$-$C_5$alkyl substituted with one to three halo atoms, $C_1$-$C_5$alkyloxy substituted with one to three halo atoms, or aryl; or

$R^6$ and $R^7$ taken together may form a fused benzene residue which may optionally be substituted with one or two substituents each independently selected from hydrogen, $C_1$-$C_5$alkyl, $C_1$-$C_5$alkyloxy, halo, $C_1$-$C_5$alkyl substituted with one to three halo atoms, $C_1$-$C_5$alkyloxy substituted with one to three halo atoms, nitro, amino and -NH-CO-M; or where $R^6$ and $R^7$ are geminally substituted, they may form a spirocyclic carbon ring with 3 to 7 carbon atoms; or $R^6$ and $R^7$ taken together may form a $C_1$-$C_5$alkanediyl or a $C_5$-

$C_7$cycloalkanediyl group being optionally substituted with one or two radicals independently selected from $C_1$-$C_5$alkyl, mono-and di(aryl)$C_1$-$C_5$alkyl, $C_1$-$C_5$alkyloxy, halo, $C_3$-$C_7$alkenyl, $C_1$-$C_5$alkyl substituted with one to three halo atoms, $C_1$-$C_5$alkyloxy substituted with one to three halo atoms and aryl; and

$R^{10}$, $R^{11}$ and $R^{12}$ are each independently hydrogen, $C_1$-$C_5$alkyl, $C_1$-$C_5$alkyloxy, halo, $C_1$-$C_5$alkyl substituted with one to three halo atoms, $C_1$-$C_5$alkyloxy substituted with one to three halo atoms, cyano, nitro, amino, mono-and di$C_1$-$C_5$alkylamino, or -NM-CO-M;

$R^{13}$ is hydrogen, $C_1$-$C_5$alkyl, $C_1$-$C_5$alkanoyl or 4-methylphenylsulfonyl;

A is hydrogen; $C_3$-$C_7$cycloalkyl optionally substituted with one or two $C_1$-$C_5$alkyl radicals; $C_1$-$C_7$alkyl optionally substituted with $C_1$-$C_7$alkyloxy or with an Ar radical; or $C_1$-$C_7$alkyl substituted with both a $C_1$-$C_7$alkyloxy and an Ar radical; or a radical selected from pyridinyl, pyrimidinyl, naphthalenyl, furanyl and thienyl, each unsubstituted or substituted with one or two radicals independently selected from $C_1$-$C_5$alkyl, $C_1$-$C_5$alkyloxy, halo, nitro, amino, mono-and di$C_1$-$C_5$alkylamino, -NH-CO-M, cyano, trifluoromethyl and difluoromethoxy;

said radical Ar being phenyl, pyridinyl, pyrimidinyl, naphthalenyl, furanyl or thienyl, each unsubstituted or substituted with one or two and in case Ar is phenyl also with three substituents selected from $C_1$-$C_5$alkyl, $C_1$-$C_5$alkyloxy, halo, nitro, amino, mono-and di$C_1$-$C_5$alkylamino, -NH-CO-M, cyano, trifluoromethyl and difluoromethoxy;

Z is naphthalenyl, thienyl, furanyl, pyrimidinyl, phenyl or pyridinyl, each unsubstituted or substituted with one or two substituents and in case Z is phenyl also with three substituents independently selected from $C_1$-$C_5$alkyl, $C_1$-$C_5$alkyloxy, halo, cyano, nitro, amino, mono-and di$C_1$-$C_5$alkylamino, -NH-CO-M, trifluoromethyl and difluoromethoxy; and

M is $C_1$-$C_5$alkyl; and

aryl is phenyl optionally substituted with one to three substituents each independently selected from $C_1$-$C_5$alkyl, $C_1$-$C_5$alkyloxy and halo.

2. A method according to claim 1 wherein L is cyano, a radical of formula -C(=O)-D-$R^2$ or -COO$R^5$.

3. A method according to claim 2 wherein D is -N($R^3$)-or -N($R^3$)-O-and $R^2$ is hydrogen, $C_1$-$C_5$alkyl, hydroxy$C_1$-$C_5$alkyl, $C_3$-$C_7$cycloalkyl, $C_3$-$C_5$alkenyl or $C_3$-$C_5$alkynyl and $R^3$ is hydrogen or $C_1$-$C_5$alkyl; and $R^5$ is phenyl optionally substituted with 1 to 3 halo atoms, or a radical of formula -N=C$R^{14}R^{15}$ wherein $R^{14}$ and $R^{15}$ independently are hydrogen or $C_1$-$C_6$alkyl; or a $C_1$-$C_6$alkyl group substituted with di-$C_1$-$C_6$alkyloxyphosphonyl, tri-$C_1$-$C_6$alkylsilyl, amino, cyano, carboxyl or $C_1$-$C_5$alkyloxycarbonyl, or $C_1$-$C_6$alkyl substituted with one to three halo's.

4. A method according to claim 3 wherein $R^1$ is hydrogen, X is 2,3-dihydro-2,2-dimethyl-1H-inden-1-yl, 2,2-dimethyltetrahydronaphthalenyl, 2,3-dimethyl-chroman-4-yl or 2-methyl-chroman-4-yl.

5. A method according to claim 4 wherein the compound is N-methyl-1-(1,2,3,4-tetrahydro-1-naphthalenyl)-1H-imidazole-5-carboxamide, 1-(2,3-dihydro-2,2-dimethyl-1H-indenyl)-1H-imidazole-5-carboxamide or N-methyl-1-(2,3-dihydro-2,2-dimethyl-1H-indenyl)-1Himidazole-5-carboxamide.

6. A method according to any of claims 1 to 5 for selectively controlling weeds in crops of useful plants.

7. A method according to claim 6 wherein the crop is rice, maize or cereals.

8. A method according to claim 6 wherein the crop is rice and the rice is transplanted rice.

9. A chemical compound having the formula (I) as defined in claim 1, a stereochemical isomeric form thereof, or a salt thereof; provided that

(i) when X is 1-aryl-1-ethyl then L is other than cyano; $CONH_2$; or COO$R^5$ wherein $R^5$ is monohalo$C_1$-$C_5$alkyl; or

(ii)when X is indanyl or 1,2,3,4-tetrahydro-1-naphthalenyl, then L is other than COO$R^5$ wherein $R^5$ is di$C_1$-$C_5$alkylamino$C_1$-$C_6$alkyl.

10. A compound according to claim 9 wherein L is cyano, a radical of formula -C(=O)-D-$R^2$ or -COO$R^5$.

11. A compound according to claim 6 wherein D is -N($R^3$)-or -N($R^3$)-O-and $R^2$ is hydrogen, $C_1$-$C_5$alkyl, hydroxy$C_1$-$C_5$alkyl, $C_3$-$C_7$cycloalkyl, $C_3$-$C_5$alkenyl or $C_3$-$C_5$alkynyl and $R^3$ is hydrogen or $C_1$-$C_5$alkyl; and $R^5$ is phenyl optionally substituted with 1 to 3 halo atoms, or a radical of formula -N=C$R^{14}R^{15}$ wherein $R^{14}$ and $R^{15}$ independently are hydrogen or $C_1$-$C_6$alkyl; or a $C_1$-$C_6$alkyl group substituted with di-$C_1$-$C_6$alkyl oxyphosphonyl, tri-$C_1$-$C_6$alkylsilyl, amino, cyano, carboxyl or $C_1$-$C_5$alkyl-oxycarbonyl, or $C_1$-$C_6$alkyl substituted with one to three halo's.

12. A compound according to claim 9 wherein the compound is N-methyl-1-(1,2,3,4-tetrahydro-1-naphthalenyl)-1H-imidazole-5-carboxamide, 1-(2,3-dihydro-2,2-dimethyl-1H-indenyl)-1H-imidazole-5-carboxamide or N-methyl-1-(2,3-dihydro-2,2-dimethyl-1H-indenyl)-1H-imidazole-5-carboxamide.

13. A herbicidal composition comprising an inert carrier and, if desired, other adjuvants, and as active ingredient a herbicidally effective amount of a chemical compound having the formula (I) as defined in claim 1.

14. A composition according to claim 13 wherein the active ingredient is a chemical compound of formula (I) as claimed in any of claims 9 to 12.

15. A process for preparing a composition as claimed in claim 13 or 14 characterized in that the active ingredient is intimately mixed with the carrier or carriers and, if desired, other adjuvants.

16. A process for preparing a chemical compound having the formula (I) as claimed in any of claims 9 to 12, characterized by

a) condensing a compound of formula

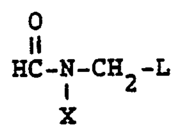

(II)

wherein X and L are as defined hereinabove, with a $C_1$-$C_4$alkyl ester of formic acid in the presence of a base in a reaction-inert solvent; and treating the resultant intermediate of formula

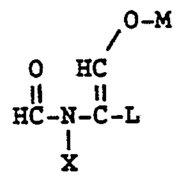

(III)

wherein X and L are as defined hereinabove and M is an alkali metal atom, either
with an alkali metal isothiocyanate in the presence of an acid, thus obtaining a 2-mercaptoimidazole of formula

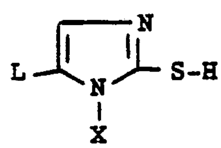

(I-a)

wherein X and L are as defined hereinabove, which optionally is converted into a compound of the formula

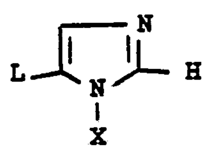

(I-b)

wherein X and L are as defined hereinabove,
by reacting the starting compound (I-a) with nitric acid optionally in the presence of an alkali metal nitrite, or with Raney-nickel in the presence of a lower aliphatic alcohol, at a temperature between 40°C and 80°C; or also by treating the starting compounds (I-a) with an aqueous hydrogen peroxide solution preferably in the presence of a carbonxylic acid, or
with a carboxylic acid amide of 1 to 3 carbon atoms, preferably formamide, in the presence of an acid at a temperature between 50°C and 250°C, preferably between 120°C and 170°C; or
with an excess of ammonium carbonate or hydrogen carbonate in a suitable solvent, which may be a reaction-inert solvent or an acid, at a temperature between 20°C and 200°C, preferably between 25°C and the reflux temperature of the reaction mixture;

b) reacting an activated derivative of formula

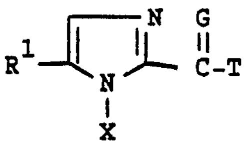 (V) .

wherein R', G and X are as defined hereinabove, and T is a reactive leaving group, with a mercaptan or amine of formula $H\text{-}D\text{-}R^2$ or with an alcohol of formula $H\text{-}O\text{-}R^5$, in a reaction-inert solvent, optionally in the presence of a base, thus preparing compounds of formula (I) wherein L is a radical $-C(=G)\text{-}D\text{-}R^2$ or $-C(=G')\text{-}OR^5$;

and, if desired, converting the compounds into each other following art-known functional group transformation reactions; and if further desired, converting the compounds of formula (I) into a salt form by treatment with an appropriate acid or base; or conversely, converting the salt into the free base with alkali, or into the free acidic form with an acid; and/or preparing stereochemically isomeric forms thereof.

17. A chemical compound having the formula

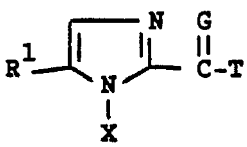 (V)

a salt or a stereoisomer thereof, wherein $R^1$, X and G are as defined in claim 1, and T is halo, $-OCO\text{-}C_1\text{-}C_5$alkyl, $-O\text{-}CO\text{-}O\text{-}C_1\text{-}C_5$alkyl or a group

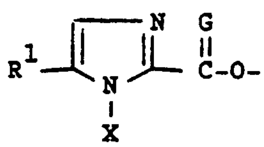 ,

provided that X is other than unsubstituted 1-indanyl, 1,2,3,4-tetrahydro-1-naphthalenyl and other than a group -CH(A)Z, A being unsubstituted $C_1\text{-}C_5$alkyl and Z being phenyl, pyridinyl or thienyl, said phenyl being unsubstituted or substituted with up to three substituents selected from $C_1\text{-}C_5$alkyl, $C_1\text{-}C_5$alkyloxy or halo, when $R^1$ is hydrogen and G is oxygen.